(19) Europäisches Patentamt
European Patent Office
Office européen des brevets

(11) EP 1 475 098 B1

(12) EUROPEAN PATENT SPECIFICATION

(45) Date of publication and mention of the grant of the patent:
**05.08.2015 Bulletin 2015/32**

(21) Application number: **03701758.9**

(22) Date of filing: **17.01.2003**

(51) Int Cl.:
***A61K 9/00*** *(2006.01)* ***A61K 9/19*** *(2006.01)*
***A61K 38/36*** *(2006.01)* ***A61K 47/10*** *(2006.01)*
***A61K 47/18*** *(2006.01)*

(86) International application number:
**PCT/JP2003/000339**

(87) International publication number:
**WO 2003/061687 (31.07.2003 Gazette 2003/31)**

(54) **HIGH-CONCENTRATION PREPARATION OF SOLUBLE THROMBOMODULIN**

HOCHKONZENTRIERTE ZUBEREITUNG VON LÖSLICHEM THROMBOMODULIN

PREPARATION A CONCENTRATION ELEVEE DE THROMBOMODULINE SOLUBLE

(84) Designated Contracting States:
**AT BE BG CH CY CZ DE DK EE ES FI FR GB GR HU IE IT LI LU MC NL PT SE SI SK TR**

(30) Priority: **18.01.2002 JP 2002009951**

(43) Date of publication of application:
**10.11.2004 Bulletin 2004/46**

(73) Proprietor: **Asahi Kasei Pharma Corporation**
**Tokyo 101-8101 (JP)**

(72) Inventor: **NISHIO, Fumihide**
**Mishima-shi, Shizuoka 411-0019 (JP)**

(74) Representative: **Forstmeyer, Dietmar et al**
**BOETERS & LIECK**
**Oberanger 32**
**80331 München (DE)**

(56) References cited:
**EP-A- 1 029 548**
**EP-A1- 1 029 548**
**GB-A- 2 015 340**
**JP-A- 9 124 481**
**JP-A- 62 289 529**
**EP-A1- 0 689 843**
**EP-A2- 0 247 584**
**JP-A- 6 321 805**
**JP-A- 10 072 364**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

Sequence Listing

<110> Asahi Kasei kabushiki Kaisha

<120> High-concentration preparation of soluble thrombomodulin

<130> ASAHI-33

<150> JP2002-009951
<151> 2002-01-18

<160> 9

<210> 1
<211> 516

<212> PRAT
<213> Artificial sequence

<220>
<223> Partial amino acid sequence of human-originated soluble thrombomodulin

<400> 1

```
Met Leu Gly Val Leu Val Leu Gly Ala Leu Ala Leu Ala Gly Leu Gly
1               5                   10                  15
Phe Pro Ala Pro Ala Glu Pro Gln Pro Gly Gly Ser Gln Cys Val Glu
            20                  25                  30
His Asp Cys Phe Ala Leu Tyr Pro Gly Pro Ala Thr Phe Leu Asn Ala
        35                  40                  45
Ser Gln Ile Cys Asp Gly Leu Arg Gly His Leu Met Thr Val Arg Ser
    50                  55                  60
Ser Val Ala Ala Asp Val Ile Ser Leu Leu Leu Asn Gly Asp Gly Gly
65                  70                  75                  80
Val Gly Arg Arg Arg Leu Trp Ile Gly Leu Gln Leu Pro Pro Gly Cys
                85                  90                  95
Gly Asp Pro Lys Arg Leu Gly Pro Leu Arg Gly Phe Gln Trp Val Thr
            100                 105                 110
Gly Asp Asn Asn Thr Ser Tyr Ser Arg Trp Ala Arg Leu Asp Leu Asn
        115                 120                 125
Gly Ala Pro Leu Cys Gly Pro Leu Cys Val Ala Val Ser Ala Ala Glu
    130                 135                 140
Ala Thr Val Pro Ser Glu Pro Ile Trp Glu Glu Gln Gln Cys Glu Val
145                 150                 155                 160
Lys Ala Asp Gly Phe Leu Cys Glu Phe His Phe Pro Ala Thr Cys Arg
                165                 170                 175
Pro Leu Ala Val Glu Pro Gly Ala Ala Ala Ala Ala Val Ser Ile Thr
            180                 185                 190
Tyr Gly Thr Pro Phe Ala Ala Arg Gly Ala Asp Phe Gln Ala Leu Pro
        195                 200                 205
Val Gly Ser Ser Ala Ala Val Ala Pro Leu Gly Leu Gln Leu Met Cys
    210                 215                 220
Thr Ala Pro Pro Gly Ala Val Gln Gly His Trp Ala Arg Glu Ala Pro
225                 230                 235                 240
Gly Ala Trp Asp Cys Ser Val Glu Asn Gly Gly Cys Glu His Ala Cys
                245                 250                 255
Asn Ala Ile Pro Gly Ala Pro Arg Cys Gln Cys Pro Ala Gly Ala Ala
            260                 265                 270
Leu Gln Ala Asp Gly Arg Ser Cys Thr Ala Ser Ala Thr Gln Ser Cys
        275                 280                 285
Asn Asp Leu Cys Glu His Phe Cys Val Pro Asn Pro Asp Gln Pro Gly
    290                 295                 300
Ser Tyr Ser Cys Met Cys Glu Thr Gly Tyr Arg Leu Ala Ala Asp Gln
305                 310                 315                 320
His Arg Cys Glu Asp Val Asp Asp Cys Ile Leu Glu Pro Ser Pro Cys
                325                 330                 335
Pro Gln Arg Cys Val Asn Thr Gln Gly Gly Phe Glu Cys His Cys Tyr
            340                 345                 350
Pro Asn Tyr Asp Leu Val Asp Gly Glu Cys Val Glu Pro Val Asp Pro
        355                 360                 365
Cys Phe Arg Ala Asn Cys Glu Tyr Gln Cys Gln Pro Leu Asn Gln Thr
```

```
 370                 375                     380
Ser Tyr Leu Cys Val Cys Ala Glu Gly Phe Ala Pro Ile Pro His Glu
385                 390                 395                 400
Pro His Arg Cys Gln Met Phe Cys Asn Gln Thr Ala Cys Pro Ala Asp
                405                 410                 415
Cys Asp Pro Asn Thr Gln Ala Ser Cys Glu Cys Pro Glu Gly Tyr Ile
            420                 425                 430
Leu Asp Asp Gly Phe Ile Cys Thr Asp Ile Asp Glu Cys Glu Asn Gly
        435                 440                 445
Gly Phe Cys Ser Gly Val Cys His Asn Leu Pro Gly Thr Phe Glu Cys
    450                 455                 460
Ile Cys Gly Pro Asp Ser Ala Leu Val Arg His Ile Gly Thr Asp Cys
465                 470                 475                 480
Asp Ser Gly Lys Val Asp Gly Gly Asp Ser Gly Ser Gly Glu Pro Pro
                485                 490                 495
Pro Ser Pro Thr Pro Gly Ser Thr Leu Thr Pro Pro Ala Val Gly Leu
            500                 505                 510
Val His Ser Gly
        515
```

<210> 2
<211> 1548
<212> DNA
<213> Artificial sequence

<220>
<223> Partial base sequence of human-originated soluble thrombomodulin gene

<400> 2

```
atgcttgggg tcctggtcct tggcgcgctg gccctggccg gcctggggtt ccccgcaccc   60
gcagagccgc agccgggtgg cagccagtgc gtcgagcacg actgcttcgc gctctacccg  120
ggccccgcga ccttcctcaa tgccagtcag atctgcgacg gactgcgggg ccacctaatg  180
acagtgcgct cctcggtggc tgccgatgtc atttccttgc tactgaacgg cgacggcggc  240
gttggccgcc ggcgcctctg gatcggcctg cagctgccac ccggctgcgg cgaccccaag  300
cgcctcgggc ccctgcgcgg cttccagtgg gttacgggag acaacaacac cagctatagc  360
aggtgggcac ggctcgacct caatggggct cccctctgcg gcccgttgtg cgtcgctgtc  420
tccgctgctg aggccactgt gcccagcgag ccgatctggg aggagcagca gtgcgaagtg  480
aaggccgatg gcttcctctg cgagttccac ttcccagcca cctgcaggcc actggctgtg  540
gagcccggcg ccgcggctgc cgccgtctcg atcacctacg gcaccccgtt cgcggcccgc  600
ggagcggact tccaggcgct gccggtgggc agctccgccg cggtggctcc cctcggctta  660
cagctaatgt gcaccgcgcc gcccggagcg gtccaggggc actgggccag ggaggcgccg  720
ggcgcttggg actgcagcgt ggagaacggc ggctgcgagc acgcgtgcaa tgcgatccct  780
ggggctcccc gctgccagtg cccagccggc gccgccctgc aggcagacgg gcgctcctgc  840
accgcatccg cgacgcagtc ctgcaacgac ctctgcgagc acttctgcgt tcccaacccc  900
gaccagccgg gctcctactc gtgcatgtgc gagaccggct accggctggc ggccgaccaa  960
caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgtcc gccacattgg caccgactgt 1440
gactccggca aggtggacgg tggcgacagc ggctctggcg agcccccgcc cagcccgacg 1500
cccggctcca ccttgactcc tccggccgtg gggctcgtgc attcgggc             1548
```

<210> 3
<211> 132
<212> PRT
<213> Artificial sequence

<220>

<223> Partial amino acid sequence of human-originated soluble thrombomodulin

<400> 3

```
Met Leu Gly Val Leu Val Leu Gly Ala Leu Ala Leu Ala Gly Leu Gly
1               5                   10                  15

Phe Pro Asp Pro Cys Phe Arg Ala Asn Cys Glu Tyr Gln Cys Gln Pro
            20                  25                  30
Leu Asn Gln Thr Ser Tyr Leu Cys Val Cys Ala Glu Gly Phe Ala Pro
        35                  40                  45
Ile Pro His Glu Pro His Arg Cys Gln Met Phe Cys Asn Gln Thr Ala
    50                  55                  60
Cys Pro Ala Asp Cys Asp Pro Asn Thr Gln Ala Ser Cys Glu Cys Pro
65                  70                  75                  80
Glu Gly Tyr Ile Leu Asp Asp Gly Phe Ile Cys Thr Asp Ile Asp Glu
                85                  90                  95
Cys Glu Asn Gly Gly Phe Cys Ser Gly Val Cys His Asn Leu Pro Gly
            100                 105                 110
Thr Phe Glu Cys Ile Cys Gly Pro Asp Ser Ala Leu Val Arg His Ile
        115                 120                 125
Gly Thr Asp Cys
    130
```

<210> 4
<211> 396
<212> DNA
<213> Artificial sequence

<220>
<223> Partial base sequence of human-originated soluble thrombomodulin gene

<400> 4

```
atgcttgggg tcctggtcct tggcgcgctg gccctggccg gcctggggtt ccccgacccg   60
tgcttcagag ccaactgcga gtaccagtgc cagcccctga accaaactag ctacctctgc  120
gtctgcgccg agggcttcgc gcccattccc cacgagccgc acaggtgcca gatgttttgc  180
aaccagactg cctgtccagc cgactgcgac cccaacaccc aggctagctg tgagtgccct  240
gaaggctaca tcctggacga cggtttcatc tgcacggaca tcgacgagtg cgaaaacggc  300
ggcttctgct ccggggtgtg ccacaacctc cccggtacct tcgagtgcat ctgcgggccc  360
gactcggccc ttgtccgcca cattggcacc gactgt                            396
```

<210> 5
<211> 516
<212> PRT
<213> Artificial sequence

<220>
<223> Partial amino acid sequence of human-originated soluble thrombomodulin

<400> 5

Met Leu Gly Val Leu Val Leu Gly Ala Leu Ala Leu Ala Gly Leu Gly
1 5 10 15
Phe Pro Ala Pro Ala Glu Pro Gln Pro Gly Gly Ser Gln Cys Val Glu
20 25 30
His Asp Cys Phe Ala Leu Tyr Pro Gly Pro Ala Thr Phe Leu Asn Ala
35 40 45
Ser Gln Ile Cys Asp Gly Leu Arg Gly His Leu Met Thr Val Arg Ser
50 55 60
Ser Val Ala Ala Asp Val Ile Ser Leu Leu Leu Asn Gly Asp Gly Gly
65 70 75 80
Val Gly Arg Arg Arg Leu Trp Ile Gly Leu Gln Leu Pro Pro Gly Cys
85 90 95
Gly Asp Pro Lys Arg Leu Gly Pro Leu Arg Gly Phe Gln Trp Val Thr
100 105 110
Gly Asp Asn Asn Thr Ser Tyr Ser Arg Trp Ala Arg Leu Asp Leu Asn
115 120 125
Gly Ala Pro Leu Cys Gly Pro Leu Cys Val Ala Val Ser Ala Ala Glu
130 135 140
Ala Thr Val Pro Ser Glu Pro Ile Trp Glu Glu Gln Gln Cys Glu Val
145 150 155 160
Lys Ala Asp Gly Phe Leu Cys Glu Phe His Phe Pro Ala Thr Cys Arg
165 170 175
Pro Leu Ala Val Glu Pro Gly Ala Ala Ala Ala Ala Val Ser Ile Thr
180 185 190

Tyr Gly Thr Pro Phe Ala Ala Arg Gly Ala Asp Phe Gln Ala Leu Pro
195 200 205
Val Gly Ser Ser Ala Ala Val Ala Pro Leu Gly Leu Gln Leu Met Cys
210 215 220
Thr Ala Pro Pro Gly Ala Val Gln Gly His Trp Ala Arg Glu Ala Pro
225 230 235 240
Gly Ala Trp Asp Cys Ser Val Glu Asn Gly Gly Cys Glu His Ala Cys
245 250 255
Asn Ala Ile Pro Gly Ala Pro Arg Cys Gln Cys Pro Ala Gly Ala Ala
260 265 270
Leu Gln Ala Asp Gly Arg Ser Cys Thr Ala Ser Ala Thr Gln Ser Cys
275 280 285
Asn Asp Leu Cys Glu His Phe Cys Val Pro Asn Pro Asp Gln Pro Gly
290 295 300
Ser Tyr Ser Cys Met Cys Glu Thr Gly Tyr Arg Leu Ala Ala Asp Gln
305 310 315 320
His Arg Cys Glu Asp Val Asp Asp Cys Ile Leu Glu Pro Ser Pro Cys
325 330 335
Pro Gln Arg Cys Val Asn Thr Gln Gly Gly Phe Glu Cys His Cys Tyr
340 345 350
Pro Asn Tyr Asp Leu Val Asp Gly Glu Cys Val Glu Pro Val Asp Pro
355 360 365
Cys Phe Arg Ala Asn Cys Glu Tyr Gln Cys Gln Pro Leu Asn Gln Thr
370 375 380
Ser Tyr Leu Cys Val Cys Ala Glu Gly Phe Ala Pro Ile Pro His Glu
385 390 395 400
Pro His Arg Cys Gln Met Phe Cys Asn Gln Thr Ala Cys Pro Ala Asp
405 410 415
Cys Asp Pro Asn Thr Gln Ala Ser Cys Glu Cys Pro Glu Gly Tyr Ile
420 425 430
Leu Asp Asp Gly Phe Ile Cys Thr Asp Ile Asp Glu Cys Glu Asn Gly
435 440 445
Gly Phe Cys Ser Gly Val Cys His Asn Leu Pro Gly Thr Phe Glu Cys
450 455 460
Ile Cys Gly Pro Asp Ser Ala Leu Ala Arg His Ile Gly Thr Asp Cys
465 470 475 480
Asp Ser Gly Lys Val Asp Gly Gly Asp Ser Gly Ser Gly Glu Pro Pro
485 490 495
Pro Ser Pro Thr Pro Gly Ser Thr Leu Thr Pro Pro Ala Val Gly Leu
500 505 510
Val His Ser Gly
515

<210> 6

<211> 1548
<212> DNA
<213> Artificial sequence

<220>
<223> Partial base sequence of human-originated soluble thrombomodulin gene

<400> 6

```
atgcttgggg tcctggtcct tggcgcgctg gccctggccg gcctggggtt ccccgcaccc   60
gcagagccgc agccgggtgg cagccagtgc gtcgagcacg actgcttcgc gctctacccg  120
ggccccgcga ccttcctcaa tgccagtcag atctgcgacg gactgcgggg ccacctaatg  180
acagtgcgct cctcggtggc tgccgatgtc atttccttgc tactgaacgg cgacggcggc  240
gttggccgcc ggcgcctctg gatcggcctg cagctgccac ccggctgcgg cgaccccaag  300
cgcctcgggc ccctgcgcgg cttccagtgg gttacgggag acaacaacac cagctatagc  360
aggtgggcac ggctcgacct caatggggct cccctctgcg gcccgttgtg cgtcgctgtc  420
tccgctgctg aggccactgt gcccagcgag ccgatctggg aggagcagca gtgcgaagtg  480
aaggccgatg gcttcctctg cgagttccac ttcccagcca cctgcaggcc actggctgtg  540
gagcccggcg ccgcggctgc cgccgtctcg atcacctacg gcaccccgtt cgcggcccgc  600
ggagcggact tccaggcgct gccggtgggc agctccgccg cggtggctcc cctcggctta  660
cagctaatgt gcaccgcgcc gcccggagcg gtccaggggc actgggccag ggaggcgccg  720
ggcgcttggg actgcagcgt ggagaacggc ggctgcgagc acgcgtgcaa tgcgatccct  780
ggggctcccc gctgccagtg cccagccggc gccgccctgc aggcagacgg gcgctcctgc  840
accgcatccg cgacgcagtc ctgcaacgac ctctgcgagc acttctgcgt tcccaacccc  900
gaccagccgg gctcctactc gtgcatgtgc gagaccggct accggctggc ggccgaccaa  960

caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgccc gccacattgg caccgactgt 1440
gactccggca aggtggacgg tggcgacagc ggctctggcg agcccccgcc cagcccgacg 1500
cccggctcca ccttgactcc tccggccgtg gggctcgtgc attcgggc             1548
```

<210> 7
<211> 132
<212> PRT
<213> Artificial sequence

<220>
<223> Partial amino acid sequence of human-originated soluble thrombomodulin

<400> 7

Met Leu Gly Val Leu Val Leu Gly Ala Leu Ala Leu Ala Gly Leu Gly
1 5 10 15
Phe Pro Asp Pro Cys Phe Arg Ala Asn Cys Glu Tyr Gln Cys Gln Pro
20 25 30
Leu Asn Gln Thr Ser Tyr Leu Cys Val Cys Ala Glu Gly Phe Ala Pro
35 40 45
Ile Pro His Glu Pro His Arg Cys Gln Met Phe Cys Asn Gln Thr Ala
50 55 60
Cys Pro Ala Asp Cys Asp Pro Asn Thr Gln Ala Ser Cys Glu Cys Pro
65 70 75 80
Glu Gly Tyr Ile Leu Asp Asp Gly Phe Ile Cys Thr Asp Ile Asp Glu
85 90 95
Cys Glu Asn Gly Gly Phe Cys Ser Gly Val Cys His Asn Leu Pro Gly
100 105 110
Thr Phe Glu Cys Ile Cys Gly Pro Asp Ser Ala Leu Ala Arg His Ile
115 120 125
Gly Thr Asp Cys
130

<210> 8
<211> 396
<212> DNA
<213> Artificial sequence

<220>
<223> Partial base sequence of human-originated soluble thrombomodulin gene

<400> 8

atgcttgggg tcctggtcct tggcgcgctg gccctggccg gcctggggtt ccccgacccg 60
tgcttcagag ccaactgcga gtaccagtgc cagcccctga accaaactag ctacctctgc 120
gtctgcgccg agggcttcgc gcccattccc cacgagccgc acaggtgcca gatgttttgc 180
aaccagactg cctgtccagc cgactgcgac cccaacaccc aggctagctg tgagtgccct 240
gaaggctaca tcctggacga cggtttcatc tgcacggaca tcgacgagtg cgaaaacggc 300
ggcttctgct ccggggtgtg ccacaacctc cccggtacct tcgagtgcat ctgcgggccc 360
gactcggccc ttgcccgcca cattggcacc gactgt 396

<210> 9
<211> 21
<212> DNA
<213> Artificial sequence

<220>
<223> Synthetic DNA for mutation

<400> 9
aatgtggcgg gcaagggccg a 21

??

??

??

??

5/12

Sequence Listing

<110> Asahi Kasei Kabushiki Kaisha

<120> High-concentration preparation of soluble thrombomodulin

<130> ASAHI-33

<150> JP2002-009951
<151> 2002-01-18

<160> 9

<210> 1
<211> 516
<212> PRT
<213> Artificial sequence

<220>
<223> Partial amino acid sequence of human-originated soluble thrombomodulin

<400> 1

```
Met Leu Gly Val Leu Val Leu Gly Ala Leu Ala Leu Ala Gly Leu Gly
1               5                   10                  15

Phe Pro Ala Pro Ala Glu Pro Gln Pro Gly Gly Ser Gln Cys Val Glu
            20                  25                  30

His Asp Cys Phe Ala Leu Tyr Pro Gly Pro Ala Thr Phe Leu Asn Ala
        35                  40                  45

Ser Gln Ile Cys Asp Gly Leu Arg Gly His Leu Met Thr Val Arg Ser
```

50 55 60

Ser Val Ala Ala Asp Val Ile Ser Leu Leu Leu Asn Gly Asp Gly Gly

65 70 75 80

Val Gly Arg Arg Arg Leu Trp Ile Gly Leu Gln Leu Pro Pro Gly Cys

85 90 95

Gly Asp Pro Lys Arg Leu Gly Pro Leu Arg Gly Phe Gln Trp Val Thr

100 105 110

Gly Asp Asn Asn Thr Ser Tyr Ser Arg Trp Ala Arg Leu Asp Leu Asn

115 120 125

Gly Ala Pro Leu Cys Gly Pro Leu Cys Val Ala Val Ser Ala Ala Glu

130 135 140

Ala Thr Val Pro Ser Glu Pro Ile Trp Glu Glu Gln Gln Cys Glu Val

145 150 155 160

Lys Ala Asp Gly Phe Leu Cys Glu Phe His Phe Pro Ala Thr Cys Arg

165 170 175

Pro Leu Ala Val Glu Pro Gly Ala Ala Ala Ala Ala Val Ser Ile Thr

180 185 190

Tyr Gly Thr Pro Phe Ala Ala Arg Gly Ala Asp Phe Gln Ala Leu Pro

195 200 205

Val Gly Ser Ser Ala Ala Val Ala Pro Leu Gly Leu Gln Leu Met Cys

210 215 220

Thr Ala Pro Pro Gly Ala Val Gln Gly His Trp Ala Arg Glu Ala Pro

225 230 235 240

Gly Ala Trp Asp Cys Ser Val Glu Asn Gly Gly Cys Glu His Ala Cys

245 250 255

Asn Ala Ile Pro Gly Ala Pro Arg Cys Gln Cys Pro Ala Gly Ala Ala

260 265 270

Leu Gln Ala Asp Gly Arg Ser Cys Thr Ala Ser Ala Thr Gln Ser Cys

275 280 285

Asn Asp Leu Cys Glu His Phe Cys Val Pro Asn Pro Asp Gln Pro Gly
290 295 300

Ser Tyr Ser Cys Met Cys Glu Thr Gly Tyr Arg Leu Ala Ala Asp Gln
305 310 315 320

His Arg Cys Glu Asp Val Asp Asp Cys Ile Leu Glu Pro Ser Pro Cys
325 330 335

Pro Gln Arg Cys Val Asn Thr Gln Gly Gly Phe Glu Cys His Cys Tyr
340 345 350

Pro Asn Tyr Asp Leu Val Asp Gly Glu Cys Val Glu Pro Val Asp Pro
355 360 365

Cys Phe Arg Ala Asn Cys Glu Tyr Gln Cys Gln Pro Leu Asn Gln Thr
370 375 380

Ser Tyr Leu Cys Val Cys Ala Glu Gly Phe Ala Pro Ile Pro His Glu
385 390 395 400

Pro His Arg Cys Gln Met Phe Cys Asn Gln Thr Ala Cys Pro Ala Asp
405 410 415

Cys Asp Pro Asn Thr Gln Ala Ser Cys Glu Cys Pro Glu Gly Tyr Ile
420 425 430

Leu Asp Asp Gly Phe Ile Cys Thr Asp Ile Asp Glu Cys Glu Asn Gly
435 440 445

Gly Phe Cys Ser Gly Val Cys His Asn Leu Pro Gly Thr Phe Glu Cys
450 455 460

Ile Cys Gly Pro Asp Ser Ala Leu Val Arg His Ile Gly Thr Asp Cys
465 470 475 480

Asp Ser Gly Lys Val Asp Gly Gly Asp Ser Gly Ser Gly Glu Pro Pro
485 490 495

Pro Ser Pro Thr Pro Gly Ser Thr Leu Thr Pro Pro Ala Val Gly Leu
500 505 510

Val His Ser Gly

515

<210> 2
<211> 1548
<212> DNA
<213> Artificial sequence

<220>
<223> Partial base sequence of human-originated soluble thrombomodulin gene

<400> 2

```
atgcttgggg tcctggtcct tggcgcgctg gccctggccg gcctggggtt ccccgcaccc   60
gcagagccgc agccgggtgg cagccagtgc gtcgagcacg actgcttcgc gctctacccg  120
ggccccgcga ccttcctcaa tgccagtcag atctgcgacg gactgcgggg ccacctaatg  180
acagtgcgct cctcggtggc tgccgatgtc atttccttgc tactgaacgg cgacggcggc  240
gttggccgcc ggcgcctctg gatcggcctg cagctgccac ccggctgcgg cgaccccaag  300
cgcctcgggc ccctgcgcgg cttccagtgg gttacgggag acaacaacac cagctatagc  360
aggtgggcac ggctcgacct caatggggct cccctctgcg gcccgttgtg cgtcgctgtc  420
tccgctgctg aggccactgt gcccagcgag ccgatctggg aggagcagca gtgcgaagtg  480
aaggccgatg gcttcctctg cgagttccac ttcccagcca cctgcaggcc actggctgtg  540
gagcccggcg ccgcggctgc cgccgtctcg atcacctacg gcaccccgtt cgcggcccgc  600
ggagcggact tccaggcgct gccggtgggc agctccgccg cggtggctcc cctcggctta  660
cagctaatgt gcaccgcgcc gcccggagcg gtccaggggc actgggccag ggaggcgccg  720
ggcgcttggg actgcagcgt ggagaacggc ggctgcgagc acgcgtgcaa tgcgatccct  780
ggggctcccc gctgccagtg cccagccggc gccgccctgc aggcagacgg gcgctcctgc  840
accgcatccg cgacgcagtc ctgcaacgac ctctgcgagc acttctgcgt tcccaacccc  900
gaccagccgg gctcctactc gtgcatgtgc gagaccggct accggctggc ggccgaccaa  960
caccggtgcg aggacgtgga tgactgcata ctggagccca gtccgtgtcc gcagcgctgt 1020
```

gtcaacacac agggtggctt cgagtgccac tgctacccta actacgacct ggtggacggc 1080

gagtgtgtgg agcccgtgga cccgtgcttc agagccaact gcgagtacca gtgccagccc 1140

ctgaaccaaa ctagctacct ctgcgtctgc gccgagggct tcgcgcccat tccccacgag 1200

ccgcacaggt gccagatgtt ttgcaaccag actgcctgtc cagccgactg cgaccccaac 1260

acccaggcta gctgtgagtg ccctgaaggc tacatcctgg acgacggttt catctgcacg 1320

gacatcgacg agtgcgaaaa cggcggcttc tgctccgggg tgtgccacaa cctccccggt 1380

accttcgagt gcatctgcgg gcccgactcg gcccttgtcc gccacattgg caccgac· t 1440

gactccggca aggtggacgg tggcgacagc ggctctggcg agcccccgcc cagcccgacg 1500

cccggctcca ccttgactcc tccggccgtg gggctcgtgc attcgggc 1548

<210> 3
<211> 132
<212> PRT
<213> Artificial sequence

<220>
<223> Partial amino acid sequence of human-originated soluble thrombomodulin

<400> 8

Met Leu Gly Val Leu Val Leu Gly Ala Leu Ala Leu Ala Gly Leu Gly

1 5 10 15

Phe Pro Asp Pro Cys Phe Arg Ala Asn Cys Glu Tyr Gln Cys Gln Pro

20 25 30

Leu Asn Gln Thr Ser Tyr Leu Cys Val Cys Ala Glu Gly Phe Ala Pro

35 40 45

Ile Pro His Glu Pro His Arg Cys Gln Met Phe Cys Asn Gln Thr Ala

50 55 60

Cys Pro Ala Asp Cys Asp Pro Asn Thr Gln Ala Ser Cys Glu Cys Pro

65 70 75 80

Glu Gly Tyr Ile Leu Asp Asp Gly Phe Ile Cys Thr Asp Ile Asp Glu

85 90 95

Cys Glu Asn Gly Gly Phe Cys Ser Gly Val Cys His Asn Leu Pro Gly

100 105 110

Thr Phe Glu Cys Ile Cys Gly Pro Asp Ser Ala Leu Val Arg His Ile

115 120 125

Gly Thr Asp Cys

130

<210> 4
<211> 396
<212> DNA
<213> Artificial sequence

<220>
<223> Partial base sequence of human-originated soluble thrombomodulin gene

<400> 4

```
atgcttgggg tcctggtcct tggcgcgctg gccctggccg gcctggggtt ccccgacccg   60
tgcttcagag ccaactgcga gtaccagtgc cagcccctga accaaactag ctacctctgc  120
gtctgcgccg agggcttcgc gcccattccc cacgagccgc acaggtgcca gatgttttgc  180
aaccagactg cctgtccagc cgactgcgac cccaacaccc aggctagctg tgagtgccct  240
gaaggctaca tcctggacga cggtttcatc tgcacggaca tcgacgagtg cgaaaacggc  300
ggcttctgct ccggggtgtg ccacaacctc cccggtacct tcgagtgcat ctgcgggccc  360
gactcggccc ttgtccgcca cattggcacc gactgt                            396
```

<210> 5
<211> 516
<212> PRT
<213> Artificial sequence

<220>
<223> Partial amino acid sequence of human-originated soluble thrombomodulin

<400> 5

Met Leu Gly Val Leu Val Leu Gly Ala Leu Ala Leu Ala Gly Leu Gly
1 5 10 15

Phe Pro Ala Pro Ala Glu Pro Gln Pro Gly Gly Ser Gln Cys Val Glu
20 25 30

His Asp Cys Phe Ala Leu Tyr Pro Gly Pro Ala Thr Phe Leu Asn Ala
35 40 45

Ser Gln Ile Cys Asp Gly Leu Arg Gly His Leu Met Thr Val Arg Ser
50 55 60

Ser Val Ala Ala Asp Val Ile Ser Leu Leu Leu Asn Gly Asp Gly Gly
65 70 75 80

Val Gly Arg Arg Arg Leu Trp Ile Gly Leu Gln Leu Pro Pro Gly Cys
85 90 95

Gly Asp Pro Lys Arg Leu Gly Pro Leu Arg Gly Phe Gln Trp Val Thr
100 105 110

Gly Asp Asn Asn Thr Ser Tyr Ser Arg Trp Ala Arg Leu Asp Leu Asn
115 120 125

Gly Ala Pro Leu Cys Gly Pro Leu Cys Val Ala Val Ser Ala Ala Glu
130 135 140

Ala Thr Val Pro Ser Glu Pro Ile Trp Glu Glu Gln Gln Cys Glu Val
145 150 155 160

Lys Ala Asp Gly Phe Leu Cys Glu Phe His Phe Pro Ala Thr Cys Arg
165 170 175

Pro Leu Ala Val Glu Pro Gly Ala Ala Ala Ala Ala Val Ser Ile Thr
180 185 190

Tyr Gly Thr Pro Phe Ala Ala Arg Gly Ala Asp Phe Gln Ala Leu Pro
195 200 205

Val Gly Ser Ser Ala Ala Val Ala Pro Leu Gly Leu Gln Leu Met Cys
210 215 220

Thr Ala Pro Pro Gly Ala Val Gln Gly His Trp Ala Arg Glu Ala Pro
225 230 235 240

Gly Ala Trp Asp Cys Ser Val Glu Asn Gly Gly Cys Glu His Ala Cys
245 250 255

Asn Ala Ile Pro Gly Ala Pro Arg Cys Gln Cys Pro Ala Gly Ala Ala
260 265 270

Leu Gln Ala Asp Gly Arg Ser Cys Thr Ala Ser Ala Thr Gln Ser Cys
275 280 285

Asn Asp Leu Cys Glu His Phe Cys Val Pro Asn Pro Asp Gln Pro Gly
290 295 300

Ser Tyr Ser Cys Met Cys Glu Thr Gly Tyr Arg Leu Ala Ala Asp Gln
305 310 315 320

His Arg Cys Glu Asp Val Asp Asp Cys Ile Leu Glu Pro Ser Pro Cys
325 330 335

Pro Gln Arg Cys Val Asn Thr Gln Gly Gly Phe Glu Cys His Cys Tyr
340 345 350

Pro Asn Tyr Asp Leu Val Asp Gly Glu Cys Val Glu Pro Val Asp Pro
355 360 365

Cys Phe Arg Ala Asn Cys Glu Tyr Gln Cys Gln Pro Leu Asn Gln Thr
370 375 380

Ser Tyr Leu Cys Val Cys Ala Glu Gly Phe Ala Pro Ile Pro His Glu

```
385                 390                 395                 400
Pro His Arg Cys Gln Met Phe Cys Asn Gln Thr Ala Cys Pro Ala Asp
                405                 410                 415
Cys Asp Pro Asn Thr Gln Ala Ser Cys Glu Cys Pro Glu Gly Tyr Ile
            420                 425                 430
Leu Asp Asp Gly Phe Ile Cys Thr Asp Ile Asp Glu Cys Glu Asn Gly
        435                 440                 445
Gly Phe Cys Ser Gly Val Cys His Asn Leu Pro Gly Thr Phe Glu Cys
    450                 455                 460
Ile Cys Gly Pro Asp Ser Ala Leu Ala Arg His Ile Gly Thr Asp Cys
465                 470                 475                 480
Asp Ser Gly Lys Val Asp Gly Gly Asp Ser Gly Ser Gly Glu Pro Pro
                485                 490                 495
Pro Ser Pro Thr Pro Gly Ser Thr Leu Thr Pro Pro Ala Val Gly Leu
            500                 505                 510
Val His Ser Gly
        515
```

<210> 6
<211> 1548
<212> DNA
<213> Artificial sequence

<220>
<223> Partial base sequence of human-originated soluble thrombomodulin gene

<400> 6

```
atgcttgggg tcctggtcct tggcgcgctg gccctggccg gcctggggtt ccccgcaccc   60
```

gcagagccgc agccgggtgg cagccagtgc gtcgagcacg actgcttcgc gctctacccg 120
ggccccgcga ccttcctcaa tgccagtcag atctgcgacg gactgcgggg ccacctaatg 180
acagtgcgct cctcggtggc tgccgatgtc atttccttgc tactgaacgg cgacggcggc 240
gttggccgcc ggcgcctctg gatcggcctg cagctgccac ccggctgcgg cgaccccaag 300
cgcctcgggc ccctgcgcgg cttccagtgg gttacgggag acaacaacac cagctatagc 360
aggtgggcac ggctcgacct caatggggct cccctctgcg gcccgttgtg cgtcgctgtc 420
tccgctgctg aggccactgt gcccagcgag ccgatctggg aggagcagca gtgcgaagtg 480
aaggccgatg gcttcctctg cgagttccac ttcccagcca cctgcaggcc actggctgtg 540
gagcccggcg ccgcggctgc cgccgtctcg atcacctacg gcaccccgtt cgcggcccgc 600
ggagcggact tccaggcgct gccggtgggc agctccgccg cggtggctcc cctcggctta 660
cagctaatgt gcaccgcgcc gcccggagcg gtccaggggc actgggccag ggaggcgccg 720
ggcgcttggg actgcagcgt ggagaacggc ggctgcgagc acgcgtgcaa tgcgatccct 780
ggggctcccc gctgccagtg cccagccggc gccgccctgc aggcagacgg gcgctcctgc 840
accgcatccg cgacgcagtc ctgcaacgac ctctgcgagc acttctgcgt tcccaacccc 900
gaccagccgg gctcctactc gtgcatgtgc gagaccggct accggctggc ggccgaccaa 960
caccggtgcg aggacgtgga tgactgcata ctggagccca gtccgtgtcc gcagcgctgt 1020
gtcaacacac agggtggctt cgagtgccac tgctacccta actacgacct ggtggacggc 1080
gagtgtgtgg agcccgtgga cccgtgcttc agagccaact gcgagtacca gtgccagccc 1140
ctgaaccaaa ctagctacct ctgcgtctgc gccgagggct tcgcgcccat tccccacgag 1200
ccgcacaggt gccagatgtt ttgcaaccag actgcctgtc cagccgactg cgaccccaac 1260
acccaggcta gctgtgagtg ccctgaaggc tacatcctgg acgacggttt catctgcacg 1320
gacatcgacg agtgcgaaaa cggcggcttc tgctccgggg tgtgccacaa cctccccggt 1380
accttcgagt gcatctgcgg gcccgactcg gcccttgccc gccacattgg caccgactgt 1440
gactccggca aggtggacgg tggcgacagc ggctctggcg agcccccgcc cagcccgacg 1500
cccggctcca ccttgactcc tccggccgtg ggctcgtgc attcgggc 1548

<210> 7
<211> 132
<212> PRT
<213> Artificial sequence

<220>
<223> Partial amino acid sequence of human-originated soluble

thrombomodulin

<400> 7

Met Leu Gly Val Leu Val Leu Gly Ala Leu Ala Leu Ala Gly Leu Gly
1               5                   10                  15

Phe Pro Asp Pro Cys Phe Arg Ala Asn Cys Glu Tyr Gln Cys Gln Pro
            20                  25                  30

Leu Asn Gln Thr Ser Tyr Leu Cys Val Cys Ala Glu Gly Phe Ala Pro
        35                  40                  45

Ile Pro His Glu Pro His Arg Cys Gln Met Phe Cys Asn Gln Thr Ala
    50                  55                  60

Cys Pro Ala Asp Cys Asp Pro Asn Thr Gln Ala Ser Cys Glu Cys Pro
65                  70                  75                  80

Glu Gly Tyr Ile Leu Asp Asp Gly Phe Ile Cys Thr Asp Ile Asp Glu
                85                  90                  95

Cys Glu Asn Gly Gly Phe Cys Ser Gly Val Cys His Asn Leu Pro Gly
            100                 105                 110

Thr Phe Glu Cys Ile Cys Gly Pro Asp Ser Ala Leu Ala Arg His Ile
        115                 120                 125

Gly Thr Asp Cys
    130

<210> 8
<211> 396
<212> DNA
<213> Artificial sequence

<220>
<223> Partial base sequence of human-originated soluble thrombomodulin gene

<400> 8

atgcttgggg tcctggtcct tggcgcgctg gccctggccg gcctggggtt ccccgacccg 60

tgcttcagag ccaactgcga gtaccagtgc cagcccctga accaaactag ctacctctgc 120

gtctgcgccg agggcttcgc gcccattccc cacgagccgc acaggtgcca gatgttttgc 180

aaccagactg cctgtccagc cgactgcgac cccaacaccc aggctagctg tgagtgccct 240

gaaggctaca tcctggacga cggtttcatc tgcacggaca tcgacgagtg cgaaaacggc 300

ggcttctgct ccggggtgtg ccacaacctc cccggtacct tcgagtgcat ctgcgggccc 360

gactcggccc ttgcccgcca cattggcacc gactgt 396

<210> 9
<211> 21
<212> DNA
<213> Artificial sequence

<220>
<223> Synthetic DNA for mutation

<400> 9
aatgtggcgg gcaagggccg a 21

**Description**

TECHNICAL FIELD

The present invention relates to a foam-inhibiting method in preparing a high-concentrated soluble thrombomodulin-containing solution by dissolving of a soluble thrombomodulin-containing freeze-dried preparation, a method of stabilizing soluble thrombomodulin in the freeze-dried preparation, and a soluble thrombomodulin-containing freeze-dried preparation suitable therefor and a kit preparation thereof.

BACKGROUND ART

Thrombomodulin is a substance found to have functions of bonding specifically to thrombin and promoting the activation of protein C by thrombin remarkably. Protein C consists of a vitamin K-dependent protein which plays an important role in the coagulation fibrinolysis system and is activated by the action of thrombin into activated protein C. It has been known that activated protein C inactivates the activated coagulation factors V and VIII in the blood coagulation system of a living body and that it participates in the production of a plasminogen activator which has a thrombolytic function [see Koji Suzuki, "Igaku-no Ayumi (Journal of Clinical and Experimental Medicine)", Vol. 125, 901 (1983)].

Thus, it has been recognized that thrombomodulin promotes activation of protein C and is useful as an anticoagulant or as a thrombolytic agent. Thrombomodulin has been conventionally expected to be used for therapy and prophylaxis of diseases, such as acute coronary syndrome (ACS), thrombosis, peripheral vascular obstruction, arteriosclerosis obliterans, angiitis, functional disorders that follow a cardiac surgery, complications that follow an organ transplantation, disseminated intravascular coagulation (DIC), angina pectoris, transient cerebral ischemic attack, gestational toxicosis, diabetes, liver veno-occlusive diseases (VOD) (such as fulminant hepatitis or liver veno-occlusive diseases following bone marrow transplantation), deep venous thrombosis (DVT), and also sepsis and adult respiratory distress syndrome (ARDS).

Conventionally, thrombomodulin was found and obtained as a glycoprotein expressed on the vascular endothelial cells of various animal species, including human. After which, cloning thereof was achieved. In other words, genes of a human thrombomodulin precursor having a signal peptide were cloned from the human lung cDNA library using a genetic engineering technique, whereafter the entire gene sequence of the thrombomodulin was analyzed and its amino acid sequence having 575 residues including a signal peptide of 18 amino acid residues was made clear (see JP 64-006219 A). It has been known that a mature thrombomodulin with the signal peptide being cut is composed of five domains, namely, the N-terminal domain (1st to 226th amino acid residue, in the order from the N-terminus), the domain having 6 EGF-like structures (227th to 462nd residue), the O-glycosylation site-rich domain (463rd to 498th residue), the transmembrane domain (499th to 521st residue) and the intracytoplasmic domain (522nd to 557th residue), enumerated respectively in the sequential order from the N-terminus of the mature peptide. The segment exhibiting the same activity as the full length thrombomodulin is mainly composed of the segment consisting of the fourth, fifth, and sixth EGF-like structures as enumerated in the order from the N-terminus in the domain having 6 EGF-like structures (see M. Zushi et al., J. Biol. Chem., 246, 10351 - 10353 (1989)).

It has been confirmed that at least such soluble thrombomodulin which is prepared so as not to contain the transmembrane domain can dissolve well even in the absence of a surfactant and that, for example, soluble thrombomodulin composed of only the three domains, i.e. the N-terminal domain, the domain having 6 EGF-like structures, and the O-glycosylation site-rich domain (namely, composed of the amino acid sequence from the 19th to 516th amino acid residues of the sequence listing SEQ ID NO.1) can be obtained by applying a gene recombination technology. Further, it was confirmed that the recombinant soluble thrombomodulin has an activity comparable to that of natural thrombomodulin (JP 64-006219 A). Further examples of soluble thrombomodulin may include: the soluble thrombomodulin described in JP 02-255699 A, JP 03-133380 A, JP 03-259084 A, JP 04-210700 A, JP 05-213998 A, and WO 92/00325, WO 92/03149, and WO 93/15755; and also include a soluble thrombomodulin derived from human urine as example of a nature type (see JP 03-086900 A, and JP 03-218399 A).

In this connection, as recognized in many cases, genes may be induced a natural mutation and artificial mutation during their obtainment, without exception for human genes in which also a polymorphic mutation had been discovered, wherein two mutants of the human thrombomodulin precursor constituted of the amino acid sequence of the above-mentioned 575 amino acid residues, were currently confirmed. The amino acid residue at the 473rd site consists of Val, for the one, and this amino acid residue consists of Ala, for the other. This corresponds, in the base sequence encoding such amino acids, to mutations of T and C of the 1418th site, respectively (Wenetal., Biochemistry, 26, 4350-4357 (1987)). The mutations have, however, no difference in the activity and in the physical properties and can be judged to be substantially the same.

UK Patent Application GB 2015340 A discloses a method to inhibit foaming of a protein preparation in a sealed container under a reduced pressure of -47995,92 Pa to 87992,52 Pa.

DISCLOSURE OF THE INVENTION

For using soluble thrombomodulin as pharmaceutical compositions, various disorders for treatment and various medication methods are known. Development of pharmaceuticals adapted to all of these disorders for treatment and medication methods have been waited for.

For solving the above problems, at first, the inventors of the present invention felt the necessity for a high-concentrated soluble-thrombomodulin preparation and decided to prepare it. It is known that soluble thrombomodulin is prepared into a freeze-dried preparation when the soluble thrombomodulin is supplied widely and stably as a pharmaceutical composition. The inventors of the present invention have confirmed for the first time that when a high-concentrated soluble-thrombomodulin solution is prepared by re-dissolving the freeze-dried preparation in water for injection or the like, innumerable minute air bubbles generate in the solution but hardly disappear and in some cases the solution may have a clouded appearance. Of course, the soluble thrombomodulin solution cannot be administered while containing air bubbles when using in medical situation. It takes much time uselessly to leave the solution until the air bubbles disappear and the solution becomes clear, which causes a problem in quality as a pharmaceutical preparation. The inventors of the present invention found such a problemnever expected in the past and dedicated in studies to solve the problem. Finally, the inventors of the present invention have completed the present invention as a result of confirming the fact that the generation of air bubbles can be inhibited in a preparation containing a non-ionic surfactant, benzyl alcohol, or chlorobutanol, as a foam-inhibition additive, in a high-concentrated soluble thrombomodulin-containing solution. In addition, the inventors of the present invention have completed the present invention as a result of confirming the fact that the generation of air bubbles can be inhibited also by coating the inner wall of a container to be used in dissolving a freeze-dried preparation containing the soluble thrombomodulin with silicone or retaining the pressure in the container at a reduced pressure at the time of dissolving the freeze-dried preparation containing the soluble thrombomodulin.

That is, according to the present invention, there is provided a method of inhibiting foaming of a high-concentrated soluble thrombomodulin-containing solution, which is used in preparing a high-concentrated soluble thrombomodulin-containing solution having a concentration of 10 mg/mL or more by dissolving a soluble thrombomodulin-containing freeze-dried preparation that contains soluble thrombomodulin as an active ingredient, the method being characterized by including at least one of : (a) the presence of at least one compound selected from the group consisting of a nonionic surfactant, benzyl alcohol, and chlorobutanol; (b) the use of a container having the inner wall coated with silicone, said container being used in dissolving the soluble thrombomodulin-containing freeze-dried preparation; or (c) a pressure in the container to be used in dissolving the soluble thrombomodulin-containing freeze-dried preparation is kept at a reduced pressure, reduced by -13332.2 Pa to -39996 Pa (-100 to -300 mm Hg).

Furthermore, there is provided a foam inhibitor for a high-concentrated soluble thrombomodulin-containing solution to be used in preparing a high-concentrated soluble thrombomodulin-containing solution having a concentration of 10 mg/mL or more, preferably 20 mg/mL or more by dissolving a soluble thrombomodulin-containing freeze-dried preparation that contains soluble thrombomodulin as an active ingredient, the form inhibitor containing at least one compound selected from the group consisting of a nonionic surfactant, benzyl alcohol, and chlorobutanol as an active ingredient.

Furthermore, according to the present invention, there is provided a soluble thrombomodulin-containing freeze-dried preparation containing soluble thrombomodulin as an active ingredient or a kit preparation which is a combination between the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more, and the soluble thrombomodulin-containing freeze-dried preparation or the kit preparation thereof being characterized by at least one of (a) that at least one compound selected from the group consisting of a nonionic surfactant, benzyl alcohol, and chlorobutanol is present in the soluble thrombomodulin-containing freeze-dried preparation and/or at least one compound selected from the group consisting of a nonionic surfactant, benzyl alcohol, and chlorobutanol is present in the dissolving aqueous solution for the soluble thrombomodulin-containing freeze-dried preparation, (b) that an inner wall of a container to be used in dissolving the soluble thrombomodulin-containing freeze-dried preparation is coated with silicone, or (c) that the pressure in the container to be used in dissolving the soluble thrombomodulin-containing freeze-dried preparation is at a reduced pressure, reduced by -13332.2 Pa to -39996.6 Pa (-100 to -300 mm Hg).

Furthermore, the inventors of the present invention have investigated the problems involved in the conventional technology in preparing a high-concentrated soluble thrombomodulin preparation. In other words, for preparing a high-concentrated soluble-thrombomodulin preparation which can be widely applied to various disorders for treatment and various medication methods, intramuscular or subcutaneous administration of a certain amount or more of a solution is difficult for adopting the preparation to a medication method of, particularly, intramuscular administration or subcutaneous administration, for example. Besides, the detailed studies conducted by the inventors of the present invention confirmed that an excessively low or high osmotic pressure of the administered solution undesirably caused strong pain.

On the other hand, it has been clarified that a multimer polymerized by denaturation, aggregation, or the like can be formed by subjecting a soluble thrombomodulin-containing solution to freeze-drying during the step of subjecting thrombomodulin to freeze-drying. Conventionally, the addition of one or two or more selected from the group consisting of amino acids or salts thereof and sugars has been known as a method of preventing denaturation of soluble thrombomodulin (JP 06-321805 A). However, the studies of the inventors of the present invention confirmed that the composition having the additive and the addition amount thereof specifically disclosed in above JP 06-321805 A may not always be preferable in terms of pain or the like. That is, experiments conducted by the inventors of the present invention confirmed that the pain was caused by the composition (e.g., Example 20) specifically disclosed in JP 06-321805 A prepared into a high-concentrated soluble thrombomodulin-containing solution in which 10 mg or more of soluble thrombomodulin was dissolved in a 0.1 mL to 2 mL aqueous solution, concerning subcutaneous or intramuscular administration.

Furthermore, the addition of maltose, lactose, sucrose, or arginine in soluble thrombomodulin has been also known (WO 95/16460) . However, WO 95/16460 describes that the addition of mannitol cannot prevent the denaturation of soluble thrombomodulin. Therefore, the mannitol was not necessarily conceived as a preferable additive for the persons skilled in the art. The inventors of the present invention have dedicated in studies and found out that, for example, the coexistence of glutamic acid or a salt thereof and mannitol cause a remarkable synergistic effect of attaining extremely high stability, compared with the case in which mannitol is solely added, even though WO 95/16460 describes that mannitol is undesirable. Therefore, the studies conducted by the inventors of the present invention have confirmed the fact that preferable stability can also be attained at concentration allowing suitable adjustment of an osmotic pressure ratio and in addition that a preparation hardly causing pain can be obtained even in subcutaneous or intramuscular administration, finally completing the present invention as described below.

That is, according to the present invention, there is provided a method of stabilizing soluble thrombomodulin in a soluble thrombomodulin-containing freeze-dried preparation that contains the soluble thrombomodulin as an active ingredient, and comprising at least one combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; and dissolving the soluble thrombomodulin-containing freeze-dried preparation in 0.1 mL to 2 mL of a dissolving aqueous solution to prepare a high-concentrated soluble thrombomodulin-containing solution having a concentration of 10 mg/mL or more with an osmotic pressure ratio calculated by dividing the osmolality of the thrombomodulin-containing solution by the osmolality of a physiological saline, i.e. 286 m Osm, in the range of 0.5 to 2.0 upon dissolution thereof.

## BEST MODE FOR CARRYING OUT THE INVENTION

Hereinafter, the present invention will be described in detail.

The present invention relates to the following foam-inhibition method or foam inhibitor. That is , according to the present invention, there is provided a method of inhibiting foaming of a high-concentrated soluble thrombomodulin-containing solution, which is used in preparing a high-concentrated soluble thrombomodulin-containing solution having a concentration of 10 mg/mL or more by dissolving a soluble thrombomodulin-containing freeze-dried preparation that contains soluble thrombomodulin as an active ingredient, the method being characterized by including at least one of: (a) the presence of at least one compound selected from the group consisting of a nonionic surfactant, benzyl alcohol, and chlorobutanol; (b) the use of a container having the inner wall coated with silicone, said container being used in dissolving the soluble thrombomodulin-containing freeze-dried preparation; and (c) a pressure in the container to be used in dissolving the soluble thrombomodulin-containing freeze-dried preparation is kept at a reduced pressure, reduced by -13332.2 Pa to -39996.6 Pa (-100 to -300 mmHg).

Means for foam inhibition in the high-concentrated soluble thrombomodulin-containing solution is preferably one of (a), (b), and (c) described above. Of those, a preferable example is (a) or alternatively the combination of (a) and (c) or of (a) and (b). In addition, the combination of (b) and (c) is another preferable example, and the combination of (a), (b), and (c) is a particularly preferable example.

First, the above (a) will be described.

Specifically, according to the present invention, there is provided a method of inhibiting foaming of a high-concentrated soluble thrombomodulin-containing solution, which is used in preparing a high-concentrated soluble thrombomodulin-containing solution having a concentration of 10 mg/mL or more by dissolving the soluble thrombomodulin-containing freeze-dried preparation, the method being characterized by including the presence of at least one compound selected from the group consisting of a nonionic surfactant, benzyl alcohol, and chlorobutanol.

More specifically, according to the present invention, there is provided a method of inhibiting foaming of a high-concentrated soluble thrombomodulin-containing solution, characterized by the presence of at least one compound selected from the group consisting of a nonionic surfactant, benzyl alcohol, and chlorobutanol in the soluble thrombomodulin-containing freeze-dried preparation or by the presence of at least one compound selected from the group consisting of a nonionic surfactant, benzyl alcohol, and chlorobutanol in a dissolving aqueous solution for dissolving the soluble thrombomodulin-containing freeze-dried preparation.

The soluble thrombomodulin of the present invention is not specifically limited as far as the soluble thrombomodulin is a substance having functions of bonding to thrombin and promoting the activation of protein C with thrombin and which can be easily dissolved even in the absence of surfactant with. In addition, persons skilled in the art recognize that the soluble thrombomodulin has a function of extending coagulation time with thrombin and/or inhibiting platelet agglutination with thrombin. A preferred example of the solubility of soluble thrombomodulin is: 1 mg/mL or more, or 10 mg/mL or more to water, for example, distilled water for injection (in the absence of a surfactant such as Triton X-100 or Polidocanol typically at approximately neutral) ; preferably 15 mg/mL or more, or 17 mg/mL or more; more preferably 20 mg/mL or more, 25 mg/mL or more, or 30 mg/mL or more; and particularly preferably 60 mg/mL or more. In some cases, the solubility is 80mg/mL or more, or 100 mg/mL or more. For determining whether soluble thrombomodulin is dissolved or not, the mixture after dissolution may be observed with the eye at a position located directly below a white light source with a brightness of approximately 1,000 lux. It can be understood that a direct index for the dissolved soluble thrombomodulin is a clear solution without the presence of any insoluble substance which can be distinctly recognized. In addition, the presence or absence of a residual substance after filtration can be confirmed. The determination of the dissolution can be also used as a confirming method at the time of dissolving together with additives such as a foam-inhibition additive, a stabilization additive, and other additives which will be described later.

Furthermore, the function of promoting the activation of protein C by thrombin can be easily confirmed, that is, the activity level of the function promoting the activation of protein C or the presence or absence of such a function can be confirmed by using test procedures clearly described in various publications including JP 64-006219 A, for example. In addition, the function of extending coagulation time with thrombin and/or the function of inhibiting platelet agglutination can be also confirmed likewise. Preferable examples of the soluble thrombomodulin include a peptide obtained from human using human urine or the like as a raw material and a variant or derivative of gene or DNA originated from human. Preferably, the soluble thrombomodulin includes an amino acid sequence at positions 19 to 132 of SEQ ID NO. 3 in the peptide. In this case, of course, the properties of soluble thrombomodulin as described above are required. The amino acid sequence at positions 19 to 132 of SEQ ID NO. 3 may be naturally or artificially varied as far as the soluble thrombomodulin retains its actions of promoting the activation of protein C with thrombin and so on. That is, one or more amino acid sequences, in other words, one or several amino acid sequences, preferably several amino acid sequences may be subjected to substitution, deletion, and addition. For example, a peptide consisting of an amino acid sequence where the amino acid sequence at positions 19 to 132 of SEQ ID NO. 7, i.e., Val at position 125 in the amino acid sequence of SEQ ID NO. 3 in the sequence listing is substituted with Ala or a peptide including such a sequence can be also used in the present invention. The degree of acceptable mutation is not specifically limited as far as the activity retains. For instance, the exemplified amino acid sequence has a homology of 50% or more, preferably 70% or more, particularly preferably 80% or more, more preferably 90% or more, or 95% or more, and most preferably 98% or more. As described below, these mutations can be easily obtained using conventional gene manipulation techniques.

Furthermore, an example of the soluble thrombomodulin that can be used in the present invention may be a peptide obtained from a transformed cell prepared by transfection of DNA encoding the amino acid sequence of SEQ ID NO. 1 to a host cell through a vector. A preferable example of the peptide obtained from the transformed cell may include the peptide consisting of an amino acid sequence at positions 19 to 516 of SEQ ID NO. 1. As described above, the amino acid sequence at positions 19 to 516 of SEQ ID NO. 1 may be naturally or artificially varied as far as the soluble thrombomodulin retains its actions of promoting the activation of protein C with thrombin. That is, one or more amino acid sequences at positions 19 to 516 of SEQ ID NO. 1 in the sequence listing, i.e., one or several amino acids more preferably several amino acids may be subjected to substitution, deletion, and addition. For instance, a peptide consisting of an amino acid sequence where Val in the amino acid sequence at positions 19 to 516 of SEQ ID NO. 5, i.e., Val at a position of 473 in the amino acid sequence of SEQ ID NO. 1 is substituted with Ala, can be used in the present invention. Depending on a host cell, in addition, there is produced a peptide in which a signal portion remains as it is or a peptide consisting of the amino acid sequence at positions 17 to 516 of SEQ ID NO. 1 or SEQ ID NO. 5 in the sequence listing described above, a mixture thereof, or the like, and any thereof may be used for the present invention. As a matter of course, the peptides have extremely high solubility, satisfying sufficiently the solubility described above. According to the present invention, the peptide consisting of the amino acid sequence at positions 19 to 516 or at positions 17 to 516 of SEQ ID NO. 1 in the above sequence listing is particularly preferable.

Furthermore, it is also preferable that soluble thrombomodulin is a peptide encoded by DNA which can be hybridized with complementary DNA having a base sequence at positions 55 to 396 of SEQ ID NO. 4 or 8 in the sequence listing under stringent conditions, and the soluble thrombomodulin has a function of promoting the activation of protein C with thrombin and can be dissolved in the absence of a surfactant. The stringent conditions refer to the conditions allowing the complementary DNA to specifically hybridize with the target DNA, for example, conditions allowing the complementary DNA hybridize with DNA, RNA, or synthetic DNA and then washing away the complementary DNA non-specifically hybridized, other than the target DNA. For instance, the stringent conditions can be determined with reference to Sambrook et al. (Molecular Cloning, 2nd Ed., Cold Spring Harbor Laboratory Press, 1989, Cold Spring Harbor, NY). For example, the conditions allow hybridization at 42°C in 6 × SSC, 5 × Denhardt's reagent, 0.5% SDS, 100 μg/mL denatured, fragmented salmon sperm DNA, and 50% formamide, and washing at 68°C with 0.1 × SSC and 0.5% SDS.

Furthermore, the peptides only need to have the amino acid sequence described above, so that a sugar chain may or may not be bonded to the peptides, and the peptides are not specifically limited in terms of sugar chains. In the gene manipulation, the type of sugar chain, and the position and degree of addition vary depending on the type of host cells used. The peptides containing any type of sugar chain at any position and degree can be used. As described later, the peptides are not specifically obtained through gene manipulation. However, in the case of obtaining the peptide through gene manipulation, examples of a signal sequence which can be used at the time of expression include: a base sequence consisting of the amino acid sequence at positions 1 to 18 of SEQ ID NO. 1 in the above sequence listing; and other known signal sequences such as a signal sequence from a human tissue type plasminogen activator (tPA) (see WO 88/9811).

Methods of introducing a soluble thrombomodulin-encoding DNA sequence into a host cell include a method of incorporating the soluble thrombomodulin-encoding DNA sequence into preferably a vector, particularly preferably an expression vector which can be expressed in an animal cell. The expression vector is a DNA molecule including a promoter sequence, a sequence providing a ribosome bonding site to mRNA, a DNA sequence that encodes a protein desired to be expressed, a splicing signal, a terminator sequence for transcription termination, a replication origin sequence. Preferable animal-cell expression vectors include: pSV2-X reported by R. C. Mulligan et al. [Proc. Natl. Acad. Sci., U.S.A. 78, 2072 (1981)]; and pBP69T(69-6) reported by P. M. Howley et al. [Method by Enzymology, 101, 387, Academic Press (1983)].

Examples of the host cell which may be used in the manufacture of the peptides include: cells of such as Chinese hamster ovary (CHO), COS-1, COS-7, VERO (ATCC CCL-81), BHK, canine kidney-originated MDCK, and hamster AV-12-664; and human-originated cells of such as HeLa, WI38, and human 293. As the CHO cell, DHFR-CHO cell is more preferable.

In the process of gene manipulation or manufacturing peptide, various microorganisms such as Escherichia coli are employed frequently, wherein a suitable host-vector system adapted for each purpose is preferably used, and thus, an adequate vector system can be selected also for each of the above host cells. The gene of soluble thrombomodulin used for the genetic recombination technique has been cloned, and production examples using the genetic recombination technique for soluble thrombomodulin and purification methods for obtaining a purified product thereof have been disclosed (see JP 64-006219 A, JP 02-255699 A, JP 05-213998 A, JP 05-310787 A, JP 07-155176 A, and J. Biol. Chem. , 264, 10351 - 10353 (1989)). Therefore, the soluble thrombomodulin according to the present invention can be manufactured using the methods described in the above-cited documents or following a technique corresponding thereto. For example, there is disclosed in JP 64-006219 A, Escherichia coli K-12 strain DH5 (ATCC Deposition No. 67283) having the plasmid pSV2TMJ2 containing the DNA encoding the full-length thrombomodulin. Further, a redeposited strain of the above strain (Escherichia coli DH5/pSV2TMJ2) (FERM BP-5570) at the National Institute of Bioscience and Human-Technology (current National Institute of Advanced Industrial Science and Technology, International Patent Organism Depositary) may also be used. Using this DNAas the starting material which encodes the full-length thrombomodulin, the soluble thrombomodulin according to the present invention can be prepared by a known gene manipulation technique.

While the soluble thrombomodulin used in the present invention may be prepared by a conventionally known method or a technique corresponding thereto, it is possible to refer to the documents of, for example, Yamamoto et al. (JP 64-006219 A,), and JP 05-213998 A, described above. In other words, the DNA encoding the amino acid sequence of the sequence listing SEQ ID NO. 1 can be obtained from a gene of a human-originated soluble thrombomodulin thorough a gene manipulation technique and, if necessary, the DNA can also be denatured. Such denaturation may include a site-specific mutation in accordance with the method described in Method in Enzymology, 100, 468 (1983), Academic Press. For example, the base T of the position 1418 of SEQ ID NO. 2 may be converted, to thereby obtain DNA conducting the base C using a synthetic DNA for mutation.

The thus-prepared DNA can then be integrated into, for example, Chinese hamster ovary (CHO) cells to obtain corresponding transformed cells. The cells are suitably selected and cultured, and the soluble thrombomodulin purified by a known technique can be manufactured from the culture mixture. As mentioned previously, the DNA which encodes the amino acid sequence of the sequence listing SEQ ID NO. 1 is preferably transfected to the host cell. The method of manufacturing the soluble thrombomodulin used in the present invention is not restricted only to the above-described methods but many alternative methods are possible including: extracting and purifying from urine, blood, and other body fluids, for example; extracting and purifying from a tissue producing the soluble thrombomodulin, or from a tissue culturemixture thereof; and further digesting by a proteolytic enzyme, if necessary.

The isolation and purification of the soluble thrombomodulin from the supernatant or from the culture mixture obtained by the above technique can be performed in accordance with a known technique (Fundamental Experimental Methods for Proteins, Enzymes, edited by Buichi Horio). Use of ion-exchange chromatography or adsorption chromatography based on the interaction between a chromatography carrier on which a functional group with ionic charge reverse to that of the soluble thrombomodulin is fixed and the soluble thrombomodulin is also preferable. Also affinity chromatography utilizing the specific affinity to the soluble thrombomodulin may favorably be exemplified. Favorable examples of adsorbent to be used include antibodies of soluble thrombomodulin and of thrombin, which may constitute a ligand of the soluble thrombomodulin. Antibodies of soluble thrombomodulin capable of recognizing an adequate function or an adequate epitope can be utilized as these antibodies. Examples of the antibodies include those described in JP 05-042920 B, JP 64-045398 A, and JP 06-205692 A. Further, gel filtration chromatography and an ultrafiltration technique utilizing the molecular size of the soluble thrombomodulin can be given. Furthermore, there may be employed hydrophobic chromatography based on a hydrophobic bond between the chromatography carrier on which a hydrophobic group is fixed and the hydrophobic site of the soluble thrombomodulin. Further, a hydroxyapatite may be used as the carrier for the adsorption chromatography, examples of which include that described in JP 09-110900 A. The above-mentioned techniques may adequately be combined. The degree of purification of the soluble thrombomodulin may adequately be selected in accordance with each specific application. However, it is favorable to purify up to such a degree that a single band will be obtained by, for example, electrophoresis, preferably, SDS-PAGE, or a single peak will be obtained by, for example, gel permeation HPLC or reversed phase HPLC of isolation and purification product.

It goes without saying that in the case where various types of soluble thrombomodulin are used, it is not required that the band become a single band, but one substantially only of soluble thrombomodulin is preferable.

Specific examples of the procedures of purification in the present invention include a technique in which the purification is proceeded using soluble thrombomodulin activity as an index. In such a technique, for example, the supernatant or the culture mixture is roughly purified by Q-Sepharose-Fast Flow of an ion-exchange column to collect fractions exhibiting soluble thrombomodulin activity. Then, the fractions are then mainly purified with DIP- thrombin agarose (diisopropylphosphorylthrombin agarose) column of an affinity column to collect fractions exhibiting higher soluble thrombomodulin activity. The collected fractions are then concentrated and subjected to gel filtration to obtain soluble thrombomodulin-active fractions as a pure soluble thrombomodulin product (see Gomi K. et al., Blood, 75, 1396 - 1399 (1990)). Examples of the soluble thrombomodulin activity as the index include the activity of thrombin to promote activation of protein C. The following methods may also be exemplified as other purification technique.

Soluble thrombomodulin is subjected to ion-exchange chromatography using an adequately selected ion-exchange resin exhibiting good adsorbing performance for the soluble thrombomodulin . An especially preferred method involves the use of Q-Sepharose-Fast Flow equilibrated with a 0.02 M Tris-HCl buffer (pH 7.4) containing 0.18 M NaCl. The retained thrombomodulin can be eluted out after being washed adequately, using, for example, a 0. 02 M Tris-HCl buffer (pH 7.4) containing 0.3 M NaCl, to obtain a crude product of soluble thrombomodulin.

Next, this product may be subjected to a purification step by, for example, affinity chromatography using a resin on which a substance having a specific affinity to soluble thrombomodulin is fixed. Preferable examples include a DIP-thrombin-agarose column and an anti-soluble thrombomodulin monoclonal antibody column. The DIP-thrombin-agarose column can be precedently equilibrated with, for example, a 20 mM Tris-HCl buffer (pH 7.4) containing 100 mM NaCl and 0.5 mM calcium chloride, the column is then charged with the above-mentioned crude product, followed by adequate washing. Subsequently, the retained thrombomodulin is eluted with, for example, a 20 mM Tris-HCl buffer (pH 7.4) containing 1.0 M NaCl and 0.5 mM calcium chloride, to obtain a purified product of the soluble thrombomodulin. In the case of the anti-soluble thrombomodulin monoclonal antibody column, the Sepharose 4FF (Pharmacia) activated precedently by CNBr are brought into contact with a 0.1 M $NaHCO_3$ buffer (pH 8.3) containing 0.5 M NaCl and an anti-soluble thrombomodulin monoclonal antibody dissolved therein, and the column is filled with resin obtained by causing the anti-soluble thrombomodulin monoclonal antibody to be coupled onto the Sepharose 4FF. Then, the column is equilibrated beforehand with, for example, a 20 mM phosphate buffer (pH 7.3) containing 0.3 M NaCl, followed by adequate washing. Then, the thrombomodulin is eluted out with, for example, a 100 mM glycine-HCl buffer (pH 3.0) containing 0.3 M NaCl. The effluent is then neutralized with an adequate buffer thereby a purified product may be obtained.

Next, the pH of the purified product thus obtained is adjusted to pH 3.5 and the product is then charged in a cation-exchanger equilibrated with a 100 mM glycine-HCl buffer (pH 3.5) containing 0.3 M NaCl, preferably SP-Sepharose FF (Pharmacia) which is a strong cation-exchanger, followed by washing with the same buffer to obtain a non-absorption fraction. The obtained fraction is neutralized with an appropriate buffer to obtain a high purity product. Preferably, the purified product is concentrated by ultrafiltration.

Further, it is also preferable to carry out exchange of the buffer by means of gel filtration. For example, a column of Sephacryl S-300 or S-200 which has been equilibrated with a 20 mM phosphate buffer (pH 7.3) containing 50 mM NaCl is charged with a high purity product concentrated by ultrafiltration. The column is subjected to developing fractionation with a 20 mM phosphate buffer (pH 7.3) containing 50 mM NaCl, followed by collecting the fractions which show activity for promotion of protein C activation by thrombin after confirming such activity and performing exchange of the buffer, whereby a high purity product can be obtained. It is preferable to filtrate the high purity product thus obtained through a suitable filter for virus removal, for example, Planova 15N (Asahi Kasei Corporation) in order to enhance safety. After that, the product can be concentrated to the target concentration by ultrafiltration. It is preferable to filtrate the product through a sterile filter at the end.

The foam inhibition of the present invention is generally understood such that a soluble thrombomodulin-containing freeze-dried preparation is dissolved in a dissolving aqueous solution and then the generation of air bubbles in the solution is prevented at the time of obtaining a high-concentrated soluble thrombomodulin-containing solution. Needless to say, most preferable is to almost completely prevent the generation of air bubbles by the foam-inhibition method of the present invention. It is understood that there is a foam-inhibition effect when the foam inhibition of the present invention improves a foaming level, compared with the case where no measures have been taken to the foam inhibition.

A method of observing the degree of turbidity by visual inspection can be given as a method of knowing the level of foam inhibition. For example, by the following method, the degree of air-bubble generation can be examined by measuring transmittance at a wavelength of 650 nm as turbidity. For instance, an injection syringe (1 mL for tuberculin, an injection needle: 26G 0.45 x 13 mm, manufactured by Terumo Corp.) suctioning a 1 mL dissolving aqueous solution (e.g., Japanese Pharmacopoeia water for injection, manufactured by Otsuka Pharmaceutical Factory) is inserted into the center of a rubber stopper of a freeze-dried preparation to be provided as a test sample (e.g., 3 mL vial (18 x 33 mm, 9.1 mm in opening internal diameter, manufactured by Namicos) and a rubber stopper (V5-F8, manufactured by Daikyo Seiko)). Then the dissolving aqueous solution is injected into the center of a freeze-dried product at a rate of 0.1 mL/sec, followed by leaving it standing while keeping the injection syringe being inserted and dissolving. After 30 seconds from the completion of injection, the vial is flipped upside down and then the freeze-dried product-dissolving solution is suctioned in an amount of approximately 0.8 mL with the syringe. The suctioned dissolving solution is gently poured into a quartz cell (a black cell, optical length: 10 mm, optical width: 2 mm, manufactured by GL Sciences) along the cell wall thereof. Transmittance at 650 nm (which is adjusted to a transmittance of 100% at 650 nm in advance using only the dissolving aqueous solution) may be measured quickly using an UV-visible spectrophotometer (UV-2500PC, manufactured by Shimadzu Corporation). For expecting the accuracy of the measurement, a time from the injection of the dissolving aqueous solution to the measurement of transmittance should be kept constant. In a preferred example, the time from the suction of the freeze-dried product-dissolving solution from a vial to the measurement of transmittance is set to approximately 15 seconds. Preferable degree of foam inhibition is, in general, such that the generation of air bubbles enough to be shown as white turbidity cannot be visually observed. It is more preferable that no air bubble can be visually observed. Furthermore, in the case of a method of measuring turbidity using the above device, the solution has a transmittance of preferably 95% or more, more preferably 97% or more, particularly preferably 98% or more, most preferably 99% or more.

In a high-concentrated soluble thrombomodulin-containing solution obtained by dissolving the soluble thrombomodulin-containing freeze-dried preparation of the present invention, an upper limit of a soluble thrombomodulin concentration is not specifically limited as far as it is equal to or less than the concentration that allows dissolving. For example, the upper limit is generally 100 mg/mL or less, or 80 mg/mL or less, preferably 60 mg/mL or less, or 50 mg/mL or less, more preferably 40 mg/mL or less, particularly preferably 30 mg/mL or less. A lower limit concentration of the soluble thrombomodulin is preferably 10 mg/mL or more, or 13 mg/mL or more, more preferably 15 mg/mL or more, or 16 mg/mL or more, still more preferably 17 mg/mL or more, particularly preferably 20 mg/mL or more. Furthermore, for example, the lower limit is preferably 25 mg/mL or more, particularly preferably about 30 mg/mL or more. By the way, in the case of seeking a foam-inhibition effect, it is preferable that the concentration be of causing the problem of foaming.

The nonionic surfactant provided as a foam-inhibition additive of the present invention (hereinafter, also referred to as a foam-inhibition additive) is preferably one which can be used as an additive in pharmaceutical preparations, but not specifically limited to. When the nonionic surfactants for the present invention are classified from their structures, preferable types thereof include polyoxyethylene sorbitan fatty acid ester, polyoxyethylene/polyoxypropylene copolymer, polyoxyethylene alkylether, polyoxyethylene fatty acid ester, and polyoxyethylene hydrogenated castor oil.

Examples of the polyoxyethylene sorbitan fatty acid ester include monolaurate (Polysorbate 20), monopalmitate (Polysorbate 40), monostearate (Polysorbate 60), tristearate, isostearate, monooleate (Polysorbate 80), and trioleate. More preferable examples thereof include Polysorbate 20, Polysorbate 40, Polysorbate 60, and Polysorbate 80. Polysorbate 80 is particularly preferable.

Further, polymerization degrees of propylene oxide and ethylene oxide in the polyoxyethylene/polyoxypropylene copolymer are not specifically limited and may be selected arbitrarily. Preferable examples of the polyoxyethylene-polyoxypropylene copolymer include Poloxamer 124, Poloxamer 188, Poloxamer 237, Poloxamer 338, and Poloxamer 407. Particularly preferable examples thereof include Poloxamer 188 and polyoxyethylene (160) polyoxypropylene (30) glycol.

Further, the polyoxyethylene alkyl ether contains higher alcohol moiety such as lauryl alcohol, myristyl alcohol, cetyl alcohol, and stearyl alcohol. A polymerization degree of ethylene oxide may be selected arbitrarily. Lauromacrogol (polyoxyethylene lauryl ether) is preferable, and lauromacrogol having 9, 21, or 25 moles of ethylene oxide units added is more preferable.

Further, examples of the polyoxyethylene fatty acid ester include monolaurate, dilaurate, monostearate, distearate, monooleate, and dioleate. Polyoxyethylene monostearate is preferable, and more preferable examples thereof having different numbers of ethylene oxide units (moles) added include Polyoxyl 12 stearate, Polyoxyl 20 stearate, Polyoxyl 40 stearate, Polyoxyl 50 stearate, and Polyoxyl 100 stearate. Polyoxyl 40 stearate is particularly preferable.

Further, preferable examples of the polyoxyethylene hydrogenated castor oil having different numbers of ethylene oxide units (moles) added include Polyoxyl 20 hydrogenated castor oil, Polyoxyl 30 hydrogenated castor oil, Polyoxyl 40 hydrogenated castor oil, Polyoxyl 50 hydrogenated castor oil (may also be referred to as "poriokishiechiren-koka-himashiyu-60"), Polyoxyl 60 hydrogenated castor oil, Polyoxyl 80 hydrogenated castor oil, and Polyoxyl 100 hydrogenated castor oil. Polyoxyl 60 hydrogenated castor oil (hereinafter, may also be referred to as "HCO-60" or "poriokishiechiren-koka-himashiyu-60") is particularly preferable. Each of the nonionic surfactants may be used alone, or two or more types thereof may be used as a mixture. Further, each of the nonionic surfactants may be used as a mixture with benzyl alcohol or chlorobutanol.

The addition amount of a foam-inhibition additive to be added in a soluble thrombomodulin-containing freeze-dried preparation of the present invention or in a dissolving aqueous solution for dissolving the soluble thrombomodulin-containing freeze-dried preparation is not specifically limited as far as it is an amount that inhibits foaming. The addition amount may be suitably defined depending on the concentration of soluble thrombomodulin or the contents of other additives. Furthermore, the amount may vary in the presence or absence of treatment of (b) coating an inner wall of a container to be used in dissolving the soluble thrombomodulin-containing freeze-dried preparation with silicone, (c) keeping a pressure in the container to be used in dissolving the soluble thrombomodulin-containing freeze-dried preparation at a reduced pressure reduced by -13332,2 Pa to -39996,6 Pa (-100 to -300 mmHg), or the like, which will be described later. Therefore, for example, it is preferable to determine the addition amount on the basis of advance measurement of the addition amount which shows a transmittance of, for example, 95% or more in a method of measuring the degree of generation of air bubbles using transmittance at a wave length of 650 nm. As an example thereof, among the additives for foam inhibition, the addition amount of a nonionic surfactant is, for example, preferably 0.001 mg or more, more preferably 0.005 mg or more, still more preferably 0.01 mg or more as a lower limit of the addition amount of the nonionic surfactant present in a high-concentrated soluble thrombomodulin-containing solution in a concentration of 10 mg/mL when such a solution is prepared by dissolving a soluble thrombomodulin-containing freeze-dried preparation containing soluble thrombomodulin as an active ingredient. In some cases, the lower limit is 0.05mg or more, 0.1 mg or more, and 0.5 mg or more. An upper limit of the addition amount of the nonionic surfactant is generally 10 mg or less, preferably 5 mg or less, more preferably 1 mg or less. In some cases, the upper limit is 0.5 mg or less. In addition, for preparing 30 mg/mL of a high-concentrated soluble thrombomodulin-containing solution, a lower limit of the addition amount of a nonionic surfactant present in the solution is, for example, preferably 0.003 mg or more, more preferably 0.015 mg or more, still more preferably 0.03 mg or more. In some cases, the lower limit is preferably 0.15 mg or more, 0.3 mg or more, or 1.5 mg or more. The upper limit of the addition amount of the nonionic surfactant, for example, is generally 30 mg or less, preferably 15 mg or less, more preferably 3 mg or less. In some cases, the upper limit is 1.5 mg or less. Furthermore, for preparing high-concentrated soluble thrombomodulin-containing solutions having 17, 20, and 25 mg/mL in concentration or other concentration, it is preferable to really measure the addition amount thereof. For instance, referring to the above exemplified addition amounts of nonionic surfactant in 10 and 30 mg/mL high-concentrated soluble thrombomodulin-containing solutions, the addition amount is preferably determined by calculation of geometrical proportion or the like on the basis of the concentration of soluble thrombomodulin.

When plural nonionic surfactants are added, the addition amount can be also determined by way of examining an addition amount that shows a transmittance of 95% or more in the method of measuring transmittance at a wavelength of 650 nm. Generally, it may be easy to define the sum of the amounts of the plural nonionic surfactants as the addition amount exemplified above. It is also preferable to add the surfactants in slightly higher concentrations.

Among additives for foam inhibition to be added to the soluble thrombomodulin-containing freeze-dried preparation of the present invention or to a dissolving aqueous solution for dissolving the soluble thrombomodulin-containing freeze-dried preparation, the addition amount of benzyl alcohol, chlorobutanol, or a mixture thereof is not specifically limited as far as it is the amounts capable of inhibiting foaming. However, an optimum addition amount can be determined on the basis of the contents of soluble thrombomodulin and other additives. In addition, for obtaining the effect of foam inhibition using benzyl alcohol or chlorobutanol, it is preferable to add these compounds to the dissolving aqueous solution. A method of studying the addition amount of these additives may be preferably, for example, a method of finding out an addition amount that shows a transmittance of 95% or more by measuring the transmittance at a wavelength of 650 nm as described above. As a specific example, when a 10 mg/mL high-concentrated soluble thrombomodulin-containing solution is prepared by dissolving the soluble thrombomodulin-containing freeze-dried preparation, the lower limit of the addition amount of benzyl alcohol, chlorobutanol, or a mixture thereof present in the solution is, for example, preferably 0.01 mg or more, more preferably 0.05 mg or more, still more preferably 0.1 mg or more. In some cases, the lower limit is 0.5 mg or more, 1mg or more, or 5 mg or more. The upper limit thereof is, for example, 100 mg or less in general, preferably 50 mg or less, more preferably 10 mg or less. In some cases, the upper limit is 5 mg or less. Furthermore, for preparing a 30 mg/mL high-concentrated soluble thrombomodulin-containing solution, the lower limit of the addition amount of benzyl alcohol, chlorobutanol, or a mixture thereof present in the solution is, for example, preferably 0.03 mg or more, more preferably 0.15 mg or more, still more preferably 0.3 mg or more. In some cases, the lower limit is 1.5 mg or more, 3 mg or more, or 15 mg or more. The upper limit thereof is generally 300 mg or less, preferably 150 mg or less, more preferably 30 mg or less. In some cases, the upper limit is 15 mg or less. Furthermore, for preparing high-concentrated soluble thrombomodulin-containing solutions in concentrations other than those described above, for example, the addition amount is preferably determined by calculation of geometrical proportion or the like on the basis of the concentration of soluble thrombomodulin with reference to the exemplified addition amounts of benzyl alcohol, chlorobutanol, or the like present in 10 or 30 mg/mL high-concentrated soluble thrombomodulin-containing solution.

Preferable examples of the dissolving aqueous solutions used in the present invention include, but not specifically limited to, for example, distilled water (or water for injection), a physiological saline, a glucose injection solution, a xylitol injection solution, and the like. Of those, distilled water is given as a preferable example. Furthermore, it is also preferable that the dissolving aqueous solution be allowed to contain at least one compound selected from the group consisting of a nonionic surfactant, benzyl alcohol, and chlorobutanol. When the freeze-dried preparation contains at least one compound selected from the group consisting of a nonionic surfactant, benzyl alcohol, and chlorobutanol, the dissolving aqueous solution used may be distilled water, a physiological saline, a glucose injection solution, or the like. Furthermore, the dissolving aqueous solution may contain at least one compound selected from the group consisting of a nonionic surfactant, benzyl alcohol, and chlorobutanol. In general, the above freeze-dried preparation preferably contains a nonionic surfactant, rather than benzyl alcohol or chlorobutanol. When the above freeze-dried preparation does not contain a nonionic surfactant, benzyl alcohol, or chlorobutanol, the dissolving aqueous solution can exert the action of foam inhibition by allowing the solution to contain at least one compound selected from the group consisting of a nonionic surfactant, benzyl alcohol, and chlorobutanol. This is also a preferable case. By the way, when such a foam-inhibition additive is added to the dissolving aqueous solution, the addition amount of the foam-inhibition additive is not specifically limited as far as a foam-inhibition effect is efficiently exerted. However, the amount is preferably the above exemplified amount as the addition amount corresponding to the soluble-thrombomodulin concentration in a high-concentrated soluble thrombomodulin-containing solution.

The addition amount of a nonionic surfactant at the time of adding the surfactant to a dissolving aqueous solution is not specifically limited as far as a foam-inhibition effect is effectively exerted, and may be suitably determined by the measurement as described above. For simplification, by presenting the addition amount of a nonionic surfactant, benzyl alcohol, or chlorobutanol in the dissolving aqueous solution, which gives an indication under normal conditions including the concentration of soluble thrombomodulin, the following will be exemplified. That is, for a nonionic surfactant, the upper limit of the usual addition amount thereof may be generally 100 mg or less per 1 mL of distilled water, or 50 mg or less, preferably 30 mg or less, or 10 mg or less, particularly preferably 1 mg or less. The lower limit may be 0.001 mg or more, preferably 0.01 mg or more, more preferably 0.03 mg or more, particularly preferably 0.05 mg or more, or 0.1 mg or more. For the addition amount of benzyl alcohol, for example, the upper limit of the addition amount thereof is generally 50 mg or less per 1 mL of distilled water, or 40 mg or less, preferably 30 mg or less, more preferably 20 mg or less, or 10 mg or less. The lower limit thereof is, for example, 0.1 mg or more, or 1 mg or more, preferably 4 mg or more, or 5 mg or more, more preferably 7 mg or more, particularly preferably 10 mg or more. In addition, for the addition amount of chlorobutanol, for example, the upper limit of the addition amount thereof is generally 20 mg or less per 1 mL of distilled water, or 15 mg or less, preferably 10 mg or less, or 8 mg or less, more preferably 7 mg or less, or 5 mg or less. The lower limit thereof is, for example, generally 0.1 mg or more, or 1 mg or more, preferably 2 mg or more, more preferably 3 mg or more, particularly preferably 5 mg or more.

In addition, the volume of the dissolving aqueous solution is not specifically limited in small quantities to large quantities. When subcutaneous or intramuscular injection is considered, the volume is, for example, generally 0.1 mL or more, preferably 0.3 mL or more, more preferably 0.5 mL or more. Furthermore, the upper limit of the volume is, for example, generally 10 mL or less, preferably 5 mL or less, more preferably 3 mL or less, particularly preferably 2 mL or less, or 1. 5 mL or less. Furthermore, particularly preferably, the volume is generally in the range of about 0.5 mL to about 1 mL.

It is desirable that the osmotic pressure ratio be of causing no pain at the time of administrating the preparation. In general, the ratio is preferably 0.5 or more, more preferably 0.8 or more. The upper limit thereof is preferably 2.0 or less, more preferably 1.5 or less, particularly preferably 1.2 or less.

For attaining the method for foam inhibition of the present invention and the effects thereof, other compositions other than the soluble thrombomodulin and the foam-inhibition additives in the soluble thrombomodulin-containing freeze-dried preparation or a kit preparation thereof are not specifically limited as far as they do not specifically interrupt the foam inhibition. For example, various types of amino acids including arginine and the salts thereof, sugars including mannitol and trehalose, and urea may be added, and other additives generally used may be added. Alternatively, a freeze-dried preparation may contain only soluble thrombomodulin in the absence of those compositions, or may consist of soluble thrombomodulin and a foam-inhibition additive. However, a soluble thrombomodulin-containing freeze-dried preparation allowed to contain one combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol, is preferable in stability and thus best-suited. In addition, an arginine, trehalose, or urea composition is given as a preferred example.

That is, according to the present invention, there is provided a method of inhibiting foaming of a high-concentrated soluble thrombomodulin-containing solution, which is characterized in that: the soluble thrombomodulin-containing freeze-dried preparation is allowed to contain any one combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; and furthermore the soluble thrombomodulin-containing freeze-dried preparation is allowed to contain at least one compound selected from the group consisting of a nonionic surfactant, benzyl alcohol, and chlorobutanol and/or a dissolving aqueous solution for dissolving the soluble thrombomodulin-containing freeze-dried preparation is allowed to contain at least one compound selected from the group consisting of a nonionic surfactant, benzyl alcohol, and chlorobutanol. It is preferable that the soluble thrombomodulin-containing freeze-dried preparation can be dissolved in 0.1 mL to 2 mL of a dissolving aqueous solution to prepare a high-concentrated soluble thrombomodulin-containing solution having a concentration of 10 mg/mL or more with an osmotic pressure ratio in the range of 0.5 to 2.0 at the time of dissolution.

Furthermore, according to the present invention, there is provided a method of inhibiting foaming of a high-concentrated soluble thrombomodulin-containing solution, which is characterized in that: the soluble thrombomodulin-containing freeze-dried preparation is allowed to contain either (1) urea or (2) urea and an amino acid and also the soluble thrombomodulin-containing freeze-dried preparation is allowed to contain at least one compound selected from the group consisting of a nonionic surfactant, benzyl alcohol, and chlorobutanol and/or a dissolving aqueous solution for dissolving the soluble thrombomodulin-containing freeze-dried preparation is allowed to contain at least one compound selected from the group consisting of a nonionic surfactant, benzyl alcohol, and chlorobutanol. In addition, a high-concentrated soluble thrombomodulin-containing solution can be prepared in a concentration of 10 mg/mL or more by dissolving the soluble thrombomodulin-containing freeze-dried preparation in a 0.1 to 2 mL dissolving aqueous solution, and the osmotic pressure ratio thereof at the time of dissolution is preferably in the range of 0.5 to 2.0.

Furthermore, according to the present invention, there is provided a method of inhibiting foaming of a high-concentrated soluble thrombomodulin-containing solution, characterized in that: the soluble thrombomodulin-containing freeze-dried preparation is allowed to contain one or two selected from the group consisting of arginine, glutamic acid, proline, serine, glycine, histidine, asparagine, lysine, phenylalanine, and valine, and salts thereof, trehalose, lactose, and sucrose; further, at least one compound selected from the group consisting of a nonionic surfactant, benzyl alcohol, and chlorobutanol is allowed to be present in the soluble thrombomodulin-containing freeze-dried preparation and/or at least one compound selected from the group consisting of a nonionic surfactant, benzyl alcohol, and chlorobutanol is allowed to be present in the dissolving aqueous solution for dissolving the soluble thrombomodulin-containing freeze-dried preparation.

Furthermore, in the present invention, not only foam inhibition at the time of dissolution but also an effect of defoaming against shaking can be expected from the high-concentrated soluble thrombomodulin-containing solution containing a foam-inhibition additive, and in particular it is preferable to allow the solution to contain benzyl alcohol or chlorobutanol.

Next, a description will be given of that (b) a container having the inner wall coated with silicone is used in dissolving the soluble thrombomodulin-containing freeze-dried preparation.

That is, in the foam-inhibition method of the present invention, it is preferable that the inner wall of the container being used in dissolving the soluble thrombomodulin-containing freeze-dried preparation is coated with silicone.

The container used in dissolving the soluble thrombomodulin-containing freeze-dried preparation in the present invention is not specifically limited as far as its material and shape can be employed for as a container for pharmaceutical preparations and are suitable for aseptic packaging, in addition the container is preferably suitable for filling with the soluble thrombomodulin-containing freeze-dried preparation. In general, the container filled with the soluble thrombomodulin-containing freeze-dried preparation is preferably used as it is also at the time of dissolution. The materials for the container used in dissolving the soluble thrombomodulin-containing freeze-dried preparation can be selected from, but not limited to: glass; plastics such as polypropylene, polyethylene, cyclic polyolef in, a copolymer of cyclic polyolefin and $\alpha$-olefin, polyethylene terephthalate, polystyrene, ABS resin, polyester, polyvinyl chloride, nylon, polytetrafluoroethylene, polymethylpentene, hexafluoro resin, polymethyl methacrylate, and polycarbonate; metals such as stainless steel, gold, aluminum, aluminum alloy, and titanium; ceramics; composites such as carbon composites; quartz; and so on. It is also preferable to use dealkalized glass as the glass. The dealkalization treatment can be performed by a method in which the glass is brought into contact with an aqueous ammonium sulfate solution, heated at high temperatures and washed or a method in which the surface of glass is brought into contact with a water-soluble sulfur oxide such as sulfur dioxide gas under high-temperature conditions to make alkali ingredients on the surface into sulfate fine crystals, followed by washing. Of these plastics, polypropylene, cyclic polyolefin, and a copolymer of cyclic olefin and α-olefin are preferable. Furthermore, cyclic polyolefin is particularly preferable.

The inner wall of the container used in dissolving the soluble thrombomodulin-containing freeze-dried preparation of the present invention means the part of the container where a dissolving solution in the container can be brought into contact with when the container is plugged (dissolved and sealed in the case of ampoule or the like).

For the coating of silicone to be applied on the inner wall of the container to be used in dissolving the soluble thrombomodulin-containing freeze-dried preparation of the present invention, the usual methods and materials can be used. Examples of silicone include: silicone oils such as dimethyl polysiloxane and methylhydrogen polysiloxane; methyl varnished silicone; and methyl phenyl varnished silicone. In addition, examples of the method of coating silicone in the present invention include a usual method, for example, in which a solvent is evaporated after a treatment of dipping the whole of the container in a silicone solution or a treatment of spraying a silicone solution onto the inner wall of the container, or a treatment of discharging the excess part of a silicone solution after bringing the silicone solution into contact with the inner wall by injecting the silicone oil into the container. Examples of solvents for the silicone solution include carbon tetrachloride and trichloroethylene. In terms of safety of the solvent, it is also preferable to use water-based emulsion prepared by emulsifying silicone. In the case of the glass, for the removal of solvent from the water-based emulsion, the emulsion is preferably heated at high temperatures of 100 to 800°C, and more preferably heated at high temperatures of 250 to 350°C. In the case of a resin or the like, the temperature is desirably on the order of those that do not allow the resin or the like to soften. Furthermore, it seems to be convenient to represent the amount of silicone by means of the concentration of a silicone solution to be adhered. For example, in the case of representing as "1% (w/v) silicone coating" when removing a solvent by allowing silicone solution with 1%(w/v) concentration to adhere to a container, the lower limit of the coating amount of silicone on the inner wall of the container is, for example, preferably 0.01% (w/v) or more, more preferably 0.05% (w/v) or more, still more preferably 0.1% (w/v) or more. In some cases, the lower limit is preferably 0.5%(w/v) or more, 1%(w/v) or more, or 2%(w/v) or more. The upper limit thereof is generally 10%(w/v) or less, preferably 7%(w/v) or less, more preferably 5%(w/v) or less. In some cases, the upper limit is 2%(w/v) or less.

Furthermore, a description is given hereinbelow of that (c) a pressure in the container to be used in dissolving the soluble thrombomodulin-containing freeze-dried preparation is kept at a reduced pressure, reduced by -13332.2 Pa to -39996.6 Pa (-100 to -300 mmHg).

That is, in the foam-inhibition method of the present invention, it is preferable that a pressure in the container to be used in dissolving the soluble thrombomodulin-containing freeze-dried preparation be kept at a reduced pressure. In general, it is understood that the pressure in the container is lower than the atmospheric pressure of 101324.7 Pa (760 mmHg). A method of making the state of reduced pressure may be any method, for example, a method in which a dissolving aqueous solution is injected using a syringe at the time of dissolving the freeze-dried preparation and then the gas in the container is aspirated from the container using the syringe to reduce the pressure in the container to less than the atmospheric pressure can be given. In addition, a method in which the gas in the container is aspirated through a needle connected to a pressure-reducing device such as a vacuum pump after the dissolving aqueous solution has been injected with the syringe can also be given. Furthermore, it is preferable to depressurize the inside of the container before injection of the dissolving aqueous solution with the syringe. At the step of manufacturing freeze-dry pharmaceutical, it may be also studied that the container is plugged at a reduced pressure. For example, preferable examples include the aspiration of gas in the container through a needle connected to an empty syringe or vacuum pump before the step of dissolution. The reduced state of the pressure in the container at the time of dissolving the soluble thrombomodulin-containing freeze-dried preparation, for example, the state of being reduced by 26664.4 Pa or 200 mmHg from the atmospheric pressure of 101 324.7 Pa (760 mmHg) may be represented as a decompression degree of -26664.4 Pa or -200 mmHg. For attaining the predetermined decompression degree at the time of dissolution, when there is a need of reducing the pressure in advance, it is preferable that the change in volume of the dissolving aqueous solution or the like to be added be checked and then an initial decompression degree is determined and the pressure in the container is reduced to the initial decompression degree prior to the dissolution. The initial decompression degree may be determined by the methods described in examples and other suitable methods.

A pressure in the container preferable for foam inhibition at the time of dissolving the soluble thrombomodulin-containing freeze-dried preparation varies to some extent depending on the capacity and type of the container and the volume of a dissolving aqueous solution. Thus, it is preferable to allow the reduced-pressure state so as to show a transmittance of 95% or more in a method of measuring transmittance at a wavelength of 650 nm as turbidity, i.e. the degree of generation of the above air bubbles Therefore, the preferable pressure in the container is not specifically limited. For example, when the soluble thrombomodulin-containing freeze-dried preparation in a 3 mL vial is dissolved in a 1 mL dissolving aqueous solution, the lower limit of the pressure in the container is -13332.2 Pa or more (-100 mmHg or more). In some cases, the lower limit is -26664.4 Pa or more (-200 mmHg or more). In addition, the upper limit is -39996.6 Pa or less (-300 mmHg or less). Furthermore, the present invention provides a soluble thrombomodulin-containing freeze-dried preparation having the above-described foam-inhibition effect and/or a stabilization effect described below and a kit preparation which is a combination between the freeze-dried preparation and a dissolving aqueous solution therefor.

That is, according to the present invention, there is provided a soluble thrombomodulin-containing freeze-dried preparation containing soluble thrombomodulin as an active ingredient or a kit preparation thereof which is a combination of the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more, the soluble thrombomodulin-containing freeze-dried preparation or the kit preparation thereof being characterized by at least one of (a) that at least one compound selected from the group consisting of a nonionic surfactant, benzyl alcohol, and chlorobutanol is present in the soluble thrombomodulin-containing freeze-dried preparation and/or at least one compound selected from the group consisting of a nonionic surfactant, benzyl alcohol, and chlorobutanol is allowed to be present in the dissolving aqueous solution for the soluble thrombomodulin-containing freeze-dried preparation, (b) that a container having the inner wall coated with silicone is used in dissolving the soluble thrombomodulin-containing freeze-dried preparation, and (c) that a pressure in the container to be used in dissolving the soluble thrombomodulin-containing freeze-dried preparation is kept at a reduced pressure, reduced by -13332.2 Pa to -39996.6 Pa (-100 to -300 mm Hg).

Any one of (a), (b), and (c) described above is preferable for the soluble thrombomodulin-containing freeze-dried preparation of the present invention or the kit preparation thereof. Of those, preferable are (a), and a combination of (a) and (c) or of (a) and (b) is also preferable. Furthermore, a combination of (b) and (c) is preferable too, and a combination of (a), (b), and (c) is given as a particularly preferable example.

More specifically, according to the present invention, there is provided a soluble thrombomodulin-containing freeze-dried preparation containing soluble thrombomodulin as an active ingredient or a kit preparation thereof which is a combination of the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more, the preparation being characterized in that: the soluble thrombomodulin-containing freeze-dried preparation is allowed to contain a combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; and the soluble thrombomodulin-containing freeze-dried preparation can be dissolved in 0.1 mL to 2 mL of the dissolving aqueous solution to prepare a high-concentrated soluble thrombomodulin-containing solution having a concentration of 10 mg/mL or more with an osmotic pressure ratio in the range of 0.5 to 2.0 at the time of dissolution.

Furthermore, according to the present invention, there is provided a soluble thrombomodulin-containing freeze-dried preparation containing soluble thrombomodulin as an active ingredient or a kit preparation thereof which is a combination of the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more, the preparation being characterized in that: the soluble thrombomodulin-containing freeze-dried preparation is allowed to contain a combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; and the soluble thrombomodulin-containing freeze-dried preparation can be dissolved in 0.1 mL to 2 mL of the dissolving aqueous solution to prepare a high-concentrated soluble thrombomodulin-containing solution having a concentration of 10 mg/mL or more with an osmotic pressure ratio in the range of 0.5 to 2.0 upon dissolution; and at least one compound selected from the group consisting of a nonionic surfactant, benzyl alcohol, and chlorobutanol is present in the soluble thrombomodulin-containing freeze-dried preparation. In this case, as described above, (b) or (c), or a combination thereof is preferable.

Furthermore, according to the present invention, there is provided a soluble thrombomodulin-containing freeze-dried preparation containing soluble thrombomodulin as an active ingredient or a kit preparation thereof which is a combination between the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more, the kit preparation of a soluble thrombomodulin-containing freeze-dried preparation being characterized in that: the soluble thrombomodulin-containing freeze-dried preparation is allowed to contain a combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; and the soluble thrombomodulin-containing freeze-dried preparation can be dissolved in 0.1 mL to 2 mL of the dissolving aqueous solution to prepare a high-concentrated soluble thrombomodulin-containing solution having a concentration of 10 mg/mL or more with an osmotic pressure ratio in the range of 0.5 to 2.0 upon dissolution; and at least one compound selected from the group consisting of a nonionic surfactant, benzyl alcohol, and chlorobutanol is present in the dissolving solution for the soluble thrombomodulin-containing freeze-dried preparation. In this case, as described above, (b) or (c), or a combination thereof is preferable.

Furthermore, according to the present invention, there is provided a soluble thrombomodulin-containing freeze-dried preparation containing soluble thrombomodulin as an active ingredient or a kit preparation thereof which is a combination between the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more, the kit preparation of a soluble thrombomodulin-containing freeze-dried preparation being characterized in that: the soluble thrombomodulin-containing freeze-dried preparation is allowed to contain a combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; the soluble thrombomodulin-containing freeze-dried preparation can be dissolved in 0.1 mL to 2 mL of the dissolving aqueous solution to prepare a high-concentrated soluble thrombomodulin-containing solution having a concentration of 10 mg/mL or more with an osmotic pressure ratio in the range of 0.5 to 2.0 upon dissolution; and at least one compound selected from the group consisting of a nonionic surfactant, benzyl alcohol, and chlorobutanol is present in the soluble thrombomodulin-containing freeze-dried preparation and at least one compound selected from the group consisting of a nonionic surfactant, benzyl alcohol, and chlorobutanol is present in the dissolving aqueous solution for the soluble thrombomodulin-containing freeze-dried preparation. In this case, as described above, (b) or (c), or a combination thereof is preferable.

In this invention, the various properties, concentrations, and so on of the soluble thrombomodulin are similar to those found in the foam-inhibition method described above.

Furthermore, according to the present invention, there is provided a method of stabilizing soluble thrombomodulin in a soluble-thrombomodulin-containing freeze-dried preparation containing soluble thrombomodulin as an active ingredient, characterized by including: the soluble-thrombomodulin-containing freeze-dried preparation is allowed to contain a combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol; and dissolving the soluble thrombomodulin-containing freeze-dried preparation in 0.1 mL to 2 mL of the dissolving aqueous solution to prepare a high-concentrated soluble thrombomodulin-containing solution having a concentration of 10 mg/mL or more, preferably 15 or 17 mg/mL or more with an osmotic pressure ratio in the range of 0.5 to 2.0 at the time of the dissolution.

Hereinafter, a stabilizing additive may refer to an additive that exhibits a stabilizing effect on soluble thrombomodulin in a high-concentrated soluble thrombomodulin-containing freeze-dried preparation, and for example, contains a combination selected from the group consisting of (1) a combination of two of glutamic acid or a salt thereof and mannitol, (2) a combination of two of glutamic acid or a salt thereof and lysine or a salt thereof, (3) a combination of two of glutamic acid or a salt thereof and asparaginic acid or a salt thereof, and (4) a combination of two of asparaginic acid or a salt thereof and mannitol.

More preferable preparations suitable for the stabilizing method of the present invention include the following. According to the present invention, there is provided a high-concentrated soluble thrombomodulin-containing freeze-dried preparation containing 10 mg/mL or more, preferably 15 or 17 mg/mL or more of soluble thrombomodulin as an active ingredient, characterized in that the preparation is allowed to contain glutamic acid or a salt thereof and mannitol, and can be dissolved in 0.1 mL to 2 mL of a dissolving aqueous solution with an osmotic pressure ratio thereof at the time of the dissolution in the range of 0.5 to 2.0.

Furthermore, according to the present invention, there is provided a high-concentrated soluble thrombomodulin-containing freeze-dried preparation containing 10 mg/mL or more, preferably 15 or 17 mg/mL or more of soluble thrombomodulin as an active ingredient, characterized in that the preparation is allowed to contain glutamic acid or a salt thereof and lysine or a salt thereof, and can be dissolved in 0.1 mL to 2 mL of a dissolving aqueous solution with an osmotic pressure ratio thereof at the time of the dissolution in the range of 0.5 to 2.0.

Furthermore, according to the present invention, there is provided a high-concentrated soluble thrombomodulin-containing freeze-dried preparation containing 10 mg/mL or more, preferably 15 or 17 mg/mL or more of soluble thrombomodulin as an active ingredient, characterized in that the preparation is allowed to contain glutamic acid or a salt thereof and asparaginic acid or a salt thereof, and can be dissolved in 0.1 mL to 2 mL of a dissolving aqueous solution with an osmotic pressure ratio thereof at the time of the dissolution in the range of 0.5 to 2.0.

Furthermore, according to the present invention, there is provided a high-concentrated soluble thrombomodulin-containing freeze-dried preparation containing 10 mg/mL or more, preferably 15 or 17 mg/mL or more of soluble thrombomodulin as an active ingredient, characterized in that the preparation is allowed to contain asparaginic acid or a salt thereof and mannitol, and can be being dissolved in 0.1 mL to 2 mL of a dissolving aqueous solution with an osmotic pressure ratio thereof at the time of the dissolution in the range of 0.5 to 2.0.

The properties, structure, and so on of the soluble thrombomodulin described above can be referenced even in the case of attaining the stabilizing effect, and the concentration of the high-concentrated soluble thrombomodulin-containing solution, the dissolving aqueous solution, and so on can also similarly be referenced.

It is preferable that the fluid volume of the high-concentrated soluble thrombomodulin-containing solution of the present invention be in the range of 0.1 mL to 2 mL and the osmotic pressure ratio of the solution be in the range of 0.5 to 2.0. It is desirable that the osmotic pressure ratio do not cause pain at administration of the preparation, so that in general the ratio is preferably 0.5 or more, more preferably 0. 8 or more. The upper limit is preferably 2.0 or less, more preferably 1.5 or less, particularly preferably 1.2 or less.

The osmotic pressure ratio of the present invention is defined as a ratio of the osmolality of a sample solution to the osmolality of a physiological saline (the 14th-revised version of Japanese pharmacopoeia, the osmotic pressure assay) and can be calculated from the following equation because the osmolality of the physiological saline (0.900 g/100 mL) is constant (286 mOsm):

[Osmotic pressure ratio

= osmolality (mOsm) of the sample solution / 286 (mOsm)].

By the way, the osmolality can be obtained by the osmotic pressure assay in the 14th-revised version of Japanese pharmacopoeia or the method described in USP 24 <785> OSMOLARITY, and can be represented using the unit of mOsm (milliosmoles per liter).

Glutamic acid in the present invention may be either optically active substance D-glutamic acid or L-glutamic acid, or a racemic form which is a mixture of the optically active substances, but is preferably L-glutamic acid. A salt of glutamic acid is preferably a water-soluble salt, and preferable examples thereof include an acid addition salt of glutamic acid. Examples of the acid that can be added to form the acid addition salt generally include hydrochloric acid, sulfuric acid, nitric acid, acetic acid, phosphoric acid, oxalic acid, citric acid, and tartaric acid. Preferable examples thereof include hydrochloric acid, acetic acid, and phosphoric acid. More preferable examples thereof include hydrochloric acid. Further, preferable examples of other salts include ammonium salts. As preferable examples salts with alkali metals or alkali earth metals can also be given. Examples of the alkali metals include sodium, potassium, and lithium. The alkali metal is preferably sodium or potassium, and more preferably sodium. Salts with alkali earth metals are also preferable. Examplesofthealkaliearthmetals include magnesium, calcium, and beryllium. The alkali earth metal is preferably magnesium or calcium, and more preferably calcium. Glutamic acid and the salts thereof may be used in the forms of anhydrides or suitable hydrates thereof. Specifically, sodium glutamate monohydrate is preferable. When the addition amounts of glutamic acid and the salts thereof are taken into consideration, the amounts are represented in terms of reduced value as free glutamic acid otherwise specifically noted.

Further, mannitol may be one of D-mannitol, L-mannitol, and a mixture thereof, but D-mannitol is preferable.

Further, trehalose may be one of D-trehalose, L-trehalose, and a mixture thereof. Further, trehalose anhydrides or suitable trehalose hydrates can be used. To be specific, D-trehalose dihydrate is preferable.

Further, lysine may be either optically active substance D-lysine or L-lysine, or a racemic form which is a mixture thereof, but is preferably L-lysine. A lysine salt is preferably a water-soluble salt, and preferable examples thereof include an acid addition salt of lysine. Examples of the acid that can be added to form the acid addition salt generally include hydrochloric acid, sulfuric acid, nitric acid, acetic acid, phosphoric acid, oxalic acid, citric acid, and tartaric acid. Preferable examples thereof include hydrochloric acid, acetic acid, and phosphoric acid. More preferable examples thereof include hydrochloric acid. Lysine and the lysine salts may be used in the forms of anhydrides or suitable hydrates thereof. To be specific, lysine hydrates are preferable. When the addition amounts of lysine and the lysine salts are taken into consideration, the amounts are represented in terms of reduced value as free lysine, otherwise specifically noted.

Further, lysine glutamate anhydride or lysine glutamate dihydrate are preferably used together as a combination of glutamic acid or the salts thereof with lysine or the lysine salts. When the amount of lysine glutamate dihydrate is taken into consideration, the amount is represented in term of reduced value as lysine or glutamic acid, separately.

Further, asparaginic acid may be either optically active substance D-asparaginic acid or L-asparaginic acid, or a racemic form which is a mixture thereof, but is preferably L-asparaginic acid. A salt of asparaginic acid is preferably a water-soluble salt, and preferable examples thereof include salts with alkali metals or alkali earth metals. Examples of the alkali metals include sodium, potassium, and lithium. The alkali metal is preferably sodium or potassium, and more preferably sodium. Salts with alkali earth metals are also preferable. Examples of the alkali earth metals include magnesium, calcium, and beryllium. The alkali earth metal is preferably magnesium or calcium, and more preferably calcium. Asparaginic acid and the salts of asparaginic acid may be used in the forms of anhydrides or suitable hydrates thereof. Tobe specific, sodium aspartate monohydrate is preferable. When the addition amounts of asparaginic acid and the salts thereof are taken into consideration, the amount is represented in terms of reduced value as free asparaginic acid otherwise specifically noted.

Further, arginine, proline, serine, glycine, histidine, asparagine, phenylalanine, and valine in the present invention may be either optically active substance D-form or L-form, or a racemic form which is a mixture of the optically active substances. Salts of the amino acids are preferably water-soluble salts, and preferable examples thereof include salts with alkali metals or alkali earth metals. The amino acids and the salts of the amino acids may be used in the forms of anhydrides or suitable hydrates thereof. Of these amino acids, for arginine, arginine hydrochloride can be given as preferable example. When the addition amounts of the amino acids or the salts of the amino acids are taken into consideration, the amounts are represented in terms of reduced value as free amino acids otherwise specifically noted.

Furthermore, a particularly preferable example of the ratio of the addition amount of the stabilizing additive described above to be added to the preparation of the present invention is 50 50, and also another preferable example is 60 : 40 to 40 : 60, and furthermore another preferable example is 80 : 20 to 20 : 80. The addition amount of each additive can be suitably selected. For example, the lower limit of the addition amount per 1 mg of soluble thrombomodulin is generally 0.0001 mmol or more, preferably 0.0003 mmol or more, more preferably 0.0007 mmol or more, particularly preferably 0.001 mmol or more. Furthermore, for example, the upper limit of the addition amount per 1 mg of soluble thrombomodulin is generally 1 mmol or less, preferably 0.7 mmol or less, more preferably 0.3 mmol or less, particularly preferably 0.1 mmol or less. The addition amount described above is not specifically limited as far as the amount exhibits the stabilizing effect of the present invention and is capable of adjusting the osmotic pressure ratio within the range of 0.5 to 2.0. For example, in the case of an additive which is ionized (dissociated) when it is dissolved in water just like hydrochloride or sodium salt, the osmotic pressure ratio is increased. Thus, it is preferable to appropriately adjust to a desired osmotic pressure ratio by adequately increasing or reducing the addition amount exemplified above.

In the present invention, the high-concentrated soluble thrombomodulin-containing preparation containing the above foam-inhibiting additive and/or the stabilizing additive may further properly contain an isotonizing agent, a buffering agent, a thickening agent, a preservative, an antiseptic agent, a soothing agent, a pH regulator or the like, which is allowable as an additive in pharmaceutical preparations as other ingredient, in addition to the above foam-inhibition additive and the stabilizing additive. In addition, benzyl alcohol or chlorobutanol is preferably used as a foam-inhibition additive because benzyl alcohol or chlorobutanol also functions as a soothing agent. Examples of surfactants include polyoxyethylene hydrogenated castor oil 60, polyoxyethylene hydrogenated castor oil 50, polysorbate 80, polysorbate 20, and polyoxyethylene (160) polyoxypropylene (30) glycol.

An appropriate method can be adopted into the manufacture of the high-concentrated soluble thrombomodulin-containing solution of the present invention. For example, the preparation can be provided by concurrently or successively mixing an appropriate amount of soluble thrombomodulin and the additive described above or a solution thereof to prepare a mixture solution of all the compositions and then subjecting the mixture solution to freeze-drying by the known method.

For instance, methods for the addition of the above foam-inhibition additive and/or stabilizing additive, or other ingredient additives include a method in which they are directly added to a high-concentrated soluble thrombomodulin-containing solution or a method in which a foam-inhibition additive, a stabilizing additive, and other ingredient additives are dissolved in water, water for injection, or an appropriate buffer in advance, followed by adding the solution to a high-concentrated soluble thrombomodulin-containing solution. The addition may be performed at any desired time. The addition, however, may be performed preferably during the step of purifying or concentrating the soluble thrombomodulin and more preferably during the step of manufacturing a preparation. During the step of manufacturing a preparation, for example, the high-concentrated soluble thrombomodulin-containing solution has a pH of particularly preferably around neutral. Furthermore, for example, the lower limit of the pH thereof is generally pH 4 or more, preferably pH 5 or more, more preferably pH 6 or more, particularly preferably pH 6.5 or more. Furthermore, for example, the upper limit of the pH is generally pH 11 or less, preferably pH 10 or less, more preferably pH 9 or less, still more preferably pH 8 or less, particularly preferably pH 7.5 or less. For attaining the above pH, an appropriate pH-adjusting agent or the like may be added to adjust the pH if required. Examples of the pH-adjusting agent include hydrochloric acid, acetic acid, citric acid, phosphoric acid, sodium dihydrogen phosphate, dibasic sodium phosphate, sodium hydroxide, and potassium hydroxide. Preferable of those are hydrochloric acid and sodium hydroxide. The time of pH adjustment is not specifically limited, but the adjustment is preferably performed just before the step of sterile filtration. Besides, the adjustment may be performed during the step of purifying or concentrating soluble thrombomodulin. An adjustment method is not specifically limited, but may include the steps of measuring the pH of a sampled solution with a pH meter and then adjusting the pH by, for example, dropping an aqueous solution of sodium hydroxide if the pH is low.

A solution containing the soluble thrombomodulin prepared by the step of manufacturing a preparation as described above, and the foam-inhibition additive and/or the stabilizing additive, which are added if required, and further containing other appropriate additives if desired is preferably subjected to sterile filtration through, for example, a 0.22 μm filter. The solution thus obtained, which contains the soluble thrombomodulin, the foam-inhibition additive and stabilizing additive added if required, or other additives, is filled in a container. Then, the solution may be provided as a preparation after tight-sealing without modification but is preferably subjected to freeze-drying. A freeze-drying method is not specifically limited, so that freeze-drying can be performed by a usual method. In addition, when the above foam-inhibition additive is allowed to be present in the soluble thrombomodulin-containing freeze-dried preparation by the freeze-drying method, the foam-inhibition additive is preferably a nonionic surfactant.

The volume of the solution to be filled in the container and subjected to freeze-drying is, for example, particularly preferably in the range of 0.5 to 1.0 mL. That is, the lower limit thereof is preferably 0.1 mL or more, more preferably 0.3 mL or more, particularly preferably 0.5 mL or more. In addition, the upper limit of the volume of the solution is, for example, generally 10 mL or less, preferably 5 mL or less, more preferably 2 mL or less, particularly preferably 1 mL or less.

For example, the amount of the soluble thrombomodul in contained in the high-concentrated soluble thrombomodulin-containing preparation of the present invention is generally 5 mg or more, preferably 10 mg or more, more preferably 15 mg or more per preparation. In addition, for example, the amount is preferably 17 mg or more, more preferably 20 mg or more, or 25 mg or more, particularly preferably 30 mg or more.

The materials or shapes for the container are as described above and not specifically limited as far as the materials or shapes can be used for containers for pharmaceutical products and are suitable for aseptic packaging. For example, as the container a glass vial (including its stopper), a glass syringe (including its rubber cap and stopper), a glass ample and the like can be given. In addition, plastic containers can be also used. In the present invention, the freeze-dried preparation is preferably provided in a form sealed in a glass vial. In addition, for example, preferable is a kit preparation in which a dissolving aqueous solution is combined with or attached to the freeze-dried preparation. The freeze-dried preparation and the dissolving aqueous solution may be filled in their respective containers, separately. As preferable example, the kit preparation comprising the combination of a glass vial aseptically filled with the freeze-dried preparation and a pre-filled syringe aseptically filled with the dissolving aqueous solution can be given. A two-chamber type syringe (there are two chambers in one syringe, for example, the freeze-dried product is enclosed in one chamber and the dissolving aqueous solution is enclosed in the other chamber) which is referred to a double or dual chamber made of glass or plastic is also preferable as a kit preparation. Furthermore, as another type of the two-chamber syringe, a preferable one is constructed so that two chambers are separated at the time of manufacture or distribution and then combined and united together at the time of use to allow dissolution.

The high-concentrated soluble thrombomodulin-containing preparation of the present invention can be stored for several months to several years during distribution and the period up to use thereof as a preparation. Methods for the administration thereof include, but not specifically limited to, administration methods generally used, i.e., parenteral administration methods such as intravenous administration, intramuscular administration, and subcutaneous administration. In particular, it is most preferable for the preparation to be used for the intramuscular administration or the subcutaneous administration. In the case of a freeze-dried preparation, as described above, the preparation can be administered to a patient by dissolving it in water, such as distilled water (or water for injection) at the time of use. In particular, in the case where the preparation is used for the intramuscular administration or the subcutaneous administration, approximately 0.1 mL to 2 mL, preferably 0.3 mL to 1.5 mL, particularly preferably 0.5 mL to 1 mL of the preparation is administered.

The dose of the high-concentrated soluble thrombomodulin-containing solution of the present invention varies according to the age and weight of the patient, the extent of the disease, the administrating path, and so on. In general, the amount of the soluble thrombomodulin is in the range of 0.001 to 5 mg/kg per weight, preferably 0.02 to 2 mg/kg, more preferably 0.05 to 1 mg/kg, particularly preferably 0.1 to 0.5 mg/kg, and the preparation is administered once or several times per day, if required. The intervals of administration may be every day, preferably once every 2 to 14 days, more preferably once every 3 to 10 days, still more preferably once every 4 to 7 days. In particular, in the intramuscular or subcutaneous administration, the above interval of administration is preferably prolonged with a blood concentration being maintained for a long period.

Furthermore, according to the present invention, there is provided a method of stabilizing soluble thrombomodulin in a soluble thrombomodulin-containing freeze-dried preparation that contains soluble thrombomodulin as an active ingredient and a preparation most suitable for such a method, the method being characterized by including: the presence of (1) urea or (2) urea and an amino acid in the preparation; and dissolving the soluble thrombomodulin-containing freeze-dried preparation in 0.1 mL to 2 mL of a dissolving aqueous solution to prepare a high-concentrated soluble thrombomodulin-containing solution having a concentration of 10 mg/mL or more, preferably 20 mg/mL or more with an osmotic pressure ratio in the range of 0.5 to 2.0 upon dissolution thereof.

The soluble thrombomodulin of the present invention is similar to one described above and the content thereof in the preparation is also similar to one described above, for example, generally 10 mg/mL or more, 15 mg/mL or more, or 17 mg/mL or more, preferably 20 mg/mL or more, 25 mg/mL or more, or 30 mg/mL or more.

Urea may be used in the form of an acid addition salt, for example such as urea phosphate or urea nitrate, but urea itself is preferable. In addition, when the addition amounts of the urea and the acid addition salt thereof are considered, otherwise specifically mentioned, the amounts are represented in terms of reduced value as free urea.

Preferable examples of an amino acid which can be combined with urea include glutamic acid, a salt thereof, asparaginic acid or a salt thereof. Detailed exemplifications of those amino acids and the salts thereof are as described above. The proportions, addition amounts, and osmotic pressure ratios of these additives are similar to those described above. Basically, the manufacturing method, the administration method, the dose, and the like can be taken the above disclosure into account. In addition, as described above, those compositions can be also utilized for a high-concentrated soluble thrombomodulin-containing preparation or a kit preparation thereof in the foam-inhibition method.

EXAMPLES

Hereinafter, the present invention will be specifically described with reference to reference examples, examples, comparative examples, administrating examples, and experimental examples. However, the present invention is not limited to these examples.

In the following reference examples, examples, and comparative examples, the following raw materials for preparations were used in addition to soluble thrombomodulin as an active ingredient.

Sodium L-glutamate monohydrate (special grade, available from Wako Pure Chemical Industries, Ltd.), D(-)-mannitol (special grade, available from Wako Pure Chemical Industries, Ltd.), L-lysine, hydrochloride (special grade, available from Wako Pure Chemical Industries, Ltd.), L-lysine-L-glutamate dihydrate (available from Ajinomoto Co.,Inc.),sodium L-aspartate monohydrate (specialgrade, available from Wako Pure Chemical Industries, Ltd.), urea (available from Kozakai Pharmaceutical Co., Ltd.), L-arginine hydrochloride (special grade, available from Wako Pure Chemical Industries , Ltd.)., sucrose (special grade, available from Wako Pure Chemical Industries, Ltd.), D-trehalose dihydrate (special grade, available from Wako Pure Chemical Industries, Ltd.), Polysorbate 80 (available from Nikko Chemicals Co., Ltd.), Polysorbate 20 (available from Nikko Chemicals Co., Ltd.), Poloxamer 188 (available from BASF Aktiengesellschaft), Polyoxyethylene (25) lauryl ether (available from Nikko Chemicals Co., Ltd.), Polyoxyl 40 stearate (available from Nikko Chemicals Co., Ltd.), HCO-60 (Polyoxyl 60 hydrogenated castor oil, available from Nikko Chemicals Co. , Ltd.), sodium lauryl sulfate (1st grade, available from Wako Pure Chemical Industries, Ltd.), benzalkonium chloride (available from ICN Biochemicals Inc.), benzyl alcohol (special grade, available from Wako Pure Chemical Industries, Ltd.), chlorobutanol (special grade, available from Wako Pure Chemical Industries, Ltd.), sodium chloride (special grade, available from Wako Pure Chemical Industries, Ltd.), phosphoric acid (special grade, available from Wako Pure Chemical Industries, Ltd.), sodium hydroxide (special grade, available from Wako Pure Chemical Industries, Ltd.), water for injection (available from Otsuka Pharmaceutical Co., Ltd.), and physiological saline (available from Otsuka Pharmaceutical Co., Ltd.).

Reference Example 1

Production of soluble thrombomodulin used in this example

Soluble thrombomodulin used in the example was obtained by production according to the above method of Yamamoto et al. (a method described in Example 10 of JP 64-006219 A) and concentration through ultrafiltration. That is, DNA encoding the amino acid sequence of SEQ ID NO. 1 in the sequence listing (specifically, consisting of the base sequence of SEQ ID NO. 2 in the sequence listing) was transfected into Chinese hamster ovary (CHO) cells, and a high purity product was then obtained from the culture medium of the transformed cells such that a active fraction was collected with a 20 mM phosphate buffer (pH 7.3) containing 50 mM NaCl by the above or usual purification method. Moreover, TMD123H having a soluble thrombomodulin concentration of 60 mg/mL was obtained by performing ultrafiltration. Similarly, using DNA encoding the amino acid sequence of SEQ ID NO. 3 in the sequence listing (specifically, comprising the base sequence of SEQ ID NO. 4 in the sequence listing), a peptide (hereinafter, which may be abbreviated as TME456H) at least having amino acids at positions 19 to 132 of the amino acid sequence of SEQ ID NO. 3 in the sequence listing was obtained.

Furthermore, DNA encoding the amino acid sequence of SEQ ID NO. 5 in the sequence listing (specifically, comprising the base sequence of SEQ ID NO. 6 in the sequence listing) was obtained by performing the above site-specific mutation using a DNA fragment containing the base sequence of SEQ ID NO. 2 in the sequence listing and synthetic DNA for mutation containing the base sequence represented in SEQ ID NO. 9 in the sequence listing. This DNA sequence was transfected into CHO cells, and a high purity product was obtained from the culture medium of the transformed cells such that a active fraction was collected with a 20-mM phosphate buffer (pH 7.3) containing 50 mM NaCl by the above or usual purification method. Furthermore, a peptide (hereinafter, which may be abbreviated as TMD123HM) having at least amino acids at positions 19 to 516 of SEQ ID NO. 5 in the sequence listing and having a soluble thrombomodulin concentration of 60 mg/mL was obtained by performing ultrafiltration. Similarly, DNA encoding the amino acid sequence of SEQ ID NO. 7 in the sequence listing (specifically, comprising the base sequence of SEQ ID NO. 8 in the sequence listing) was obtained by performing the above site-specific mutation using a DNA fragment containing the base sequence of SEQ ID NO. 4 in the sequence listing and synthetic DNA for mutation containing the base sequence represented in SEQ IDNO. 9 in the sequence listing. This DNA sequence was transfected into CHO cells and then a peptide (hereinafter, which may be abbreviated as TME456HM) having at least amino acids at positions 19 to 132 of SEQ ID NO. 7 in the sequence listing was obtained by the method described above.

Example 1-1

Preparation of additive solution

In a 20 mL measuring flask, 2.5 mmol of sodium L-glutamate monohydrate and 2.5 mmol of D- (-) -mannitol were weighed and placed (each of which was 50 times as high as the amount of the additive described in Table 1). Then, water for injection was added to the mixture and dissolved, followed by adjusting 20 mL in all.

Preparation of sample solution

4 mL of the above additive solution was placed in a 15 mL assist tube. Then 5 mL of TMD123H (containing 300 mg of soluble thrombomodulin, the amount corresponding to 10 times as high as one described in table 1) and 1 mL of water for injection were added to the tube and stirred. The sample solution was sterilized by filtration through a filter of 0.22 μm in pore size (MILLEX-GV, manufactured by Millipore) using a 25 mL disposal syringe (manufactured by Terumo Corp.), otherwise specifically limited, followed by dispensing each 1 mL thereof into sterile vials (3010, manufactured by Fuji Glass) which were not coated with silicone.

Freeze-drying

A high-concentrated soluble thrombomodulin-containing preparation containing 30 mg of soluble thrombomodulin, 0.05 mmol of sodium L-glutamate monohydrate, and 0.05 mmol of D (-) -mannitol in one container was obtained by performing a freeze-drying step under the conditions described below in the order of: plugging by half with a rubber stopper (manufactured by Daikyo Seiko, Ltd.) → freeze-drying → nitrogen-filling → plugging with the rubber stopper → winding a cap up (Example 1-1-1).

[Freeze-drying conditions]

Preliminary cooling (from room temperature to 15°C while spending 15 minutes) →main cooling (from 15°C to -45°C while spending 2 hours) → retaining (-45°C for 2 hours) → vacuum-starting (-45°C for 18 hours) → warming-up (from -45°C to 25°C while spending 20 hours) → retaining (25°C for 15 hours) → warming-up (from 25°C to 45°C while spending 1 hour) → retaining (45°C for 5 hours) → lowering to room temperature (from 45°C to 25°C while spending 2 hours) → nitrogen-filling for recovering pressure (recovering pressure up to -13332,2 Pa (-100 mmHg) with nitrogen) → plugging → winding a cap up.

Example 1-1-2, Example 1-1-3, and Example 1-1-4

Similarly, high-concentrated soluble thrombomodulin-containing preparations were obtained according to the above method by changing the types of soluble thrombomodulin, that is, by using TMD123HM (Example 1-1-2), TME456H (Example 1-1-3), or TME456HM (Example 1-1-4) instead of TMD123H of Example 1-1-1.

Table 1

| Example & Comparative Example | Composition | Addition amount per container |
|---|---|---|
| Example 1-1-1 | TMD123H<br>Sodium L-glutamate monohydrate<br>D(-)-mannitol | 30 mg<br>0.05 mmol<br>0.05 mmol |
| Example 1-1-2 | TMD123HM<br>Sodium L-glutamate monohydrate<br>D(-)-mannitol | 30 mg<br>0.05 mmol<br>0.05 mmol |

(continued)

| Example & Comparative Example | Composition | Addition amount per container |
|---|---|---|
| Example 1-1-3 | TME456H<br>Sodium L-glutamate monohydrate<br>D(-)-mannitol | 30 mg<br>0.05 mmol<br>0.05 mmol |
| Example 1-1-4 | TME456HM<br>Sodium L-glutamate monohydrate<br>D(-)-mannitol | 30 mg<br>0.05 mmol<br>0.05 mmol |
| Example 1-2-1 | TMD123H<br>Sodium L-glutamate monohydrate<br>D(-)-mannitol | 30 mg<br>0.075 mmol<br>0.075 mmol |
| Example 1-3-1 | TMD123H<br>Sodium L-glutamate monohydrate<br>D(-)-mannitol | 30 mg<br>0.025 mmol<br>0.025 mmol |
| Example 1-4-1 | TMD123H<br>Sodium L-glutamate monohydrate<br>D(-)-mannitol | 30 mg<br>0.02 mmol<br>0.10 mmol |
| Example 1-5-1 | TMD123H<br>Sodium L-glutamate monohydrate<br>D(-)-mannitol | 30 mg<br>0.07 mmol<br>0.02 mmol |
| Example 1-6-1 | TMD123H<br>Sodium L-glutamate monohydrate<br>D(-)-mannitol | 30 mg<br>0.03 mmol<br>0.15 mmol |
| Example 1-7-1 | TMD123H<br>Sodium L-glutamate monohydrate<br>D(-)-mannitol | 30 mg<br>0.10 mmol<br>0.03 mmol |
| Example 1-8-1 | TMD123H<br>Sodium L-glutamate monohydrate<br>L-lysine hydrochloride | 30 mg<br>0.05 mmol<br>0.05 mmol |
| Example 1-9-1 | TMD123H<br>Sodium L-glutamate monohydrate<br>L-lysine hydrochloride | 30 mg<br>0.03 mmol<br>0.07 mmol |
| Example 1-10-1 | TMD123H<br>(L-lysine-L-glutamate dihydrate)<br>Lysine<br>Glutamic acid | 30 mg<br><br>0.10 mmol<br>0.10 mmol |
| Example 1-11-1 | TMD123H<br>(L-lysine-L-glutamate dihydrate)<br>Lysine<br>Glutamic acid | 30 mg<br><br>0.15 mmol<br>0.15 mmol |
| Example 1-12-1 | TMD123H<br>Sodium L-glutamate monohydrate<br>Sodium L-aspartate monohydrate | 30 mg<br>0.06 mmol<br>0.06 mmol |
| Example 1-13-1 | TMD123H<br>Sodium L-aspartate monohydrate<br>D(-)-mannitol | 30 mg<br>0.05 mmol<br>0.05 mmol |

(continued)

| Example & Comparative Example | Composition | Addition amount per container |
|---|---|---|
| Example 1-14-1 | TMD123H<br>Urea | 30 mg<br>0.07 mmol |
| Example 1-15-1 | TMD123H<br>Urea<br>Sodium L-glutamate monohydrate | 30 mg<br>0.05 mmol<br>0.05 mmol |
| Example 1-16-1 | TMD123H<br>Urea<br>Sodium L-aspartate monohydrate | 30 mg<br>0.05 mmol<br>0.05 mmol |
| Example 1-17-1 | TMD123H<br>Sodium L-glutamate monohydrate<br>D(-)-mannitol | 30 mg<br>0.05 mmol<br>0.05 mmol |
| Example 1-18-1 | TMD123H<br>Sodium L-aspartate monohydrate<br>D(-)-mannitol | 30 mg<br>0.05 mmol<br>0.05 mmol |
| Example 1-19-1 | TMD123H<br>Sodium L-glutamate monohydrate<br>D(-)-mannitol | 15 mg<br>0.025 mmol<br>0.025 mmol |
| Example 1-20-1 | TMD123H<br>Sodium L-glutamate monohydrate<br>D(-)-mannitol | 10 mg<br>0.017 mmol<br>0.017 mmol |
| Example 1-21-1 | TMD123H<br>Sodium L-glutamate monohydrate<br>D (-) -mannitol | 15 mg<br>0.038 mmol<br>0.038 mmol |
| Example 1-22-1 | TMD123H<br>Sodium L-glutamate monohydrate<br>D(-)-mannitol | 10 mg<br>0.025 mmol<br>0.025 mmol |
| Example 1-23-1 | TMD123H<br>Sodium L-glutamate monohydrate<br>D(-)-mannitol | 15 mg<br>0.0125 mmol<br>0.0125 mmol |
| Example 1-24-1 | TMD123H<br>Sodium L-glutamate monohydrate<br>D(-)-mannitol | 10 mg<br>0.0083 mmol<br>0.0083 mmol |
| Example 1-25-1 | TMD123H<br>Sodium L-aspartate monohydrate<br>D(-)-mannitol | 30 mg<br>0.075 mmol<br>0.075 mmol |
| Example 1-26-1 | TMD123H<br>Sodium L-aspartate monohydrate<br>D(-)-mannitol | 30 mg<br>0.025 mmol<br>0.025 mmol |
| Example 1-27-1 | TMD123H<br>Sodium L-aspartate monohydrate<br>D(-)-mannitol | 30 mg<br>0.02 mmol<br>0.10 mmol |
| Example 1-28-1 | TMD123H<br>Sodium L-aspartate monohydrate<br>D(-) -mannitol | 30 mg<br>0.07 mmol<br>0.02 mmol |

(continued)

| Example & Comparative Example | Composition | Addition amount per container |
|---|---|---|
| Example 1-29-1 | TMD123H<br>Sodium L-aspartate monohydrate<br>D(-)-mannitol | 30 mg<br>0.03 mmol<br>0.15 mmol |
| Example 1-30-1 | TMD123H<br>Sodium L-aspartate monohydrate<br>D(-)-mannitol | 30 mg<br>0.10 mmol<br>0.03 mmol |
| Example 1-31-1 | TMD123H<br>Sodium L-aspartate monohydrate<br>D(-)-mannitol | 15 mg<br>0.025 mmol<br>0.025 mmol |
| Example 1-32-1 | TMD123H<br>Sodium L-aspartate monohydrate<br>D(-)-mannitol | 10 mg<br>0.017 mmol<br>0.017 mmol |
| Example 1-33-1 | TMD123H<br>Sodium L-aspartate monohydrate<br>D(-)-mannitol | 15 mg<br>0.038 mmol<br>0.038 mmol |
| Example 1-34-1 | TMD123H<br>Sodium L-aspartate monohydrate<br>D(-)-mannitol | 10 mg<br>0.025 mmol<br>0.025 mmol |
| Example 1-35-1 | TMD123H<br>Sodium L-aspartate monohydrate<br>D(-)-mannitol | 15 mg<br>0.0125 mmol<br>0.0125 mmol |
| Example 1-36-1 | TMD123H<br>Sodium L-aspartate monohydrate<br>D(-)-mannitol | 10 mg<br>0.0083 mmol<br>0.0083 mmol |
| Example 1-37-1 | TMD123H<br>Sodium L-glutamate monohydrate<br>D(-)-mannitol<br>Sodium chloride<br>Phosphoric acid (neutralized to pH 7.3 with sodium hydroxide) | 30 mg<br>0.05 mmol<br>0.05 mmol<br>0.05 mmol<br>0.02 mmol |
| Example 1-38-1 | TMD123H<br>Sodium L-aspartate monohydrate<br>D(-)-mannitol<br>Sodium chloride<br>Phosphoric acid (neutralized to pH 7.3 with sodium hydroxide) | 30 mg<br>0.05 mmol<br>0.05 mmol<br>0.05 mmol<br>0.02 mmol |
| Example 1-39-1 | TMD123H<br>Sodium L-aspartate monohydrate<br>D(-)-mannitol<br>Polysorbate 80 | 30 mg<br>0.05 mmol<br>0.05 mmol<br>0.1 mg |
| Example 1-40-1 | TMD123H<br>Sodium L-glutamate monohydrate<br>D(-)-mannitol<br>Polysorbate 80 | 30 mg<br>0.05 mmol<br>0.05 mmol<br>0.1 mg |
| Example 1-41-1 | TMD123H | 30 mg |

(continued)

| Example & Comparative Example | Composition | Addition amount per container |
|---|---|---|
| | Sodium L-aspartate monohydrate<br>D(-)-mannitol<br>Poloxamer 188 | 0.05 mmol<br>0.05 mmol<br>0.1 mg |
| Example 1-42-1 | TMD123H<br>Sodium L-aspartate monohydrate<br>D(-)-mannitol | 30 mg<br>0.05 mmol<br>0.05 mmol |
| | Polysorbate 80 (added in a dissolving aqueous solution) | 0.1 mg |
| Example 1-43-1 | TMD123H<br>Sodium L-aspartate monohydrate<br>D(-)-mannitol | 30 mg<br>0.05 mmol<br>0.05 mmol |
| | Benzyl alcohol (added in a dissolving aqueous solution) | 10 mg |
| Example 1-44-1 | TMD123H<br>Sodium L-aspartate monohydrate<br>D(-)-mannitol | 30 mg<br>0.05 mmol<br>0.05 mmol |
| | Chlorobutanol (added in a dissolving aqueous solution) | 5 mg |
| Example 1-45-1 | TMD123H<br>Sodium L-glutamate monohydrate<br>D(-)-mannitol | 30 mg<br>0.05 mmol<br>0.05 mmol |
| | Polysorbate 80 (added in a dissolving aqueous solution) | 0.1 mg |
| Example 1-46-1 | TMD123H<br>Sodium L-glutamate monohydrate<br>D(-)-mannitol | 30 mg<br>0.05 mmol<br>0.05 mmol |
| | Benzyl alcohol (added in a dissolving aqueous solution) | 10 mg |
| Example 1-47-1 | TMD123H<br>Sodium L-glutamate monohydrate<br>D(-)-mannitol | 30 mg<br>0.05 mmol<br>0.05 mmol |
| | Chlorobutanol (added in a dissolving aqueous solution) | 5 mg |
| Example 1-48-1 | TMD123H<br>Sodium L-aspartate monohydrate<br>D(-)-mannitol<br>Polysorbate 80 | 30 mg<br>0.05 mmol<br>0.05 mmol<br>0.05 mg |
| | Polysorbate 80 (added in a dissolving aqueous solution) | 0.05 mg |
| Example 1-49-1 | TMD123H<br>Sodium L-aspartate monohydrate<br>D(-)-mannitol<br>Polysorbate 80 | 30 mg<br>0.05 mmol<br>0.05 mmol<br>0.05 mg |
| | Benzyl alcohol (added in a dissolving aqueous solution) | 5 mg |
| Example 1-50-1 | TMD123H<br>Sodium L-aspartate monohydrate<br>D(-)-mannitol<br>Polysorbate 80 | 30 mg<br>0.05 mmol<br>0.05 mmol<br>0.05 mg |
| | Chlorobutanol (added in a dissolving aqueous solution) | 2.5 mg |
| Example 1-51-1 | TMD123H | 30 mg |

(continued)

| Example & Comparative Example | Composition | Addition amount per container |
|---|---|---|
| | Sodium L-glutamate monohydrate<br>D(-)-mannitol<br>Polysorbate 80 | 0.05 mmol<br>0.05 mmol<br>0.05 mg |
| | Polysorbate 80 (added in a dissolving aqueous solution) | 0.05 mg |
| Example 1-52-1 | TMD123H<br>Sodium L-glutamate monohydrate<br>D(-)-mannitol<br>Polysorbate 80 | 30 mg<br>0.05 mmol<br>0.05 mmol<br>0.05 mg |
| | Benzyl alcohol (added in a dissolving aqueous solution) | 5 mg |
| Example 1-53-1 | TMD123H<br>Sodium L-glutamate monohydrate<br>D(-)-mannitol<br>Polysorbate 80 | 30 mg<br>0.05 mmol<br>0.05 mmol<br>0.05 mg |
| | Chlorobutanol (added in a dissolving aqueous solution) | 2.5 mg |
| Comparative Example 1-1 | TMD123H<br>L-arginine hydrochloride<br>Sucrose | 15 mg<br>0.75 mmol<br>0.75 mmol |
| Comparative Example 1-2 | TMD123H<br>L-arginine hydrochloride<br>Sucrose | 10 mg<br>0.5 mmol<br>0.5 mmol |
| Comparative Example 1-3 | TMD123H | 30 mg |
| Comparative Example 1-4 | TMD123H<br>Sodium L-glutamate monohydrate | 30 mg<br>0.025 mmol |
| Comparative Example 1-5 | TMD123H<br>D(-)-mannitol | 30 mg<br>0.025 mmol |
| Comparative Example 1-6 | TMD123H<br>Sodium L-glutamate monohydrate | 30 mg<br>0.05 mmol |
| Comparative Example 1-7 | TMD123H<br>D(-)-mannitol | 30 mg<br>0.05 mmol |
| Comparative Example 1-8 | TMD123H<br>Sodium L-glutamate monohydrate | 30 mg<br>0.075 mmol |
| Comparative Example 1-9 | TMD123H<br>D(-)-mannitol | 30 mg<br>0.075 mmol |
| Comparative Example 1-10 | TMD123H<br>Sodium L-aspartate monohydrate | 30 mg<br>0.025 mmol |
| Comparative Example 1-11 | TMD123H<br>Sodium L-aspartate monohydrate | 30 mg<br>0.05 mmol |
| Comparative Example 1-12 | TMD123H<br>Sodium L-aspartate monohydrate | 30 mg<br>0.075 mmol |

Example 1-2

High-concentrated soluble thrombomodulin-containing preparations listed in Table 1 were obtained in the same manner as in Example 1-1 (Example 1-2-1, Example 1-2-2, Example 1-2-3, and Example 1-2-4 were prepared by changing the types of soluble thrombomodulin according to Examples 1-1-1, 1-1-2, 1-1-3, and 1-1-4 respectively).

Example 1-3

High-concentrated soluble thrombomodulin-containing preparations listed in Table 1 were obtained in the same manner as in Example 1-1 (just like the above description, Example 1-3-1, Example 1-3-2, Example 1-3-3, and Example 1-3-4 were prepared respectively).

Example 1-4

High-concentrated soluble thrombomodulin-containing preparations listed in Table 1 were obtained in the same manner as in Example 1-1 (just like the above description, Example 1-4-1, Example 1-4-2, Example 1-4-3, and Example 1-4-4 were prepared respectively).

Example 1-5

High-concentrated soluble thrombomodulin-containing preparations listed in Table 1 were obtained in the same manner as in Example 1-1 (just like the above description, Example 1-5-1, Example 1-5-2, Example 1-5-3, and Example 1-5-4 were prepared respectively).

Example 1-6

High-concentrated soluble thrombomodulin-containing preparations listed in Table 1 were obtained in the same manner as in Example 1-1 (just like the above description, Example 1-6-1, Example 1-6-2, Example 1-6-3, and Example 1-6-4 were prepared respectively).

Example 1-7

High-concentrated soluble thrombomodulin-containing preparations listed in Table 1 were obtained in the same manner as in Example 1-1 (just like the above description, Example 1-7-1, Example 1-7-2, Example 1-7-3, and Example 1-7-4 were prepared respectively).

Example 1-8

High-concentrated soluble thrombomodulin-containing preparations listed in Table 1 were obtained in the same manner as in Example 1-1 (just like the above description, Example 1-8-1, Example 1-8-2, Example 1-8-3, and Example 1-8-4 were prepared respectively).

Example 1-9

High-concentrated soluble thrombomodulin-containing preparations listed in Table 1 were obtained in the same manner as in Example 1-1 (just like the above description, Example 1-9-1, Example 1-9-2, Example 1-9-3, and Example 1-9-4 were prepared respectively).

Example 1-10

High-concentrated soluble thrombomodulin-containing preparations listed in Table 1 were obtained in the same manner as in Example 1-1 (just like the above description, Example 1-10-1, Example 1-10-2, Example 1-10-3, and Example 1-10-4 were prepared respectively).

Example 1-11

High-concentrated soluble thrombomodulin-containing preparations listed in Table 1 were obtained in the same manner as in Example 1-1 (just like the above description, Example 1-11-1, Example 1-11-2, Example 1-11-3, and Example 1-11-4 were prepared respectively).

Example 1-12

High-concentrated soluble thrombomodulin-containing preparations listed in Table 1 were obtained in the same manner as in Example 1-1 (just like the above description, Example 1-12-1, Example 1-12-2, Example 1-12-3, and Example 1-12-4 were prepared respectively).

Example 1-13

High-concentrated soluble thrombomodulin-containing preparations listed in Table 1 were obtained in the same manner as in Example 1-1 (just like the above description, Example 1-13-1, Example 1-13-2, Example 1-13-3, and Example 1-13-4 were prepared respectively).

Example 1-14

High-concentrated soluble thrombomodulin-containing preparations listed in Table 1 were obtained in the same manner as in Example 1-1 (just like the above description, Example 1-14-1, Example 1-14-2, Example 1-14-3, and Example 1-14-4 were prepared respectively).

Example 1-15

High-concentrated soluble thrombomodulin-containing preparations listed in Table 1 were obtained in the same manner as in Example 1-1 (just like the above description, Example 1-15-1, Example 1-15-2, Example 1-15-3, and Example 1-15-4 were prepared respectively).

Example 1-16

High-concentrated soluble thrombomodulin-containing preparations listed in Table 1 were obtained in the same manner as in Example 1-1 (just like the above description, Example 1-16-1, Example 1-16-2, Example 1-16-3, and Example 1-16-4 were prepared respectively).

Example 1-17

In the preparation of the sample solution in Example 1-1, each 1 mL aliquot of the sample solution was dispensed into one chamber of two-chamber syringe (manufactured by Alter) instead of dispensing into the sterile vial. They were subjected to the freeze-drying step (under the same freeze-drying conditions as Example 1-1) in the order of: plugging with a middle stopper by half → freeze-drying → nitrogen-filling → plugging with the middle stopper. Subsequently, 0.75 mL of water for injection was aseptically filled in the other chamber and sealed with a rubber stopper, followed by providing with a plunger rod, resulting in kit preparations composed of the high-concentrated soluble thrombomodulin-containing preparations (like the above description, Example 1-17-1, Example 1-17-2, Example 1-17-3, and Example 1-17-4 were prepared respectively).

Example 1-18

In the preparation of the sample solution in Example 1-13, each 1 mL aliquot of the sample solution was dispensed into one chamber of two-chamber syringe (manufactured by Alter) instead of the sterile vial. They were subjected to the freeze-drying step (under the same freeze-drying conditions as Example 1-1) in the order of: plugging with a middle stopper by half → freeze-drying → nitrogen-filling → plugging with the middle stopper. Subsequently, 0.75 mL of water for injection was aseptically filled in the other chamber and sealed with a rubber stopper, followed by providing with a plunger rod, resulting in kit preparations composed of the high-concentrated soluble thrombomodulin-containing preparations (like the above description, Example 1-18-1, Example 1-18-2, Example 1-18-3, and Example 1-18-4 were prepared respectively).

Example 1-19

High-concentrated soluble thrombomodulin-containing preparations listed in Table 1 were obtained in the same manner as in Example 1-1, except that each 0.5 mL aliquot of a sterile-filtered sample solution was dispersed into a sterile vial (like the above description, Example 1-19-1, Example 1-19-2, Example 1-19-3, and Example 1-19-4 were prepared respectively).

Example 1-20

High-concentrated soluble thrombomodulin-containing preparations listed in Table 1 were obtained in the same manner as in Example 1-1, except that each 0.333 mL aliquot of a sterile-filtered sample solution was dispersed into a sterile vial (like the above description, Example 1-20-1, Example 1-20-2, Example 1-20-3, and Example 1-20-4 were prepared respectively).

Example 1-21.

High-concentrated soluble thrombomodulin-containing preparations listed in Table 1 were obtained in the same manner as in Example 1-2, except that each 0.5 mL aliquot of a sterile-filtered sample solution was dispersed into a sterile vial (like the above description, Example 1-21-1, Example 1-21-2, Example 1-21-3, and Example 1-21-4 were prepared respectively).

Example 1-22

High-concentrated soluble thrombomodulin-containing preparations listed in Table 1 were obtained in the same manner as in Example 1-2, except that each 0.333 mL aliquot of a sterile-filtered sample solution was dispersed into a sterile vial (like the above description, Example 1-22-1, Example 1-22-2, Example 1-22-3, and Example 1-22-4 were prepared respectively).

Example 1-23

High-concentrated soluble thrombomodulin-containing preparations listed in Table 1 were obtained in the same manner as in Example 1-3, except that each 0.5 mL aliquot of a sterile-filtered sample solution was dispersed into a sterile vial (like the above description, Example 1-23-1, Example 1-23-2, Example 1-23-3, and Example 1-23-4 were prepared respectively).

Example 1-24

High-concentrated soluble thrombomodulin-containing preparations listed in Table 1 were obtained in the same manner as in Example 1-3, except that each 0.333 mL aliquot of a sterile-filtered sample solution was dispersed into a sterile vial (like the above description, Example 1-24-1, Example 1-24-2, Example 1-24-3, and Example 1-24-4 were prepared respectively).

Example 1-25

High-concentrated soluble thrombomodulin-containing preparations listed in Table 1 were obtained in the same manner as in Example 1-1 (just like the above description, Example 1-25-1, Example 1-25-2, Example 1-25-3, and Example 1-25-4 were prepared respectively).

Example 1-26

High-concentrated soluble thrombomodulin-containing preparations listed in Table 1 were obtained in the same manner as in Example 1-1 (just like the above description, Example 1-26-1, Example 1-26-2, Example 1-26-3, and Example 1-26-4 were prepared respectively).

Example 1-27

High-concentrated soluble thrombomodulin-containing preparations listed in Table 1 were obtained in the same manner as in Example 1-1 (just like the above description, Example 1-27-1, Example 1-27-2, Example 1-27-3, and Example 1-27-4 were prepared respectively).

Example 1-28

High-concentrated soluble thrombomodulin-containing preparations listed in Table 1 were obtained in the same manner as in Example 1-1 (just like the above description, Example 1-28-1, Example 1-28-2, Example 1-28-3, and Example 1-28-4 were prepared respectively).

Example 1-29

High-concentrated soluble thrombomodulin-containing preparations listed in Table 1 were obtained in the same manner as in Example 1-1 (just like the above description, Example 1-29-1, Example 1-29-2, Example 1-29-3, and Example 1-29-4 were prepared respectively).

Example 1-30

High-concentrated soluble thrombomodulin-containing preparations listed in Table 1 were obtained in the same manner as in Example 1-1 (just like the above description, Example 1-30-1, Example 1-30-2, Example 1-30-3, and Example 1-30-4 were prepared).

Example 1-31

High-concentrated soluble thrombomodulin-containing preparations listed in Table 1 were obtained in the same manner as in Example 1-13, except that each 0.5 mL aliquot of a sterile-filtered sample solution was dispersed into a sterile vial (like the above description, Example 1-31-1, Example 1-31-2, Example 1-31-3, and Example 1-31-4 were prepared respectively).

Example 1-32

High-concentrated soluble thrombomodulin-containing preparations listed in Table 1 were obtained in the same manner as in Example 1-13, except that each 0.333 mL aliquot of a sterile-filtered sample solution was dispersed into a sterile vial (like the above description, Example 1-32-1, Example 1-32-2, Example 1-32-3, and Example 1-32-4 were prepared respectively).

Example 1-33

High-concentrated soluble thrombomodulin-containing preparations listed in Table 1 were obtained in the same manner as in Example 1-25, except that each 0.5 mL aliquot of a sterile-filtered sample solution was dispersed into a sterile vial (like the above description, Example 1-33-1, Example 1-33-2, Example 1-33-3, and Example 1-33-4 were prepared respectively).

Example 1-34

High-concentrated soluble thrombomodulin-containing preparations listed in Table 1 were obtained in the same manner as in Example 1-25, except that each 0.333 mL aliquot of a sterile-filtered sample solution was dispersed into a sterile vial (like the above description, Example 1-34-1, Example 1-34-2, Example 1-34-3, and Example 1-34-4 were prepared respectively).

Example 1-35

High-concentrated soluble thrombomodulin-containing preparations listed in Table 1 were obtained in the same manner as in Example 1-26, except that each 0.5 mL aliquot of a sterile-filtered sample solution was dispersed into a sterile vial (like the above description, Example 1-35-1, Example 1-35-2, Example 1-35-3, and Example 1-35-4 were prepared respectively).

Example 1-36

High-concentrated soluble thrombomodulin-containing preparations listed in Table 1 were obtained in the same manner as in Example 1-26, except that each 0.333 mL aliquot of a sterile-filtered sample solution was dispersed into a sterile vial (like the above description, Example 1-36-1, Example 1-36-2, Example 1-36-3, and Example 1-36-4 were prepared respectively).

Example 1-37

Preparation of additive solution

In a 20 mL measuring flask, 2.5 mmol of sodium L-glutamate monohydrate and 2.5 mmol of D- (-) -mannitol were weighed and placed. Then, water for injection was added to the mixture and dissolved to adjust the solution to 20 mL in all.

Preparation of sample solution

A 4 mL aliquot of the above additive solution was placed in a 15 mL assist tube. Then 5 mL of TMD123H (containing 300 mg of soluble thrombomodulin) obtained in Reference Example 1 and 1 mL of 20 mM phosphate buffer (pH 7.3) containing 50 mM NaCl were added to the tube and stirred. The sample solution was sterilized by filtration through a filter of 0.22 μm in pore size (MILLEX-GV, manufactured by Millipore) using a 25 mL disposal syringe (manufactured by Terumo Corp.), followed by dispensing each 1 mL thereof into sterile vials (3010, manufactured by Fuji Glass).

Freeze-drying

A high-concentrated soluble thrombomodulin-containing preparation was obtained by performing a freeze-drying step under the conditions described below in the order of: plugging with a rubber stopper by half → freeze-drying → nitrogen-filling plugging with the rubber stopper → winding a cap up (Example 1-37-1) [Method of preparing 20-mM phosphate buffer (pH 7.3) containing 50 mM NaCl]

In a 1000 mL beaker, 2,306 mg of phosphoric acid and 2,937 mg of sodium chloride are weighted and placed. Then, 750 mL of water for injection is added to the beaker, and the contents are stirred for dissolution. Then, the pH of the resultant solution is adjusted to pH7.3 with a 17% sodium hydroxide solution (100 mL of solution prepared by dissolving 17.71 g of sodium hydroxide in water for injection). Then, water for injection is added to adjust a solution to 1,000 mL in all.

[Freeze-drying conditions]

Preliminary cooling (from room temperature to 15°C while spending 15 minutes) → main cooling (from 15°C to -45°C while spending 2 hours) → retaining (-45°C for 2 hours) → vacuum-starting (-45°C for 18 hours) → warming-up (from -45°C to 25°C while spending 20 hours) → retaining (25°C for 15 hours) → warming-up (from 25°C to 45°C while spending 1 hour) → retaining (45°C for 5 hours) → lowering to room temperature (from 45°C to 25°C while spending 2 hours) → nitrogen-filling for recovering pressure (recovering pressure up to -13332,2 Pa (-100 mmHg) with nitrogen) → plugging → winding a cap up.

Similarly, high-concentrated soluble thrombomodulin-containing preparations were obtained according to the above method by changing the types of soluble thrombomodulin, i.e. using TMD123HM (Example 1-37-2), TME456H (Example 1-37-3), and TME456HM (Example 1-37-4) instead of TMD123H obtained in Reference Example 1.

Example 1-38

High-concentrated soluble thrombomodulin-containing preparations were obtained in the same manner as in Example 1-37, except that sodium L-aspartate monohydrate was adopted instead of sodium L-glutamate monohydrate (by changing the types of soluble thrombomodulin according to Examples 1-37-1, 1-37-2, 1-37-3, and 1-37-4, Example 1-38-1, Example 1-38-2, Example 1-38-3, and Example 1-38-4 were prepared respectively).

Example 1-39

Preparation of additive solution

In a 20 mL measuring flask, 2.5 mmol of sodium L-aspartate monohydrate, 2.5 mmol of D (-) -mannitol, and Polysorbate 80 (5 mg) were weighed and placed. Then water for injection was added and dissolved to adjust the solution to 20 mL in all.

High-concentrated soluble thrombomodulin-containing preparations were obtained in the same manner as in Example 1-1, except that the additive solution was prepared as described above (like the description described above, Example 1-39-1, Example 1-39-2, Example 1-39-3, and Example 1-39-4 were prepared respectively).

Example 1-40

Preparation of additive solution

In a 20 mL measuring flask, 2.5 mmol of sodium L-glutamate monohydrate, 2.5 mmol of D(-)-mannitol, and Polysorbate 80 (5 mg) were weighed and placed. Then water for injection was added and dissolved to adjust the solution to 20 mL in all.

A high-concentrated soluble thrombomodulin-containing preparation was obtained in the same manner as in Example 1-1, except that the additive solution was prepared as described above (like the description described above, preparation in Example 1-40-1 was prepared).

Example 1-41

Preparation of additive solution

In a 20 mL measuring flask, 2.5 mmol of sodium L-aspartate monohydrate, 2.5 mmol of D(-)-mannitol, and Polysorbate 188 (5 mg) were weighed and placed. Then water for injection was added and dissolved to adjust the solution to 20 mL in all.

A high-concentrated soluble thrombomodulin-containing preparation was obtained in the same manner as in Example 1-1, except that the additive solution was prepared as described above (like the description described above, preparation in Example 1-41-1 was prepared).

Example 1-42

A kit preparation of a soluble thrombomodulin-containing freeze-dried preparation was obtained by combining the soluble thrombomodulin-containing freeze-dried preparation obtained in Example 1-13 and an ampule aseptically filled with 1 mL of solution containing 0.1 mg of Polysorbate 80 in 1 mL of water for injection as a dissolving aqueous solution (like the above description, Example 1-42-1 was prepared).

Example 1-43

A kit preparation of a soluble thrombomodulin-containing freeze-dried preparation was obtained by combining the soluble thrombomodulin-containing freeze-dried preparation obtained in Example 1-13 and an ampule aseptically filled with 1 mL of solution containing 10 mg benzyl alcohol in 1 mL of water for injection as a dissolving aqueous solution (like the above description, the Example 1-43-1 was prepared).

Example 1-44

A kit preparation of a soluble thrombomodulin-containing freeze-dried preparation was obtained by combining the soluble thrombomodulin-containing freeze-dried preparation obtained in Example 1-13 and an ampule aseptically filled with 1 mL of solution containing 5 mg chlorobutanol in 1 mL of water for injection as a dissolving aqueous solution (the Example 1-44-1 was prepared).

Example 1-45

Kit preparations of soluble thrombomodulin-containing freeze-dried preparations were obtained by combining the soluble thrombomodulin-containing freeze-dried preparation obtained in Example 1-1 and an ampule aseptically filled with 1 mL of solution containing 0.1 mg of Polysorbate 80 in 1 mL of water for injection as a dissolving aqueous solution (like the above description, Example 1-45-1, Example 1-45-2, Example 1-45-3, and Example 1-45-4 were prepared respectively).

Example 1-46

A kit preparation of a soluble thrombomodulin-containing freeze-dried preparation was obtained by combining the soluble thrombomodulin-containing freeze-dried preparation obtained in Example 1-1 and an ampule aseptically filled with 1 mL of solution containing 10 mg of benzyl alcohol in 1 mL of water for injection as a dissolving aqueous solution (like the above description, Example 1-46-1 was prepared).

Example 1-47

A kit preparation of a soluble thrombomodulin-containing freeze-dried preparation was obtained by combining the soluble thrombomodulin-containing freeze-dried preparation obtained in Example 1-1 and an ampule aseptically filled with 1 mL of solution containing 5 mg of chlorobutanol in 1 mL of water for injection as a dissolving aqueous solution (like the above description, Example 1-47- was prepared).

Example 1-48

Preparation of additive solution

In a 20 mL measuring flask, 2.5 mmol of sodium L-aspartate monohydrate, 2.5 mmol of D(-)-mannitol, and Polysorbate 80 (2.5 mg) were weighed and placed. Then water for injection was added and dissolved to adjust the solution to 20 mL in all.

A kit preparation of a soluble thrombomodulin-containing freeze-dried preparation was obtained by combining the soluble thrombomodulin-containing freeze-dried preparation obtained in the same manner as in Example 1-1 except that the additive solution was prepared as described above and an ampule aseptically filled with 1 mL of solution containing 0.05 mg of Polysorbate 80 in 1 mL of water for injection as a dissolving aqueous solution (like the above description, Example 1-48-1 was prepared).

Example 1-49

Preparation of additive solution

In a 20 mL measuring flask, 2.5 mmol of sodium L-aspartate monohydrate, 2.5 mmol of D(-)-mannitol, and Polysorbate 80 (2.5 mg) were weighed and placed. Then water for injection was added and dissolved to adjust the solution to 20 mL in all.

A kit preparation of a soluble thrombomodulin-containing freeze-dried preparation was obtained by combining the soluble thrombomodulin-containing freeze-dried preparation obtained in the same manner as in Example 1-1 except that the above additive solution was prepared as described above and an ampule aseptically filled with 1 mL of solution containing 5 mg of benzyl alcohol in 1 mL of water for injection as a dissolving aqueous solution (like the above description, Example 1-49-1 was prepared).

Example 1-50

Preparation of additive solution

In a 20 mL measuring flask, 2.5 mmol of sodium L-aspartate monohydrate, 2.5 mmol of D(-)-mannitol, and Polysorbate 80 (2.5 mg) were weighed and placed. Then water for injection was added and dissolved to adjust the solution to 20 mL in all.

A kit preparation of a soluble thrombomodulin-containing freeze-dried preparation was obtained by combining the soluble thrombomodulin-containing freeze-dried preparation obtained in the same manner as in Example 1-1 except that the above additive solution was prepared as described above and an ampule aseptically filled with 1 mL of solution containing 2.5 mg of chlorobutanol in 1 mL of water for injection as a dissolving aqueous solution (like the above description, Example 1-50-1 was prepared).

Example 1-51

Preparation of additive solution

In a 20 mL measuring flask, 2.5 mmol of sodium L-glutamate monohydrate, 2.5 mmol of D(-)-mannitol, and Polysorbate 80 (2.5 mg) were weighed and placed. Then water for injection was added and dissolved to adjust the solution to 20 mL in all.

A kit preparation of a soluble thrombomodulin-containing freeze-dried preparation was obtained by combining the soluble thrombomodulin-containing freeze-dried preparation obtained in the same manner as in Example 1-1 except that the above additive solution was prepared as described above and an ampule aseptically filled with 1 mL of solution containing 0.05 mg of Polysorbate 80 in 1 mL of water for injection as a dissolving aqueous solution (like the above description, Example 1-51-1 was prepared).

Example 1-52

Preparation of additive solution

In a 20 mL measuring flask, 2.5 mmol of sodium L-glutamate monohydrate, 2.5 mmol of D(-)-mannitol, and Polysorbate 80 (2.5 mg) were weighed and placed. Then water for injection was added and dissolved to adjust the solution to 20 mL in all.

A kit preparation of a soluble thrombomodulin-containing freeze-dried preparation was obtained by combining the soluble thrombomodulin-containing freeze-dried preparation obtained in the same manner as in Example 1-1 except that the above additive solution was prepared as described above and an ampule aseptically filled with 1 mL of solution containing 5 mg of benzyl alcohol in 1 mL of water for injection as a dissolving aqueous solution (like the above description, Example 1-52-1 was prepared).

Example 1-53

Preparation of additive solution

In a 20 mL measuring flask, 2.5 mmol of sodium L-glutamate monohydrate, 2.5 mmol of D(-)-mannitol, and Polysorbate 80 (2.5 mg) were weighed and placed. Then water for injection was added and dissolved to adjust the solution to 20 mL in all.

A kit preparation of a soluble thrombomodulin-containing freeze-dried preparation was obtained by combining the soluble thrombomodulin-containing freeze-dried preparation obtained in the same manner as in Example 1-1 except that the above additive solution was prepared as described above and an ampule aseptically filled with 1 mL of solution containing 2.5 mg of chlorobutanol in 1 mL of water for injection as a dissolving aqueous solution (like the above description, Example 1-53-1 was prepared).

Comparative Example 1-1

In a 20 mL measuring flask, 20 mmol of L-arginine hydrochloride and 20 mmol of sucrose were weighed and placed. Then water for injection was added and dissolved to adjust the solution to 20 mL in all. 7.5 mL of this solution was placed in a 15 mL assist tube. Then 2.5 mL of TMD123H (containing 150 mg of soluble thrombomodulin) obtained in Reference Example 1 was added and the whole was stirred for mixing. The sample solution was sterilizedby filtration through a filterof 0.22 μm inporesize (MILLEX-GV, manufactured by Millipore) using a 25 mL disposable syringe (manufactured by Terumo Corp.), otherwise specifically limited, followed by dispensing each 1 mL thereof into sterile vials (3010, manufactured by Fuji Glass) which were not coated with silicone.

Freeze-drying

A composition containing 15 mg of soluble thrombomodulin, 0.75 mmol of L-arginine hydrochloride, and 0.75 mmol of sucrose in a container was obtained by performing a freeze-drying step under the conditions described in Example 1-1 in the order of: plugging with a rubber stopper by half → Freeze-drying → nitrogen-filling → plugging with the rubber stopper → winding a cap up.

Comparative Example 1-2

In a 20 mL measuring flask, 20 mmol of L-arginine hydrochloride and 20 mmol of sucrose were weighed and placed. Then water for injection was added and dissolved to adjust the solution to 20 mL in all. A composition listed in Table 1 was obtained in the same manner as in Comparative Example 1-1, except that 5 mL of this solution was placed in a 15mL assist tube and then 1.67 mL of TMD123H (containing 100 mg of soluble thrombomodulin) obtained in Reference Example 1 and 3.33 mL of water for injection were added and the whole was stirred for mixing.

Comparative Example 1-3

A composition listed in Table 1 was obtained in the same manner as in Comparative Example 1-1, except that 5 mL of TMD123H (containing 300 mg of soluble thrombomodulin) obtained in Reference Example 1 and 5 mL of water for injection were added to a 15 mL assist tube and the whole was stirred for mixing.

Comparative Example 1-4

A composition listed in Table 1 was obtained in the same manner as in Example 1-1, except that in the preparation of the additive solution 1.25 mmol of additive listed in Table 1 was weighed and used.

Comparative Example 1-5

A composition listed in Table 1 was obtained in the same manner as in Example 1-1, except that in the preparation of the additive solution 1.25 mmol of additive listed in Table 1 was weighed and used.

Comparative Example 1-6

A composition listed in Table 1 was obtained in the same manner as in Example 1-1, except that in the preparation of the additive solution 2.5 mmol of additive listed in Table 1 was weighed and used.

Comparative Example 1-7

A composition listed in Table was obtained in the same manner as in Example 1-1, except that in the preparation of the additive solution 2.5 mmol of additive listed in Table 1 was weighed and used.

Comparative Example 1-8

A composition listed in Table 1 was obtained in the same manner as in Example 1-1, except that in the preparation of the additive solution 3.75 mmol of additive listed in Table 1 was weighed and used.

Comparative Example 1-9

A composition listed in Table 1 was obtained in the same manner as in Example 1-1, except that in the preparation of the additive solution 3.75 mmol of additive listed in Table 1 was weighed and used.

Comparative Example 1-10

A composition listed in Table 1 was obtained in the same manner as in Example 1-1, except that in the preparation of the additive solution 1.25 mmol of additive listed in Table 1 was weighed and used.

Comparative Example 1-11

A composition listed in Table 1 was obtained in the same manner as in Example 1-1, except that in the preparation of the additive solution 2.5 mmol of additive listed in Table 1 was weighed and used.

Comparative Example 1-12

A composition listed in Table 1 was obtained in the same manner as in Example 1-1, except that in the preparation of the additive solution 3.75 mmol of additive listed in Table 1 was weighed and used.

Administration Example 1

An administration composition was obtained by redissolving the high-concentrated soluble thrombomodulin-containing preparation obtained in Example 1-1-1 with the addition of 0.75 mL of water for injection.

Administration Example 2

An administration composition was obtained by redissolving the high-concentrated soluble thrombomodulin-containing preparation obtained in Example 1-1-2 with the addition of 0.75 mL of water for injection.

Administration Example 3

An administration composition was obtained by redissolving the high-concentrated soluble thrombomodulin-containing preparation obtained in Example 1-1-3 with the addition of 0.75 mL of water for injection.

Administration Example 4

An administration composition was obtained by redissolving the high-concentrated soluble thrombomodulin-containing preparation obtained in Example 1-1-4 with the addition of 0.75 mL of water for injection.

Administration Example 5

An administration composition was obtained by redissolving the high-concentrated soluble thrombomodulin-containing preparation obtained in Example 1-2-1 with the addition of 1.0 mL of water for injection.

Administration Example 6

An administration composition was obtained by redissolving the high-concentrated soluble thrombomodulin-containing preparation obtained in Example 1-1-1 with the addition of 1.5 mL of water for injection.

Administration Example 7

An administration composition was obtained by redissolving the high-concentrated soluble thrombomodulin-containing preparation obtained in Example 1-1-1 with the addition of 0.5 mL of water for injection.

Comparative Administration Example 1

A physiological saline was used as an administration composition.

Comparative Administration Example 2

An administration composition was obtained by dissolving 9.00 g of sodium chloride in water for injection to adjust the volume to 100 mL in all.

Comparative Administration Example 3

An administration composition was obtained by redissolving the composition obtained in Comparative Example 1-1 with the addition of 1.0 mL of water for injection.

Comparative Administration Example 4

An administration composition was obtained by redissolving the composition obtained in Comparative Example 1-2 with the addition of 2.0 mL of water for injection.

Comparative Administration Example 5

An administration composition was obtained by redissolving the composition obtained in Comparative Example 1-3 with the addition of 1.0 mL of water for injection.

Experimental Example 1

Pain Test

The degree of pain caused by the administration solution used in Administration Example or Comparative Administration Example shown in Table 2 was evaluated according to a method of Quartaroli M. et al. (J. Pharmacol. Exp. Ther., 290 (1): 158-69, 1999). CD mice (25-30 g, Charles River) were fasted overnight, divided into 12 groups, and then acclimated by placing them in individual transparent cages for 15 minutes. 20 μL of the administration solution of each of Administration Examples and Comparative Administration Examples was subcutaneously administered into the left hind leg. The mice were returned into the individual cages immediately after the administration. Then the duration of lifting the left hind leg and the duration of licking the administrated site were totally counted for 5 minutes and provided as the index of pain. Each experiment was performed on every one mouse and the average time was obtained with respect to 1 group (n) = 6 mice. The results are shown in Table 2.

Experimental Example 2

An osmotic pressure ratio of each administration solution used in Experimental Example 1 was determined.

Method of determining osmotic pressure ratio

The osmotic pressure ratio was defined as a ratio of the osmolality of a sample solution to the osmolality of a physiological saline and (the 14th-revised version of Japanese pharmacopoeia, the osmotic pressure assay) and was calculated from the following equation because the osmolality of the physiological saline (0.900 g/100 mL) is constant (286 mOsm):

[Osmotic pressure ratio

= osmolality (mOsm) of the sample solution / 286 (mOsm)].

The osmolality was obtained by the osmotic pressure assay of the 14th-revised version of Japanese pharmacopoeia. That is, the osmolality of the sample solution was determined using an osmotic-pressure measuring device (OM802-D, manufactured by VOGEL), which had been previously calibrated by a two-point calibration procedure according to the following method to confirm the adaptability of the device in advance.

Calibration method of device

A zero-point adjustment was per formed using water for injection (available from Otsuka Pharmaceutical Co., Ltd.) and then calibration was performed using device-calibrating osmolality-standard solutions of 200 mOsm and 400 mOsm.

[Preparation of device-calibrating osmolality-standard solution of 200 mOsm]

Sodium chloride (Wako Pure Chemical Industries, Ltd.) was dried for 50 minutes at 600°C and left standing to cool in a desiccator (silica gel). 0.626 g of the sodium chloride was exactly weighed and dissolved with the addition of 100 g of water for injection.

[Preparation of device-calibrating osmolality-standard solution of 400 mOsm]

Sodium chloride (Wako Pure Chemical Industries , Ltd.) was dried for 50 minutes at 600°C and left standing to cool in a desiccator (silica gel). 1.270 g of the sodium chloride was exactly weighed and dissolved with the addition of 100 g of water for injection.

Adaptability of device

A 300 mOsm standard solution (available from VOGEL) was measured repeatedly for 6 times to confirm that the relative standard deviation was not more than 2% with respect to the 6-time measurement and the measurement values were in the range of 291 to 309 mOsm.

Measurement of sample solution

Each administration solution was taken in a dedicated sample cap (manufactured by VOGEL) and subjected to measurement. In addition, the administration solutions obtained in Comparative Administration Examples 2, 3, and 4 were diluted with water for injection (available from Otsuka Pharmaceutical Co., Ltd.) to 10-, 10-, and 3-fold, respectively. Then, the same measurement was performed on those solutions and the resulting osmolality levels were multiplied 10-, 10-, and 3-fold, respectively. The results were shown in Table 2.

As is evident from Table 2, it was found that each of the administration solutions of Administration Examples 1 to 7 had an extremely short pain duration in mice, the duration being almost equal to that of the physiological saline of Comparative Administration Example 1, and that the degree of pain was not high. The administration solutions of Comparative Administration Examples 2 to 4 each had a long duration of pain, so that they were confirmed not to be preferable because of their high degrees of pain. As a result of investigating the osmotic pressure ratio of Experimental Example 2, as shown in Table 2, a composition having a short pain duration had an osmotic pressure ratio of approximately 1 and a composition having a remarkably long pain duration had high or low in osmotic pressure ratio.

As is evident from Table 2, the osmotic pressure ratio at the time of dissolving the high-concentrated soluble thrombomodulin-containing solution obtained in Example in 0.1 mL to 2 mL of dissolving aqueous solution showed osmotic pressure ratio of approximately 1. Thus, it becomes evident that the preparation can be adopted into the administration methods such as intramuscular administration and subcutaneously administration.

Table 2

| Administration Example | Administration solution | | Pain duration (second) | Osmotic pressure ratio |
|---|---|---|---|---|
| | Composition | Amount of redissolved solution (ml) | | |
| Administration Example 1 | Example 1-1-1 | 0.75 | 15 | 1.1 |
| Administration Example 2 | Example 1-1-2 | 0.75 | 14 | 1.1 |
| Administration Example 3 | Example 1-1-3 | 0.75 | 16 | 1.1 |
| Administration Example 4 | Example 1-1-4 | 0.75 | 17 | 1.1 |
| Administration Example 5 | Example 1-2-1 | 1.0 | 15 | 1.0 |
| Administration Example 6 | Example 1-1-1 | 1.5 | 19 | 0.6 |
| Administration Example 7 | Example 1-1-1 | 0.5 | 20 | 1.6 |
| Comparative Administration Example 1 | Physiological saline | - | 12 | 1.0 |
| Comparative Administration Example 2 | 9% saline | - | 125 | 10.0 |
| Comparative Administration Example 3 | Comparative Example 1-1 | 1.0 | 127 | 10.1 |
| Comparative Administration Example 4 | Comparative Example 1-2 | 2.0 | 81 | 3.4 |
| Comparative Administration Example 5 | Comparative Example 1-3 | 1.0 | 45 | 0.2 |

Experimental Example 3

Hereinafter, the high-concentrated soluble thrombomodulin-containing preparation obtained in Example and the composition obtained in Comparative Example were stored in a thermostatic chamber at 60 °C and then the preparation was redissolved on the 2nd week. The aggregate-generating ratio (%) of the administration preparation was measured.

Method of measuring aggregate-generating ratio

The aggregate-generating ratio of soluble thrombomodulin was measured by an HPLC-analysis gel-filtration method. That is, a mobile phase used was 50-mmol/L phosphate buffer (pH 7.3) containing 0.1 mmol/L sodium sulfate. A column used was TSK-gel 3000SWXL (Tosoh Corp.) and the column temperature was kept at constant temperature of approximately 25°C. The flow rate was adjusted so that the soluble thrombomodulin had a retention time of 9 minutes. The sample solution was prepared by dilution with the addition of the mobile phase of 50-mmol/L phosphate buffer containing 0.1 mmol/L sodium sulfate so that the content of soluble thrombomodulin became 1 mg/mL. 0.15 mL of the solution was injected. The peak area (A) of soluble thrombomodulin and the peak area (B) of aggregate generated after approximately 7 to 8 minutes were obtained using a spectrophotometer for ultraviolet and visible region (measurement wavelength: 280 nm) to calculate the aggregate-generating ratio (%) from $100 \times B / (A + B)$. The results were shown in Table 3.

As is evident from Table 3, if no additive was added (Comparative Example 1-3), the aggregate-generating ratio was high. Comparing with the case of independently adding as in Comparative Examples 1-4 to 1-11, a marked synergistic effect that extremely high stability was attained by combining two types of the additives as in the Examples was confirmed. In addition, the results of restraining the aggregate-generating ratio were obtained as in the case of Table 3 with respect to the high-concentrated soluble thrombomodulin-containing preparation obtained in Examples 1-19 to 1-38 as well as the high-concentrated soluble thrombomodulin-containing preparation containing TME456H or TME456HM obtained in Examples 1-1 to 1-18.

Therefore, those results confirmed that the examples of the present invention had small aggregate-generating ratios and there were a few pains when they were dissolved for administration.

Table 3

| | Additive | | Addition amount (mmol) | Aggregate-generating ratio (%) Type of soluble thrombomodulin used; Addition amount 30 mq | |
|---|---|---|---|---|---|
| | | | | TMD123H | TMD123HM |
| Example 1-1 | Sodium L-glutamate monohydrate | | 0.05 | 0.32 | 0.4 |
| | D(-)-Mannitol | | 0.05 | | |
| Example 1-2 | Sodium L-glutamate monohydrate | | 0.075 | 0.34 | 0.36 |
| | D(-)-Mannitol | | 0.075 | | |
| Example 1-3 | Sodium L-glutamate monohydrate | | 0.025 | 0.52 | 0.53 |
| | D(-)-Mannitol | | 0.025 | | |
| Example 1-4 | Sodium L-glutamate monohydrate | | 0.02 | 0.36 | 0.41 |
| | D(-)-Mannitol | | 0.10 | | |
| Example 1-5 | Sodium L-glutamate monohydrate | | 0.07 | 0.37 | 0.33 |
| | D(-)-Mannitol | | 0.02 | | |
| Example 1-6 | Sodium L-glutamate monohydrate | | 0.03 | 0.39 | 0.37 |
| | D(-)-Mannitol | | 0.15 | | |
| Example 1-7 | Sodium L-glutamate monohydrate | | 0.10 | 0.43 | 0.39 |
| | D(-)-Mannitol | | 0.03 | | |
| Example 1-8 | Sodium L-glutamate monohydrate | | 0.05 | 0.40 | 0.43 |
| | L-lysine hydrochloride | | 0.05 | | |
| Example 1-9 | Sodium L-glutamate monohydrate | | 0.03 | 0.32 | 0.35 |
| | L-lysine hydrochloride | | 0.07 | | |
| Example 1-10 | L-lysine-L -glutamate dihydrate | Lysine | 0.10 | 0.39 | 0.36 |
| | | Glutamic acid | 0.10 | | |
| Example 1-11 | L-lysine-L -glutamate dihydrate | Lysine | 0.15 | 0.42 | 0.45 |
| | | Glutamic acid | 0.15 | | |
| Example 1-12 | Sodium L-glutamate monohydrate | | 0.06 | 0.55 | 0.5 |
| | Sodium L-aspartate monohydrate | | 0.06 | | |

(continued)

| | Additive | Addition amount (mmol) | Aggregate-generating ratio (%) | |
|---|---|---|---|---|
| | | | Type of soluble thrombomodulin used; Addition amount 30 mq | |
| | | | TMD123H | TMD123HM |
| Example 1-13 | Sodium L-aspartate monohydrate | 0.05 | 0.41 | 0.44 |
| | D(-)-Mannitol | 0.05 | | |
| Example 1-14 | Urea | 0.07 | 0.59 | 0.56 |
| Example 1-15 | Urea | 0.05 | 0.42 | 0.45 |
| . | Sodium L-glutamate monohydrate | 0.05 | | |
| Example 1-16 | Urea | 0.05 | 0.53 | 0.55 |
| | Sodium L-aspartate monohydrate | 0.05 | | |
| Example 1-17 | Sodium L-glutamate monohydrate | 0.05 | 0.34 | 0.38 |
| | D(-)-Mannitol | 0.05 | | |
| Example 1-18 | Sodium L-aspartate monohydrate | 0.05 | 0.39 | 0.42 |
| | D(-)-Mannitol | 0.05 | | |
| Comparative Example 1-3 | No additive | 0 | 3.21 | 3.51 |
| Comparative Example 1-4 | Sodium L-glutamate monohydrate | 0.025 | 1.56 | 1.59 |
| Comparative Example 1-5 | D(-)-Mannitol | 0.025 | 1.89 | 1.97 |
| Comparative Example 1-6 | Sodium L-glutamate monohydrate | 0.05 | 0.80 | 0.87 |
| Comparative Example 1-7 | D(-)-Mannitol | 0.05 | 0.91 | 1.16 |
| Comparative Example 1-8 | Sodium L-glutamate monohydrate | .0.075 | 0.73 | 0.77 |
| Comparative Example 1-9 | D(-)-Mannitol | 0.075 | 0.83 | 0.92 |
| Comparative Example 1-11 | Sodium L-aspartate monohydrate | 0.05 | 0.98 | 1.05 |

Experimental Example 4 - 1

For high-concentrated soluble thrombomodulin (TMD123H)-containing preparations or kit preparations obtained in Examples 1-39-1 to 1-53-1, high-concentrated soluble thrombomodulin-containing solutions were prepared and then the osmotic pressure ratios and aggregate-generating ratios thereof were determined by the same methods as those of Example 2 or Example 3. The results were shown in Table 4.

Method of preparing high-concentrated soluble thrombomodulin-containing solution

High-concentrated soluble thrombomodulin-containing preparations which the soluble thrombomodulins obtained in Examples 1-39-1 to 1-41-1 were TMD123H were redissolved with the addition of 1 mL of water for injection to obtain high-concentrated soluble thrombomodulin-containing solutions. In addition, the kit preparations of the high-concentrated soluble thrombomodulin-containing preparations which the soluble thrombomodulins obtained in Examples 1-42-1 to 1-53-1 were TMD123H were redissolved by aspirating 1 mL of dissolving aqueous solution and inj ecting into vials using 1 mL disposable syringes (manufactured by Terumo Corp.) to obtain high-concentrated soluble thrombomodulin-containing solutions.

As is evident from the results shown in Table 4, the osmotic pressure ratio of the high-concentrated soluble thrombomodulin-containing solutions obtained in Examples became almost 1 so that the results of restraining the aggregate-generating ratio were obtained.

Furthermore, with respect to the high-concentrated soluble thrombomodulin-containing preparations or kit preparations thereof obtained in Examples 1-39-2 to 1-39-4, each containing TTMD123HM, TME456H, or TME456HM as soluble thrombomodulin, the osmotic pressure ratio of each of them became almost 1 just as in the case of Table 4, and the results of restraining the aggregate-generating ratio were obtained.

Therefore, those results confirmed that the examples of the present invention had low aggregate-generating ratios and low pains when dissolved for administration, and they could be adopted into administration methods such as intramuscular administration and subcutaneous administration.

Table 4

| | Osmotic pressure ratio | Aggregate-generating ratio (%) |
|---|---|---|
| Example 1-39-1 | 1.0 | 0.41 |
| Example 1-40-1 | 1.0 | 0.33 |
| Example 1-41-1 | 1.0 | 0.48 |
| Example 1-42-1 | 1.0 | 0.44 |
| Example 1-43-1 | 1.2 | 0.46 |
| Example 1-44-1 | 1.2 | 0.39 |
| Example 1-45-1 | 1.0 | 0.35 |
| Example 1-46-1 | 1.2 | 0.35 |
| Example 1-47-1 | 1.2 | 0.36 |
| Example 1-48-1 | 1.0 | 0.41 |
| Example 1-49-1 | 1.1 | 0.38 |
| Example 1-50-1 | 1.1 | 0.39 |
| Example 1-51-1 | 1.0 | 0.35 |
| Example 1-52-1 | 1.1 | 0.34 |
| Example 1-53-1 | 1.1 | 0.35 |

Example 2-1

Preparation of additive solution

Polysorbate 80 (5 mg) was weighed and placed in a 20 mLmeasuring flask (corresponding to 50-fold of the addition amount listed in the column of composition in Table 5) and dissolved by the addition of water for injection to adjust the solution to 20 mL in all.

Preparation of sample solution

4 mL of the above additive solution was put in a 15 mL assist tube and also 5 mL of TMD123H obtained in Reference Example 1 (soluble thrombomodulin concentration: 60 mg/mL) was put in the same assist tube (corresponding to 10-fold of the description in the column of addition amount in Table 5 as soluble thrombomodulin). Then, water for injection was added so that the amount of the mixture was adjusted to 10 mL, and then the mixture was stirred. The sample solution was sterilized by filtration through a filter of 0.22 μm in pore size (MILLEX-GV, manufactured by Millipore) using a 25 mL disposal syringe (manufactured by Terumo Corp.), otherwise specifically limited, followed by dispensing 1 mL thereof into sterile vials (3 mL in volume, 18 x 33 mm, 9.1 mm in opening internal diameter, manufactured by Namicos) which were not coated with silicone.

Freeze-drying step

A high-concentrated soluble thrombomodulin-containing preparation containing 30 mg of soluble thrombomodulin and Polysorbate 80 (0.1 mg) in a container was obtained by performing a freeze-drying step under the conditions described below in the order of: plugging with a rubber stopper (V5-F8, manufactured by Daikyo Seiko) by half → freeze-drying → nitrogen-filling → plugging with the rubber stopper → winding a cap up (Example 2-1-1).

[Freeze-drying conditions]

Preliminary cooling (from room temperature to 15°C while spending 15 minutes) → maincooling (from 15°C to - 45°C while spending 2 hours) → retaining (-45°C for 2 hours) → vacuum-starting (-45°C for 18 hours) → warming-up (from -45°C to 25°C while spending 20 hours) → retaining (25°C for 15 hours) → warming-up (from 25°C to 45°C while spending 1 hour) → retaining (45°C for 5 hours) → lowering to room temperature (from 45°C to 25°C while spending 2 hours) → nitrogen-filling for recovering pressure (recovering pressure up to -13332,2 Pa (-100 mmHg) with nitrogen) → plugging → winding a cap up.

[Confirmation of the pressure reduction degree in dissolution]

In advance, an empty vial was subj ected to the steps of plugging with a rubber stopper and winding a cap up at -13332,2 Pa (-100 mmHg), and 1 mL of water for injection was injected into the vial, followed by confirming the pressure reduction degree with a U-shaped vacuum meter (U-400, manufactured by Okano Works). The result confirmed that the pressure was almost atmospheric pressure.

Example 2-1-2, Example 2-1-3, and Example 2-1-4

Similarly, the types of soluble thrombomodulin were changed such that TMD123HM (Example 2-1-2), TME456H (Example 2-1-3), and TME456HM (Example 2-1-4) were used instead of TMD123H of Example 2-1-1 and then high-concentrated soluble thrombomodulin-containing preparations were obtained by the above method respectively.

Example 2-2

A high-concentrated soluble thrombomodulin-containing preparation listed in the column of composition in Table 5 was obtained in the same manner as in Example 2-1 (Example 2-2-1 was prepared according to Example 2-1-1).

Example 2-3

A high-concentrated soluble thrombomodulin-containing preparation listed in the column of composition in Table 5 was obtained in the same manner as in Example 2-1 (like the above description, Example 2-3-1 was prepared)

Example 2-4

A high-concentrated soluble thrombomodulin-containing preparation listed in the column of composition in Table 5 was obtained in the same manner as in Example 2-1 (like the above description, Example 2-4-1 was prepared).

Example 2-5

A high-concentrated soluble thrombomodulin-containing preparation listed in the column of composition in Table 5 was obtained in the same manner as in Example 2-1 (like the above description, preparation in Example 2-5-1 was prepared).

Example 2-6

A high-concentrated soluble thrombomodulin-containing preparation listed in the columnof composition in Table 5 was obtained in the same manner as in Example 2-1 (like the above description, Example 2-6-1 was prepared).

Example 2-7

A high-concentrated soluble thrombomodulin-containing preparation listed in the column of composition in Table 5 was obtained in the same manner as in Example 2-1 (like the above description, Example 2-7-1 was prepared).

Example 2-8

A high-concentrated soluble thrombomodulin-containing preparation listed in the column of composition in Table 5 was obtained in the same manner as in Example 2-1 (like the above description, Example 2-8-1 was prepared).

Example 2-9

A high-concentrated soluble thrombomodulin-containing preparation listed in the column of composition in Table 5 was obtained in the same manner as in Example 2-1 (like the above description, Example 2-9-1 was prepared).

Example 2-10

A high-concentrated soluble thrombomodulin-containing preparation listed in the column of composition in Table 5 was obtained in the same manner as in Example 2-1 (like the above description, Example 2-10-1 was prepared).

Example 2-11

A high-concentrated soluble thrombomodulin-containing preparation listed in the column of composition in Table 5 was obtained in the same manner as in Example 2-1 (like the above description, preparation in Example 2-11-1 was prepared).

Example 2-12

A high-concentrated soluble thrombomodulin-containing preparation listed in the column of composition in Table 5 was obtained in the same manner as in Example 2-1 (like the above description, Example 2-12-1 was prepared).

Example 2-13

A high-concentrated soluble thrombomodulin-containing preparation listed in the column of composition in Table 5 was obtained in the same manner as in Example 2-1 (like the above description, Example 2-13-1 was prepared).

Example 2-14

A high-concentrated soluble thrombomodulin-containing preparation listed in the column of composition in Table 5 was obtained in the same manner as in Example 2-1 (like the above description, Example 2-14-1 was prepared).

Example 2-15

A high-concentrated soluble thrombomodulin-containing preparation listed in the column of composition in Table 5 was obtained in the same manner as in Example 2-1 (like the above description, Example 2-15-1 was prepared).

Example 2-16

A high-concentrated soluble thrombomodulin-containing preparation listed in the column of composition in Table 5 was obtained in the same manner as in Example 2-1 (like the above description, preparation in Example 2-16-1 was prepared).

Example 2-17

A high-concentrated soluble thrombomodulin-containing preparation listed in the column of composition in Table 5 was obtained in the same manner as in Example 2-1 (like the above description, Example 2-17-1 was prepared).

Example 2-18

A high-concentrated soluble thrombomodulin-containing preparation listed in the column of composition in Table 5 was obtained in the same manner as in Example 2-1 (like the above description, Example 2-18-1 was prepared).

Example 2-19

A high-concentrated soluble thrombomodulin-containing preparation listed in the column of composition in Table 5 was obtained in the same manner as in Example 2-1 (like the above description, Example 2-19-1 was prepared).

Example 2-20

A high-concentrated soluble thrombomodulin-containing preparation listed in the column of composition in Table 5 was obtained in the same manner as in Example 2-1 (like the above description, Example 2-20-1 was prepared).

Example 2-21

A high-concentrated soluble thrombomodulin-containing preparation listed in the column of composition in Table 5 was obtained in the same manner as in Example 2-1 (like the above description, Example 2-21-1 was prepared).

Example 2-22

A high-concentrated soluble thrombomodulin-containing preparation listed in the column of composition in Table 5 was obtained in the same manner as in Example 2-1 (like the above description, Example 2-22-1 was prepared).

Example 2-23

A high-concentrated soluble thrombomodulin-containing preparation listed in the column of composition in Table 5 was obtained in the same manner as in Example 2-18, except that the vial used was a 0.1%(w/v) silicone-coating vial (3 mL, 18 x 33 mm, 9.1 mm in opening internal diameter, manufactured by Namicos) (just like the above description, Example 2-23-1 was prepared).

Example 2-24

A high-concentrated soluble thrombomodulin-containing preparation listed in the column of composition in Table 5 was obtained in the same manner as in Example 2-18, except that the vial used was a 0.5%(w/v) silicone-coating vial (3 mL, 18 x 33 mm, 9.1 mm in opening internal diameter, manufactured by Namicos) (just like the above description, Example 2-24-1 was prepared).

Example 2-25

A high-concentrated soluble thrombomodulin-containing preparation listed in the column of composition in Table 5 was obtained in the same manner as in Example 2-18, except that the vial used was a 1.0%(w/v) silicone-coating vial (3 mL, 18 x 33 m, 9.1 mm in opening internal diameter, manufactured by Namicos) (just like the above description, Example 2-25-1 was prepared).

Example 2-26

A high-concentrated soluble thrombomodulin-containing preparation listed in the column of composition in Table 5 was obtained in the same manner as in Example 2-18, except that the vial used was a 2.0%(w/v) silicone-coating vial (3 mL, 18 x 33 m, 9.1 mm in opening internal diameter, manufactured by Namicos) (just like the above description, Example 2-26-1 was prepared).

Example 2-27

A high-concentrated soluble thrombomodulin-containing preparation listed in the column of composition in Table 5 was obtained in the same manner similar as in Example 2-18 (just like the above description, Example 2-27-1 was prepared). In addition, by the following method, the pressure reduction degree was decreased to an initial pressure reduction degree so as to obtain a pressure reduction degree of -26664,4 Pa (-200 mmHg) at the time of redissolving the preparation.

The pressure reduction degree was confirmed using a U-shaped vacuum meter (U-400, manufactured Okano Works) when sucking with a vacuum pump (LV-140, manufactured by Nitto Kohki Co., Ltd.). Previously, a rubber stopper and a cap were provided on an empty vial, and then the needle of a syringe connected to the pump was inserted into the vial. Then, the pressure in the container was reduced by switching the vacuum pump on (initial pressure reduction degree) to calculate the initial pressure reduction degree when the pressure reduction degree would become -26664,4 Pa (-200 mmHg) immediately after the injection of 1 mL of water for injection in the vial. Each preparation was depressurized until it reached the initial pressure reduction degree.

Example 2-28

A high-concentrated soluble thrombomodulin-containing preparation listed in the column of composition in Table 5 was obtained in the same manner as in Example 2-18, except that the vial used was a 2.0%(w/v) silicone-coating vial (3 mL, 18 x 33 mm, 9.1 mm in opening internal diameter, manufactured by Namicos) (just like the above description, Example 2-28-1 was prepared). Further, in the same manner as in Example 2-27, an initial pressure reduction degree was adjusted so as to obtain a pressure reduction degree of -26664,4 Pa (-200 mmHg) at the time of redissolving the preparation.

Example 2-29

A high-concentrated soluble thrombomodulin-containing preparation listed in the column of composition in Table 5 was obtained in the same manner as in Example 2-11, except that the vial used was a 2.0%(w/v) silicone-coating vial (3 mL, 18 x 33 mm, 9.1 mm in opening internal diameter, manufactured by Namicos) (just like the above description, Example 2-29-1 was prepared). Further, in the same manner as in Example 2-27, an initial pressure reduction degree was adjusted so as to obtain a pressure reduction degree of -26664,4 Pa (-200 mmHg) at the time of redissolving the preparation.

Example 2-30

A high-concentrated soluble thrombomodulin-containing preparation listed in the column of composition in Table 5 was obtained in the same manner as in Example 2-15, except that the vial used was a 2.0%(w/v) silicone-coating vial (3 mL, 18 x 33 mm, 9.1 mm in opening internal diameter, manufactured by Namicos) (just like the above description, Example 2-30-1 was prepared). Further, in the same manner as in Example 2-27, an initial pressure reduction degree was adjusted so as to obtain a pressure reduction degree of -26664,4 Pa (-200 mmHg) at the time of redissolving the preparation.

Example 2-31

A kit preparation of a soluble thrombomodulin-containing freeze-dried preparation obtained by making a combination of: the soluble thrombomodulin-containing freeze-dried preparation listed in the column of composition in Table 5 obtained in the same manner as in Example 2-14, except that Polysorbate 80 was not added in the additive solution; and an ampule aseptically filled with 1 mL of solution containing 10 mg of benzyl alcohol in 1 mL of water for injection as a dissolving aqueous solution (just like the above description, Example 2-31-1 was prepared).

Example 2-32

A kit preparation of a soluble thrombomodulin-containing freeze-dried preparation obtained by making a combination of: the soluble thrombomodulin-containing freeze-dried preparation listed in the column of composition in Table 5 obtained in the same manner as in Example 2-14, except that Polysorbate 80 was not added in the additive solution; and an ampule aseptically filled with 1 mL of solution containing 5 mg of benzyl alcohol in 1 mL of water for injection as a dissolving aqueous solution (just like the above description, Example 2-32-1 was prepared).

Example 2-33

A kit preparation of a soluble thrombomodulin-containing freeze-dried preparation obtained by making a combination of: the soluble thrombomodulin-containing freeze-dried preparation listed in the column of composition in Table 5 obtained in the same manner as in Example 2-14, except that Polysorbate 80 was not added in the additive solution; and an ampule aseptically filled with 1 mL of solution containing 5 mg of chlorobutanol in 1 mL of water for injection as a dissolving aqueous solution (just like the above description, Example 2-33-1 was prepared).

Example 2-34

A kit preparation of a soluble thrombomodulin-containing freeze-dried preparation obtained by making a combination of: the soluble thrombomodulin-containing freeze-dried preparation listed in the column of composition in Table 5 obtained in the same manner as in Example 2-14, except that Polysorbate 80 was not added in the additive solution; and an ampule aseptically filled with 1 mL of solution containing 2.5 mg of chlorobutanol in 1 mL of water for injection as a dissolving aqueous solution (just like the above description, Example 2-34-1 was prepared).

Example 2-35

Each 1 mL aliquot of sample solution was dispensed into one chamber of a two-chamber syringe (manufactured by ARTE) instead of the sterile vial in the preparation of the sample solution in Example 2-1. They were subjected to the freeze-drying step (under the same freeze-drying conditions as those of Example 2-1) in the order of: plugging with a middle stopper by half → freeze-drying → nitrogen-filling → plugging with the middle stopper. Subsequently, 1.0 mL of water for injection was aseptically filled in the other chamber and sealed with a rubber stopper, followed by providing with a plunger rod, resulting in a kit preparation composed of the high-concentrated soluble thrombomodulin(TMD123H)-containing preparation (Example 2-35-1).

Comparative Example 2-1

Preparation of sample solution

1.67 mL of TMD123H (soluble thrombomodulin concentration: 60 mg/mL) obtained in Reference Example 1 (containing 100 mg as soluble thrombomodulin, i.e. , 10-fold of the amount described in the column of addition amount in Table 5) was placed in a 15 mL assist tube. Then, water for injection was added so that the amount of the mixture was 10 mL, and then the mixture was stirred. The sample solution was sterilized by filtration through a filter of 0.22 μm in pore size (MILLEX-GV, manufactured by Millipore) using a 25 mL disposal syringe (manufactured by Terumo Corp.), followed by dispensing each 1 mL thereof into sterile vials (3 mL in volume, 18 x 33 mm, 9.1 mm in opening internal diameter, manufactured by Namicos) which were not coated with silicone.

Freeze-drying step

A Composition listed in the column of composition in Table 5 was obtained by performing a freeze-drying step under the conditions described in Example 2-1 in the order of: plugging with a rubber stopper (V5-F8,manufactured by Daikyo Seiko) by half → freeze-drying → nitrogen-filling → plugging with the rubber stopper → winding a cap up.

Comparative Example 2-2

A composition listed in the column of composition in Table 5 was obtained in the same manner as in Comparative Example 2-1.

Comparative Example 2-3

A composition listed in the column of composition in Table 5 was obtained in the same manner as in Comparative Example 2-1 (Comparative Example 2-3-1).

Similarly, the types of soluble thrombomodulin were changed such that TMD123HM (Comparative Example 2-3-2), TME456H (Comparative Example 2-3-3), and TME456HM (Comparative Example 2-3-4) were used instead of TMD123H of Comparative Example 2-3-1 and compositions were obtained by the above method respectively.

Example 2-4

Preparation of additive solution

500 mg of sodium L-glutamate monohydrate and 500 mg of D(-)-mannitol were weighed and placed in a 20 mL measuring flask (corresponding to 50-fold of the addition amount listed in the column of composition in Table 5) and dissolved by the addition of water for injection to adjust the solution to 20 mL in all.

Preparation of sample solution

4 mL aliquot of the above additive solution was placed in a 15 mL assist tube and also 5 mL of TMD123H (soluble thrombomodulin concentration: 60 mg/mL) obtained in Reference Example 1 (containing 300 mg as soluble thrombomodulin, i.e., corresponding to 10-fold of the amount described in the column of addition amount in Table 5) was placed in the same assist tube. Then, water for injection was added so that the amount of the mixture was 10 mL, and then the mixture was stirred. The sample solution was sterilized by filtration through a filter of 0.22 μm in pore size (MILLEX-GV, manufactured by Millipore) using a 25 mL disposal syringe (manufactured by Terumo Corp.), followed by dispensing 1 mL thereof into sterile vials (3 mL in volume, 18 x 33 mm, 9.1 mm in opening internal diameter, manufactured by Namicos) which were not coated with silicone.

Freeze-drying step

A Composition listed in the column of composition in Table 5 was obtained by performing a freeze-drying step under the conditions described in Example 2-1 in the order of: plugging with a rubber stopper (V5-F8 , manufacturedby Daikyo Seiko) by half → freeze-drying → nitrogen-filling → plugging with the rubber stopper → winding a cap up.

Comparative Example 2-5

A composition listed in the column of composition in Table 5 was obtained in the same manner as in Comparative Example 2-4.

Comparative Example 2-6

A composition listed in the column of composition in Table 5 was obtained in the same manner as in Comparative Example 2-4. Experimental Example 4 - 2

The soluble thrombomodulin-containing freeze-dried preparations or kit preparations, which were obtained in Example 2-1 to Example 2-34 shown in Table 5, and the compositions obtained in Comparative Example 2-1 to 2-6 were redissolved in 1 mL of dissolving aqueous solution listed in the column of dissolving aqueous solution in Table 5. Then, the foam-inhibiting effects thereof at that time were determined by their transmittance using the following turbidity measurement and the typical results thereof were shown in Table 5.

Method of measuring turbidity

An injection syringe (for tuberculin, 1 mL, an injection needle: 26G 0.45 x 13 mm, manufactured by Terumo Corp.) suctioning a 1 mL dissolving aqueous solution is inserted into the center of a rubber stopper of a freeze-dried preparation to be provided as a test sample. Then the dissolving aqueous solution is injected into the center of a freeze-dried product at a rate of 0.1 mL/second, followed by leaving it standing while keeping the injection syringe being inserted. After 30 seconds from the completion of injection, the vial is flipped upside down and then approximately 0.8 mL of the high-concentrated soluble thrombomodulin-containing solution is suctioned with the syringe. The suctioned dissolving solution is gently poured into a quartz cell (a black cell, optical path length: 10 mm, optical path width: 2 mm, manufactured by GL Sciences) along the cell wall thereof. Transmittance at 650 nm (transmittance at 650 nm using only the dissolving aqueous solution is adjusted to 100% in advance) is measured quickly using an UV-visible spectrophotometer (UV-2500PC, manufactured by Shimadzu Corporation). The time from the suction of the dissolving aqueous solution for the freeze-dried product in the vial to the measurement of transmittance is set to 15 seconds. By the way, otherwise specifically described, the transmittance is represented as the average of three measurement values.

Table 5

| Example & Comparative Example | Composition | Addition amount (mg) | Dissolving aqueous solution (*1) | Silicone coating (*2) | Pressure reduction degree (*3) | Transmittance (%) |
|---|---|---|---|---|---|---|
| Example 2-1-1 | TMD123H<br>Polysorbate 80 | 30<br>0.1 | W | - | - | 99.5 |
| Example 2-1-2 | TMD123HM<br>Polvsorbate 80 | 30<br>0.1 | W | - | - | 99.3 |
| Example 2-1-3 | TME456H<br>Polysorbate 80 | 30<br>0.1 | W | - | - | 99.5 |
| Example 2-1-4 | TME456HM<br>Polysorbate 80 | 30<br>0.1 | W | - | - | 99.4 |
| Example 2-2-1 | TMD123H<br>Polysorbate 80 | 10<br>0.01 | W | - | - | 99.5 |
| Example 2-3-1 | TMD123H<br>Polysorbate 80 | 15<br>0.015 | W | - | - | 99.4 |
| Example 2-4-1 | TMD123H<br>Polysorbate 80 | 17<br>0.017 | W | - | - | 99.3 |
| Example 2-5-1 | TMD123H<br>Polvsorbate 80 | 20<br>0.02 | W | - | - | 99.4 |
| Example 2-6-1 | TMD123H<br>Polysorbate 80 | 25<br>0.025 | W | - | - | 99.3 |
| Example 2-7-1 | TMD123H<br>Polysorbate 80 | 30<br>0.03 | W | - | - | 99.4 |
| Example 2-8-1 | TMD123H<br>Polysorbate 80 | 30<br>0.05 | W | - | - | 99.5 |
| Example 2-9-1 | TMD123H<br>Polysorbate 80 | 30<br>0.2 | W | - | - | 99.8 |
| Example 2-10-1 | TMD123H<br>Polysorbate 80 | 30<br>0.5 | W | - | - | 100.0 |
| Example 2-11-1 | TMD123H<br>L-arginine hydrochloride<br>Polysorbate 80 | 30<br>10<br>0.1 | W | - | - | 99.2 |
| Example 2-12-1 | TMD123H<br>D-trehalose dihydrate<br>Polysorbate 80 | 30<br>10<br>0.1 | W | - | - | 98.8 |
| Example 2-13-1 | TMD123H<br>L-arginine hydrochloride<br>D-trehalose dihydrate<br>Polysorbate 80 | 30<br>10<br>10<br>0.1 | W | - | - | 98.7 |

(continued)

| Example & Comparative Example | Composition | Addition amount (mg) | Dissolving aqueous solution (*1) | Silicone coating (*2) | Pressure reduction degree (*3) | Transmittance (%) |
|---|---|---|---|---|---|---|
| Example 2-14-1 | TMD123H<br>Sodium L-glutamate monohydrate<br>D-trehalose dihydrate<br>Polysorbate 80 | 30<br>10<br>10<br>0.1 | W | - | - | 98.8 |
| Example 2-15-1 | TMD123H<br>Sodium L-glutamate monohydrate<br>D-(-)-mannitol<br>Polysorbate 80 | 30<br>10<br>10<br>0.1 | W | - | - | 99.2 |
| Example 2-16-1 | TMD123H<br>sodium L-aspartate monohydrate<br>D-(-)-mannitol<br>Polysorbate 80 | 30<br>10<br>10<br>0.1 | W | - | - | 99.3 |
| Example 2-17-1 | TMD123H<br>L-glutamic acid<br>D-(-)-mannitol<br>sodium hydroxide<br>Polysorbate 80 | 30<br>10<br>10<br>2.8<br>0.1 | W | - | - | 99.1 |
| Example 2-18-1 | TMD123H<br>Sodium L-glutamate monohydrate<br>D-(-)-mannitol<br>Polysorbate 20 | 30<br>10<br>10<br>0.1 | W | - | - | 97.7 |
| Example 2-19-1 | TMD123H<br>Sodium L-glutamate monohydrate<br>D-(-)-mannitol<br>Poloxamer 188 | 30<br>10<br>10<br>0.1 | W | - | - | 98.7 |
| Example 2-20-1 | TMD123H<br>Sodium L-glutamate monohydrate<br>D-(-)-mannitol<br>Polyoxyethylene (25) lauryl ether | 30<br>10<br>10<br>0.1 | W | - | - | 97.2 |

(continued)

| Example & Comparative Example | Composition | Addition amount (mg) | Dissolving aqueous solution (*1) | Silicone coating (*2) | Pressure reduction degree (*3) | Transmittance (%) |
|---|---|---|---|---|---|---|
| Example 2-21-1 | TMD123H<br>Sodium L-glutamate monohydrate<br>D-(-)-mannitol<br>Polyoxyl 40 stearate | 30<br>10<br>10<br>0.1 | W | - | - | 98.1 |
| Example 2-22-1 | TMD123H<br>Sodium L-glutamate monohydrate<br>D-(-)-mannitol<br>HCO-60 | 30<br>10<br>10<br>0.1 | W | - | - | 97.8 |
| Example 2-23-1 | TMD123H<br>Sodium L-glutamate monohydrate<br>D-(-)-mannitol<br>Polysorbate 20 | 30<br>10<br>10<br>0.1 | W | 0.1 % | - | 98.0 |
| Example 2-24-1 | TMD123H<br>Sodium L-glutamate monohydrate<br>D-(-)-mannitol<br>Polysorbate 20 | 30<br>10<br>10<br>0.1 | W | 0.5 % | - | 98.3 |
| Example 2-25-1 | TMD123H<br>Sodium L-glutamate monohydrate<br>D-(-)-mannitol<br>Polysorbate 20 | 30<br>10<br>10<br>0.1 | W | 1.0 % | - | 98.6 |
| Example 2-26-1 | TMD123H<br>Sodium L-glutamate monohydrate<br>D-(-)-mannitol<br>Polysorbate 20 | 30<br>10<br>10<br>0.1 | W | 2.0 % | - | 99.1 |
| Example 2-27-1 | TMD123H<br>Sodium L-glutamate monohydrate<br>D-(-)-mannitol<br>Polysorbate 20 | 30<br>10<br>10<br>0.1 | W | - | + | 98.2 |

(continued)

| Example & Comparative Example | Composition | Addition amount (mg) | Dissolving aqueous solution (*1) | Silicone coating (*2) | Pressure reduction degree (*3) | Transmittance (%) |
|---|---|---|---|---|---|---|
| Example 2-28-1 | TMD123H<br>Sodium L-glutamate monohydrate<br>D-(-)-mannitol<br>Polysorbate 20 | 30<br>10<br>10<br>0.1 | W | 2.0 % | + | 99.3 |
| Example 2-29-1 | TMD123H<br>L-arginine hydrochloride<br>Polysorbate 80 | 30<br>10<br>0.1 | W | 2.0 % | + | 99.7 |
| Example 2-30-1 | TMD123H<br>Sodium L-glutamate monohydrate<br>D-(-)-mannitol<br>Polysorbate 80 | 30<br>10<br>10<br>0.1 | W | 2.0 % | + | 99.5 |
| Example 2-31-1 | TMD123H<br>Sodium L-glutamate monohydrate<br>D-trehalose dihydrate | 30<br>10<br>10 | Be10 | - | - | 98.2 |
| Example 2-32-1 | TMD123H<br>Sodium L-glutamate monohydrate<br>D-trehalose dihydrate | 30<br>10<br>10 | Be05 | - | - | 97.9 |
| Example 2-33-1 | TMD123H<br>Sodium L-glutamate monohydrate<br>D-trehalose dihydrate | 30<br>10<br>10 | Ch50 | - | - | 98.0 |
| Example 2-34-1 | TMD123H<br>Sodium L-glutamate monohydrate<br>D-trehalose dihydrate | 30<br>10<br>10 | Ch25 | - | - | 97.7 |
| Comparative Example 2-1 | TMD123H | 10 | W | - | - | 90.5 |
| Comparative Example 2-2 | TMD123H | 15 | W | - | - | 84.9 |

(continued)

| Example & Comparative Example | Composition | Addition amount (mg) | Dissolving aqueous solution (*1) | Silicone coating (*2) | Pressure reduction degree (*3) | Transmittance (%) |
|---|---|---|---|---|---|---|
| Comparative Example 2-3-1 | TMD123H | 30 | W | - | - | 79.7 |
| Comparative Example 2-4 | TMD123H<br>Sodium L-glutamate monohydrate<br>D-(-)-mannitol | 30<br>10<br>10 | W | - | - | 62.2 |
| Comparative Example 2-5 | TMD123H<br>Sodium L-glutamate monohydrate<br>D-(-)-mannitol<br>Sodium lauryl sulfate | 30<br>10<br>10<br>0.1 | W | - | - | 57.7 |
| Comparative Example 2-6 | TMD123H<br>Sodium L-glutamate monohydrate<br>D-(-)-mannitol<br>Benzalkonium chloride . | 30<br>10<br>10<br>0.1 | W | - | - | 82.7 |

Description of symbols in the table
*1: Dissolving aqueous solution
W ; Distilled water for injection 1 mL
Be10; Distilled water for injection 1 mL + benzyl alcohol 10 mg
Be05; Distilled water for injection 1 mL + benzyl alcohol 5 mg
Ch50; Distilled water for injection 1 mL + chlorobutanol 5 mg
Ch25; Distilled water for injection 1 mL + chlorobutanol 2.5 mg
2*: Silicone coating
- ; Uncoated vial
0.1%; 0.1%(w/v) silicone-coating vial
0.5%; 0.5%(w/v) silicone-coating vial
1.0%; 1.0%(w/v) silicone-coating vial
2.0%; 2.0%(w/v) silicone-coating vial
3*: Pressure reduction degree
-; Substantially no pressure reduction state in pressure at the time of dissolution.
+; A pressure reduction state of -200 mmHg at the time of dissolution.

The results shown in the column of transmittance (%) in Table 5 confirmed that when the soluble thrombomodulin contains a nonionic surfactant in its high-concentrated preparation having a concentration of 10 mg/mL or more, the transmittance is higher and the generation of air-bubble is inhibited, in comparison with the case where no nonionic surfactant shown in Comparative Example 2-3-1 is contained and the case where the anionic surfactant (Comparative Example 2-5) or cationic surfactant (Comparative Example 2-6) is added.

By the way, the transmittance of freeze-dried composition containing TMD123HM, TME456H, or TME456HM in Comparative Example 2-3-2 to Comparative Example 2-3-4 was almost equal to that of Comparative Example 2-3-1.

In addition, for the two-chamber type syringe kit preparation obtained in Example 2-35-1, a plunger rod was pushed into a position (with a constant rate of about 0.1 mL/second) where plugging with the stopper was done by half and then the plunger rod was further pushed 30 seconds after the injection of water for injection into one chamber with freeze-dried composition. Subsequently, the high-concentrated soluble thrombomodulin-containing solution was gently poured in a quartz cell along the cell wall thereof and then the transmittance thereof was determined according to the method described above, showing a high transmittance just as in the case of Table 5.

The above results confirmed that the generation of air bubbles can be inhibited by the preparation that contains a nonionic surfactant, benzyl alcohol, or chlorobutanol as a foam-inhibition additive in the high-concentrated soluble thrombomodulin-containing solution. Inaddition, the above results also confirmed that the generation of air bubbles can be inhibited by coating the inner wall of a container to be used in dissolving the soluble thrombomodulin-containing freeze-dried preparation with silicone or by keeping the pressure in the container in dissolving the soluble thrombomodulin-containing freeze-dried preparation at a reduced pressure reduced by -13332,2 Pa to -39996,6 Pa (-100 to -300 mmHg).

Experimental Example 5

Kit preparations were prepared by combining the soluble thrombomodulin (TMD123H)-containing freeze-dried preparations obtained in Example 1-1-1 to Example 1-38-1 shown in Table 1 and the dissolving aqueous solutions shownin Table 6. Then their effects of foam inhibition were confirmed by the same method as in Experimental Example 4 - 2 described above. The result confirmed that the generation of air bubbles is inhibited.

Table 6

| Dissolving aqueous solution | Composition | Volume |
|---|---|---|
| Dissolving solution 1 | Water for injection<br>Benzyl alcohol | 1 mL<br>10 mg |
| Dissolving solution 2 | Water for injection<br>Chlorobutanol | 1 mL<br>5 mg |
| Dissolving solution 3 | Water for injection<br>Polysorbate 80 | 1 mL<br>0.1 mg |
| Dissolving solution 4 | Water for injection<br>Poloxamer 188 | 1 mL<br>0.1 mg |
| Dissolving solution 5 | Water for injection<br>Polyoxyethlene (25) lauryl ether | 1 mL<br>0.1 mg |

INDUSTRIAL APPLICABILITY

According to the present invention, the generation of air bubbles in a solution that contains high-concentrated soluble thrombomodulin can be inhibited. The inhibition will be useful in clinical application. In addition, a high-concentrated soluble thrombomodulin-containing preparation and a kit preparation capable of inhibiting the generation of such air bubbles can be provided.

## Claims

1. A method of inhibiting foaming of a high-concentrated soluble thrombomodulin-containing solution, which is used in preparing a high-concentrated soluble thrombomodulin-containing solution having a concentration of 10 mg/mL or more by dissolving a soluble thrombomodulin-containing freeze-dried preparation that contains soluble thrombomodulin as an active ingredient, the method being **characterized by** comprising at least one of:

   (a) the presence of at least one compound selected from the group consisting of a nonionic surfactant, benzyl alcohol, and chlorobutanol;
   (b) the use of a container having the inner wall coated with silicone, said container being used in dissolving the soluble thrombomodulin-containing freeze-dried preparation; and
   (c) a pressure in the container to be used in dissolving the soluble thrombomodulin-containing freeze-dried preparation is kept at a reduced pressure, reduced by -13332.2 Pa to -39996.6 Pa (-100 to -300 mmHg).

2. The method of inhibiting foaming of a high-concentrated soluble thrombomodulin-containing solution according to claim 1, which is used in preparing a high-concentrated soluble thrombomodulin-containing solution having a concentration of 10 mg/mL or more by dissolving the soluble thrombomodulin-containing freeze-dried preparation, the method being **characterized by** comprising allowing the presence of at least one compound selected from the group consisting of a nonionic surfactant, benzyl alcohol, and chlorobutanol.

3. The method of inhibiting foaming of a high-concentrated soluble thrombomodulin-containing solution according to claim 1 or 2, **characterized by** allowing the presence of at least one compound selected from the group consisting of a nonionic surfactant, benzyl alcohol, and chlorobutanolin the soluble thrombomodulin-containing freeze-dried preparation.

4. The method of inhibiting foaming of a high-concentrated soluble thrombomodulin-containing solution according to any one of claims 1 to 3, **characterized by** allowing the presence of at least one compound selected from the group consisting of a nonionic surfactant, benzyl alcohol, and chlorobutanol in a dissolving aqueous solution for dissolving the soluble thrombomodulin-containing freeze-dried preparation.

5. The method of inhibiting foaming of a high-concentrated soluble thrombomodulin-containing solution according to any one of claims 1 to 4, **characterized in that** the inner wall of the container to be used in dissolving the soluble thrombomodulin-containing freeze-dried preparation is coated with silicone.

**6.** The method of inhibiting foaming of a high-concentrated soluble thrombomodulin-containing solution according to any one of claims 1 to 5, **characterized in that** the pressure in the container to be used in dissolving the soluble thrombomodulin-containing freeze-dried preparation is kept at a reduced pressure, reduced by -13332.2 Pa to 39996.6 Pa (-100 to -300 mmHg).

**7.** The method of inhibiting foaming of a high-concentrated soluble thrombomodulin-containing solution according to any one of claims 1 to 6, **characterized in that** the high-concentrated soluble thrombomodulin-containing solution has a soluble thrombomodulin concentration of 17 mg/mL or more.

**8.** The method of inhibiting foaming of a high-concentrated soluble thrombomodulin-containing solution according to any one of claims 1 to 6, **characterized in that** the high-concentrated soluble thrombomodulin-containing solution has a soluble thrombomodulin concentration of 25 mg/mL or more.

**9.** The method of inhibiting foaming of a high-concentrated soluble thrombomodulin-containing solution according to any one of claims 1 to 8, **characterized in that** a fluid volume of the high-concentrated soluble thrombomodulin-containing solution prepared by dissolution is in a range of 0.1 mL to 2 mL and an osmotic pressure ratio upon dissolution thereof is in a range of 0.5 to 2.0.

**10.** The method of inhibiting foaming of a high-concentrated soluble thrombomodulin-containing solution according to any one of claims 1 to 9, **characterized in that** the soluble thrombomodulin-containing freeze-dried preparation is allowed to contain at least one combination selected from the group consisting of

(1) a combination containing two of glutamic acid or a salt thereof and mannitol,
(2) a combination containing two of glutamic acid or a salt thereof and lysine or a salt thereof,
(3) a combination containing two of glutamic acid or a salt thereof and asparaginic acid or a salt thereof, and
(4) a combination containing two of asparaginic acid or a salt thereof and mannitol; and

at least one compound selected from the group consisting of a nonionic surfactant, benzyl alcohol, and chlorobutanol is allowed to be present in the soluble thrombomodulin-containing freeze-dried preparation and/or at least one compound selected from the group consisting of a nonionic surfactant, benzyl alcohol, and chlorobutanol is allowed to be present in the dissolving aqueous solution for dissolving the soluble thrombomodulin-containing freeze-dried preparation.

**11.** The method of inhibiting foaming of a high-concentrated soluble thrombomodulin-containing solution according to any one of claims 1 to 9, **characterized in that**: the soluble thrombomodulin-containing freeze-dried preparation is allowed to contain any one of (1) urea or (2) urea and an amino acid; and at least one compound selected from the group consisting of a nonionic surfactant, benzyl alcohol, and chlorobutanol is allowed to be present in the soluble thrombomodulin-containing freeze-dried preparation and/or at least one compound selected from the group consisting of a nonionic surfactant, benzyl alcohol, and chlorobutanol is allowed to be present in the dissolving aqueous solution for dissolving the soluble thrombomodulin-containing freeze-dried preparation.

**12.** The method of inhibiting foaming of a high-concentrated soluble thrombomodulin-containing solution according to any one of claims 1 to 8, **characterized in that**: the soluble thrombomodulin-containing freeze-dried preparation is allowed to contain one or two compounds selected from the group consisting of arginine, glutamic acid, proline, serine, glycine, histidine, asparagine, lysine, phenylalanine, and valine, or salts thereof, trehalose, lactose, and sucrose; further, at least one compound selected from the group consisting of a nonionic surfactant, benzyl alcohol, and chlorobutanol is allowed to be present in the soluble thrombomodulin-containing freeze-dried preparation and/or at least one compound selected from the group consisting of a nonionic surfactant, benzyl alcohol, and chlorobutanol is allowed to be present in the dissolving aqueous solution for dissolving the soluble thrombomodulin-containing freeze-dried preparation.

**13.** The method of inhibiting foaming of a high-concentrated soluble thrombomodulin-containing solution according to any one of claims 1 to 12, **characterized in that** the nonionic surfactant comprises at least one compound selected from the group consisting of polyoxyethylene sorbitan fatty acid ester, polyoxyethylene/polyoxypropylene copolymer, polyoxyethylene alkylether, polyoxyethylene fatty acid ester, and polyoxyethylene hydrogenated castor oil.

**14.** The method of inhibiting foaming of a high-concentrated soluble thrombomodulin-containing solution according to any one of claims 1 to 13, **characterized in that** the nonionic surfactant has an existential amount of 0.01 mg or more per 10 mg of the soluble thrombomodulin.

**15.** The method of inhibiting foaming of a high-concentrated soluble thrombomodulin-containing solution according to any one of claims 1 to 14, **characterized in that** the soluble thrombomodulin comprises a peptide that can be dissolved in water in a concentration of 30 mg/mL or more.

**16.** The method of inhibiting foaming of a high-concentrated soluble thrombomodulin-containing solution according to any one of claims 1 to 15, **characterized in that** the soluble thrombomodulin comprises a peptide containing one of the following sequences, has an action of promoting activation of protein C with thrombin, and can be dissolved in the absence of a surfactant:

i) an amino acid sequence at positions 19 to 132 of SEQ ID NO. 3 in a sequence listing;
ii) an amino acid sequence at positions 19 to 132 of SEQ ID NO. 7 in the sequence listing; and
iii) an amino acid sequence obtained by addition of, deletion of, or substitution with at least one amino acid in the amino acid sequence of the (i) or (ii).

**17.** The method of inhibiting foaming of a high-concentrated soluble thrombomodulin-containing solution according to any one of claims 1 to 15, **characterized in that** the soluble thrombomodulin comprises a peptide encoded by DNA capable of hybridizing with complementary DNA having a base sequence corresponding to 55 to 396 of SEQ ID NO. 4 or 8 in the sequence listing under stringent conditions, has an action of promoting activation of protein C with thrombin, and can be dissolved in the absence of a surfactant.

**18.** The method of inhibiting foaming of a high-concentrated soluble thrombomodulin-containing solution according to any one of claims 1 to 15, **characterized in that** the soluble thrombomodulin comprises a peptide containing one of the following sequences, has an action of promoting activation of protein C with thrombin, and can be dissolved in the absence of a surfactant:

i) an amino acid sequence at positions 19 to 516 of SEQ ID NO. 1 in a sequence listing;
ii) an amino acid sequence at positions 19 to 516 of SEQ ID NO. 5 in the sequence listing; and
iii) an amino acid sequence obtained by addition of, deletion of, or substitution with at least one amino acid in the amino acid sequence of the (i) or (ii).

**19.** A soluble thrombomodulin-containing freeze-dried preparation containing soluble thrombomodulin as an active ingredient or a kit preparation thereof which is a combination of the soluble thrombomodulin-containing freeze-dried preparation and a dissolving aqueous solution therefor, the soluble thrombomodulin-containing freeze-dried preparation being provided for preparing a high-concentrated soluble thrombomodulin-containing solution having a concentration of 10 mg/mL or more,
the soluble thrombomodulin-containing freeze-dried preparation or the kit preparation thereof being **characterized by** at least one of

(a) that at least one compound selected from the group consisting of a nonionic surfactant, benzyl alcohol, and chlorobutanol is present in the soluble thrombomodulin-containing freeze-dried preparation and/or at least one compound selected from the group consisting of a nonionic surfactant, benzyl alcohol, and chlorobutanol is present in the dissolving aqueous solution for the soluble thrombomodulin-containing freeze-dried preparation,
(b) that an inner wall of a container to be used in dissolving the soluble thrombomodulin-containing freeze-dried preparation is coated with silicone, and
(c) that the pressure in the container to be used in dissolving the soluble thrombomodulin-containing freeze-dried preparation is at a reduced pressure, reduced by -13332.2 Pa to -39996.6 Pa (-100 to -300 mmHg).

**20.** The soluble thrombomodulin-containing freeze-dried preparation or the kit preparation of the soluble thrombomodulin-containing freeze-dried preparation and a dissolving aqueous solution therefor according to claim 19, the soluble thrombomodulin-containing solution being provided for preparing a high-concentrated soluble thrombomodulin-containing solution having a concentration of 10 mg/mL or more, the soluble thrombomodulin-containing freeze-dried preparation or the kit preparation thereof being **characterized in that** at least one compound selected from the group consisting of a nonionic surfactant, benzyl alcohol and chlorobutanol is present in the soluble thrombomodulin-containing freeze-dried preparation, and/or at least one compound selected from the group consisting of a nonionic surfactant, benzyl alcohol, and chlorobutanol is allowed to be present in the dissolving aqueous solution for the soluble thrombomodulin-containing freeze-dried preparation.

**21.** The soluble thrombomodulin-containing freeze-dried preparation or the kit preparation thereof according to claim 19 or 20, **characterized in that** the high-concentrated soluble thrombomodulin-containing solution has a soluble thrombomodulin concentration of 25 mg/mL or more.

**22.** The soluble thrombomodulin-containing freeze-dried preparation according to any one of claims 19 to 21, **characterized in that**: the soluble thrombomodulin-containing freeze-dried preparation is allowed to contain at least one combination selected from the group consisting of

(1) a combination containing two of glutamic acid or a salt thereof and mannitol,
(2) a combination containing two of glutamic acid or a salt thereof and lysine or a salt thereof,
(3) a combination containing two of glutamic acid or a salt thereof and asparaginic acid or a salt thereof, and
(4) a combination containing two of asparaginic acid or a salt thereof and mannitol; and

the soluble thrombomodulin-containing freeze-dried preparation can be dissolved in 0.1 mL to 2 mL of a dissolving aqueous solution to prepare a high-concentrated soluble thrombomodulin-containing solution having a concentration of 10 mg/mL or more with an osmotic pressure ratio calculated by dividing the osmolality of the thrombomodulin-containing solution by the osmolality of a physiological saline, i.e. 286 m Osm, in a range of 0.5 to 2.0 upon dissolution thereof.

**23.** The soluble thrombomodulin-containing freeze-dried preparation according to any one of claims 19 to 21, **characterized in that**: the soluble thrombomodulin-containing freeze-dried preparation is allowed to contain at least one combination selected from the group consisting of

(1) a combination containing two of glutamic acid or a salt thereof and mannitol,
(2) a combination containing two of glutamic acid or a salt thereof and lysine or a salt thereof,
(3) a combination containing two of glutamic acid or a salt thereof and asparaginic acid or a salt thereof, and
(4) a combination containing two of asparaginic acid or a salt thereof and mannitol; and

the soluble thrombomodulin-containing freeze-dried preparation can be dissolved in 0.1 mL to 2 mL of a dissolving aqueous solution to prepare a high-concentrated soluble thrombomodulin-containing solution having a concentration of 10 mg/mL or more with an osmotic pressure ratio calculated by dividing the osmolality of the thrombomodulin-containing solution by the osmolality of a physiological saline, i.e. 286 m Osm, in a range of 0.5 to 2.0 upon dissolution thereof; and at least one compound selected from the group consisting of a nonionic surfactant, benzyl alcohol, and chlorobutanol is allowed to be present in the soluble thrombomodulin-containing freeze-dried preparation.

**24.** The kit preparation of a soluble thrombomodulin-containing freeze-dried preparation according to any one of claims 19 to 21, **characterized in that**: the soluble thrombomodulin-containing freeze-dried preparation is allowed to contain at least one combination selecting from the group consisting of

(1) a combination containing two of glutamic acid or a salt thereof and mannitol,
(2) a combination containing two of glutamic acid or a salt thereof and lysine or a salt thereof,
(3) a combination containing two of glutamic acid or a salt thereof and asparaginic acid or a salt thereof, and
(4) a combination containing two of asparaginic acid or a salt thereof and mannitol; and

the soluble thrombomodulin-containing freeze-dried preparation can be dissolved in 0.1 mL to 2 mL of dissolving aqueous solution to prepare a high-concentrated soluble thrombomodulin-containing solution having a concentration of 10 mg/mL or more with an osmotic pressure ratio calculated by dividing the osmolality of the thrombomodulin-containing solution by the osmolality of a physiological saline, i.e. 286 m Osm, in a range of 0.5 to 2.0 upon dissolution thereof; and is **characterized by** at least one of

(a) that at least one compound selected from the group consisting of a nonionic surfactant, benzyl alcohol, and chlorobutanol is present in the dissolving aqueous solution for the soluble thrombomodulin-containing freeze-dried preparation,

(b) that an inner wall of a container to be used in dissolving the soluble thrombomodulin-containing freeze-dried preparation is coated with silicone, and
(c) that a pressure in the container to be used in dissolving the soluble thrombomodulin-containing freeze-dried preparation is kept at a reduced pressure, reduced by -13332.2 Pa to -39996.6 Pa (-100 to -300 mmHg).

**25.** The kit preparation of a soluble thrombomodulin-containing freeze-dried preparation according to any one of claims 19 to 21, **characterized in that** the soluble thrombomodulin-containing freeze-dried preparation contains at least one combination selected from the group consisting of

(1) a combination containing two of glutamic acid or a salt thereof and mannitol,
(2) a combination containing two of glutamic acid or a salt thereof and lysine or a salt thereof,
(3) a combination containing two of glutamic acid or a salt thereof and asparaginic acid or a salt thereof, and
(4) a combination containing two of asparaginic acid or a salt thereof and mannitol; and

that the soluble thrombomodulin-containing freeze-dried preparation can be dissolved in 0.1 mL to 2 mL of a dissolving aqueous solution to prepare a high-concentrated soluble thrombomodulin-containing solution having a concentration of 10 mg/mL or more with an osmotic pressure ratio calculated by dividing the osmolality of the thrombomodulin-containing solution by the osmolality of a physiological saline, i.e. 286 mOsm, in a range of 0.5 to 2.0 upon dissolution thereof, and further **characterized by** at least one of

(a) that at least one compound selected from the group consisting of a nonionic surfactant, benzyl alcohol, and chlorobutanol is present in the soluble thrombomodulin-containing freeze-dried preparation and at least one compound selected from the group consisting of a nonionic surfactant, benzyl alcohol, and chlorobutanol is allowed to be present in the dissolving aqueous solution for the soluble thrombomodulin-containing freeze-dried preparation contains,
(b) that an inner wall of a container to be used in dissolving the soluble thrombomodulin-containing freeze-dried preparation is coated with silicone, and
(c) that a pressure in the container to be used in dissolving the soluble thrombomodulin-containing freeze-dried preparation is kept at a reduced pressure, reduced by -13332.2 Pa to -39996.6 Pa (-100 to -300 mmHg).

**26.** The kit preparation of a soluble thrombomodulin-containing freeze-dried preparation according to any one of claims 19 to 21, 24, and 25, **characterized in that** a fluid volume of the dissolving aqueous solution for the soluble thrombomodulin-containing freeze-dried preparation is in a range of 0.1 mL to 2 mL and an osmotic pressure ratio calculated by dividing the osmolality of the thrombomodulin-containg solution by the osmolality of a physiological saline, i.e. 286 mOsm, upon dissolution thereof is in a range of 0.5 to 2.0.

**27.** The soluble thrombomodulin-containing freeze-dried preparation or the kit preparation thereof according to any one of claims 19 to 26, **characterized in that** the soluble thrombomodulin-containing freeze-dried preparation or the kit preparation thereof comprises a preparation for subcutaneous or intramuscular injection.

**28.** The soluble thrombomodulin-containing freeze-dried preparation or the kit preparation thereof according to any one of claims 19, and 20, and 23 to 25, **characterized in that** the nonionic surfactant comprises at least one compound selected from the group consisting of polyoxyethylene sorbitan fatty acid ester, polyoxyethylene/polyoxypropylene copolymer, polyoxyethylene alkylether, polyoxyethylene fatty acid ester, and polyoxyethylene hydrogenated castor oil.

**29.** The soluble thrombomodulin-containing freeze-dried preparation or the kit preparation thereof according to any one of claims 19, 20, 24 to 25, and 28, **characterized in that** the nonionic surfactant has an existential amount of 0.01 mg or more per 10 mg of the soluble thrombomodulin.

**30.** The soluble thrombomodulin-containing freeze-dried preparation or the kit preparation thereof according to any one of claims 19 to 29, **characterized in that** the soluble thrombomodulin comprises a peptide that can be dissolved in water in a concentration of 30 mg/mL or more.

**31.** The soluble thrombomodulin-containing freeze-dried preparation or the kit preparation thereof according to any one of claims 19 to 30, **characterized in that** the soluble thrombomodulin comprises a peptide containing one of the following sequences, has an action of promoting activation of protein C with thrombin, and can be dissolved in the absence of a surfactant:

i) an amino acid sequence at positions 19 to 132 of SEQ ID NO. 3 in a sequence listing;
ii) an amino acid sequence at positions 19 to 132 of SEQ ID NO. 7 in the sequence listing; and
iii) an amino acid sequence obtained by addition of, deletion of, or substitution with at least one amino acid in the amino acid sequence of the (i) or (ii).

**32.** The soluble thrombomodulin-containing freeze-dried preparation or the kit preparation thereof according to any one of claims 19 to 30, **characterized in that** the soluble thrombomodulin comprises a peptide encoded by DNA capable of hybridizing with complementary DNA having a base sequence corresponding to 55 to 396 of SEQ ID NO. 4 or 8 in the sequence listing under stringent conditions, has an action of promoting activation of protein C with thrombin, and can be dissolved in the absence of a surfactant.

**33.** The soluble thrombomodulin-containing freeze-dried preparation or the kit preparation thereof according to any one of claims 19 to 30, **characterized in that** the soluble thrombomodulin comprises a peptide containing one of the following sequences, has an action of promoting activation of protein C with thrombin, and can be dissolved in the absence of a surfactant:

i) an amino acid sequence at positions 19 to 516 of SEQ ID NO. 1 in a sequence listing;
ii) an amino acid sequence at positions 19 to 516 of SEQ ID NO. 5 in the sequence listing; and
iii) an amino acid sequence obtained by addition of, deletion of, or substitution with at least one amino acid in the amino acid sequence of the (i) or (ii).

**34.** A method of stabilizing soluble thrombomodulin in a soluble thrombomodulin-containing freeze-dried preparation that contains the soluble thrombomodulin as an active ingredient, and comprising at least one combination selected from the group consisting of

(1) a combination of two of glutamic acid or a salt thereof and mannitol,
(2) a combination of two of glutamic acid or a salt thereof and lysine or a salt thereof,
(3) a combination of two of glutamic acid or a salt thereof and asparaginic acid or a salt thereof, and
(4) a combination of two of asparaginic acid or a salt thereof and mannitol; and

dissolving the soluble thrombomodulin-containing freeze-dried preparation in 0.1 mL to 2 mL of a dissolving aqueous solution to prepare a high-concentrated soluble thrombomodulin-containing solution having a concentration of 10 mg/mL or more with an osmotic pressure ratio in a range of 0.5 to 2.0 upon dissolution thereof.

**35.** The method of stabilizing soluble thrombomodulin according to claim 34, **characterized by** further comprising a nonionic surfactant in the soluble thrombomodulin-containing freeze-dried preparation.

**36.** The method of stabilizing soluble thrombomodulin according to claim 35, **characterized in that** the nonionic surfactant is at least one compound selected from the group consisting of polyoxyethylene sorbitan fatty acid ester, polyoxyethylene/polyoxypropylene copolymer, polyoxyethylene alkylether, polyoxyethylene fatty acid ester, and polyoxyethylene hydrogenated castor oil.

## Patentansprüche

**1.** Verfahren zum Verhindern des Schäumens einer hochkonzentrierten lösliches Thrombomodulin umfassenden Lösung, die beim Herstellen einer hochkonzentrierten lösliches Thrombomodulin umfassenden Lösung mit einer Konzentration von 10 mg/mL oder mehr durch Lösen einer löslichen Thrombomodulin umfassenden gefriergetrockneten Zubereitung, die lösliches Thrombomodulin als einen Wirkstoff umfasst, verwendet wird, wobei das Verfahren **dadurch gekennzeichnet ist, dass** es mindestens eines der folgenden Merkmale umfasst:

(a) die Gegenwart von mindestens einer Verbindung, die aus der Gruppe ausgewählt wird, die aus einer nichtionischen grenzflächenaktiven Verbindung, Benzylalkohol, und Chlorbutanol besteht;
(b) die Verwendung eines Behälters, der die Innenwand aufweist, die mit Silicon beschichtet ist, wobei der Behälter beim Lösen der lösliches Thrombomodulin umfassenden gefriergetrockneten Zubereitung verwendet wird; und
(c) dass ein Druck in dem Behälter, der beim Lösen der lösliches Thrombomodulin umfassenden gefriergetrockneten Zubereitung verwendet wird, bei einem verringerten Druck gehalten wird, wobei der Druck um von -13332,2 Pa bis -39996,6 Pa (von -100 bis -300 mmHg) verringert ist.

**2.** Verfahren zum Verhindern des Schäumens einer hochkonzentrierten lösliches Thrombomodulin umfassenden Lösung nach Anspruch 1, das beim Herstellen einer hochkonzentrierten lösliches Thrombomodulin umfassenden Lösung mit einer Konzentration von 10 mg/mL oder mehr durch Lösen der lösliches Thrombomodulin umfassenden gefriergetrockneten Zubereitung verwendet wird,
wobei das Verfahren **dadurch gekennzeichnet ist, dass** es die Gegenwart von mindestens einer Verbindung zulässt, die aus der Gruppe ausgewählt wird, die aus einer nichtionischen grenzflächenaktiven Verbindung, Benzylalkohol, und Chlorbutanol besteht.

**3.** Verfahren zum Verhindern des Schäumens einer hochkonzentrierten lösliches Thrombomodulin umfassenden Lösung nach Anspruch 1 oder 2, **dadurch gekennzeichnet, dass** es die Gegenwart von mindestens einer Verbindung in der lösliches Thrombomodulin umfassenden gefriergetrockneten Zubereitung zuläßt, wobei die Verbindung aus der Gruppe ausgewählt wird, die aus einer nichtionischen grenzflächenaktiven Verbindung, Benzylalkohol, und Chlorbutanol besteht.

**4.** Verfahren zum Verhindern des Schäumens einer hochkonzentrierten lösliches Thrombomodulin umfassenden Lösung nach einem beliebigen der Ansprüche 1 bis 3, **dadurch gekennzeichnet, dass** es die Gegenwart von mindestens einer Verbindung in einer lösenden wässerigen Lösung zum Lösen der lösliches Thrombomodulin umfassenden gefriergetrockneten Zubereitung zuläßt, wobei die Verbindung aus der Gruppe ausgewählt wird, die aus einer nichtionischen grenzflächenaktiven Verbindung, Benzylalkohol, und Chlorbutanol besteht.

**5.** Verfahren zum Verhindern des Schäumens einer hochkonzentrierten lösliches Thrombomodulin umfassenden Lösung nach einem beliebigen der Ansprüche 1 bis 4, **dadurch gekennzeichnet, dass** die Innenwand des Behälters, der beim Lösen der lösliches Thrombomodulin umfassenden gefriergetrockneten Zubereitung verwendet werden soll, mit Silicon beschichtet ist.

**6.** Verfahren zum Verhindern des Schäumens einer hochkonzentrierten lösliches Thrombomodulin umfassenden Lösung nach einem beliebigen der Ansprüche 1 bis 5, **dadurch gekennzeichnet, dass** der Druck in dem Behälter, der beim Lösen der lösliches Thrombomodulin umfassenden gefriergetrockneten Zubereitung verwendet werden soll, bei einem verringerten Druck gehalten wird, wobei der Druck um von -13332,2 Pa bis -39996,6 Pa (von -100 bis -300 mmHg) verringert ist.

**7.** Verfahren zum Verhindern des Schäumens einer hochkonzentrierten lösliches Thrombomodulin umfassenden Lösung nach einem beliebigen der Ansprüche 1 bis 6, **dadurch gekennzeichnet, dass** die hochkonzentrierte lösliches Thrombomodulin umfassende Lösung eine Konzentration an löslichem Thrombomodulin von 17 mg/mL oder mehr aufweist.

**8.** Verfahren zum Verhindern des Schäumens einer hochkonzentrierten lösliches Thrombomodulin umfassenden Lösung nach einem beliebigen der Ansprüche 1 bis 6, **dadurch gekennzeichnet, dass** die hochkonzentrierte lösliches Thrombomodulin umfassende Lösung eine Konzentration an löslichem Thrombomodulin von 25 mg/mL oder mehr aufweist.

**9.** Verfahren zum Verhindern des Schäumens einer hochkonzentrierten lösliches Thrombomodulin umfassenden Lösung nach einem beliebigen der Ansprüche 1 bis 8, **dadurch gekennzeichnet, dass** ein fluides Volumen der hochkonzentrierten lösliches Thrombomodulin umfassenden Lösung, die durch Lösen hergestellt wird, in einem Berich von 0,1 mL bis 2 mL liegt, und dass ein Verhältnis des osmotischen Druckes beim Lösen davon in einem Bereich von 0,5 bis 2,0 liegt.

**10.** Verfahren zum Verhindern des Schäumens einer hochkonzentrierten lösliches Thrombomodulin umfassenden Lösung nach einem beliebigen der Ansprüche 1 bis 9, **dadurch gekennzeichnet, dass** es zugelassen wird, dass die lösliches Thrombomodulin umfassende gefriergetrocknete Zubereitung mindestens eine Kombination umfasst, die aus der Gruppe ausgewählt wird, die besteht aus:

(1) einer Kombination, die zwei von Glutaminsäure oder einem Salz davon und Mannitol umfasst,
(2) einer Kombination, die zwei von Glutaminsäure oder einem Salz davon und Lysin oder einem Salz davon umfasst,
(3) einer Kombination, die zwei von Glutaminsäure oder einem Salz davon und Asparaginsäure oder einem Salz davon umfasst, und
(4) einer Kombination, die zwei von Asparaginsäure oder einem Salz davon und Mannitol umfasst; und

dass es zugelassen wird, dass mindestens eine Verbindung, die aus der Gruppe ausgewählt wird, die aus einer nichtionischen grenzflächenaktiven Verbindung, Benzylalkohol, und Chlorbutanol besteht, in der lösliches Thrombomodulin umfassenden gefriergetrockneten Zubereitung vorliegt und/oder dass es zugelassen wird, dass mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorliegt.

**11.** Verfahren zum Verhindern des Schäumens einer hochkonzentrierten lösliches Thrombomodulin umfassenden Lösung nach einem beliebigen der Ansprüche 1 bis 9, **dadurch gekennzeichnet, dass**: es zugelassen wird, dass die lösliches Thrombomodulin umfassende gefriergetrocknete Zubereitung, ein beliebiges von (1) Harnstoff oder (2) Harnstoff und einer Aminosäure umfasst; und es zugelassen wird, dass mindestens eine Verbindung, die aus der Gruppe ausgewählt wird, die aus einer nichtionischen grenzflächenaktiven Verbindung, Benzylalkohol, und Chlorbutanol besteht, in der lösliches Thrombomodulin umfassenden gefriergetrockneten Zubereitung vorhanden ist und/oder es zugelassen wird, dass mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorhanden ist.

**12.** Verfahren zum Verhindern des Schäumens einer hochkonzentrierten lösliches Thrombomodulin umfassenden Lösung nach einem beliebigen der Ansprüche 1 bis 8, **dadurch gekennzeichnet, dass**: es zugelassen wird, dass die lösliches Thrombomodulin umfassende gefriergetrocknete Zubereitung eine oder zwei Verbindungen umfasst, die aus der Gruppe ausgewählt werden, die aus Arginin, Glutaminsäure, Prolin, Serin, Glycin, Histidin, Asparagin, Lysin, Phenylalanin, und Valin, oder Salzen davon, Trehalose, Lactose, und Sucrose besteht; es weiter zugelassen wird, dass mindestens eine Verbindung, die aus der Gruppe ausgewählt wird, die aus einer nichtionischen grenzflächenaktiven Verbindung, Benzylalkohol, und Chlorbutanol besteht, in der lösliches Thrombomodulin umfassenden gefriergetrockneten Zubereitung vorhanden ist und/oder es zugelassen wird, dass mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorhanden ist.

**13.** Verfahren zum Verhindern des Schäumens einer hochkonzentrierten lösliches Thrombomodulin umfassenden Lösung nach einem beliebigen der Ansprüche 1 bis 12, **dadurch gekennzeichnet, dass** die nichtionische grenzflächenaktive Verbindung mindestens eine Verbindung ist, die aus der Gruppe ausgewählt wird, die aus Polyoxyethylensorbitanfettsäureester, Polyoxyethylen/Polyoxypropylen-Copolymer, Polyoxyethylenalkylether, Polyoxyethylenfettsäureester, und Polyoxyethylen-hydriertes Rizinusöl besteht.

**14.** Verfahren zum Verhindern des Schäumens einer hochkonzentrierten lösliches Thrombomodulin umfassenden Lösung nach einem beliebigen der Ansprüche 1 bis 13, **dadurch gekennzeichnet, dass** die nichtionische grenzflächenaktive Verbindung eine Existenzmenge von 0,01 mg oder mehr pro 10 mg des löslichen Thrombomodulins aufweist.

**15.** Verfahren zum Verhindern des Schäumens einer hochkonzentrierten lösliches Thrombomodulin umfassenden Lösung nach einem beliebigen der Ansprüche 1 bis 14, **dadurch gekennzeichnet, dass** das lösliche Thrombomodulin ein Peptid umfasst, das in Wasser in einer Konzentration von 30 mg/mL oder mehr gelöst werden kann.

**16.** Verfahren zum Verhindern des Schäumens einer hochkonzentrierten lösliches Thrombomodulin umfassenden Lösung nach einem beliebigen der Ansprüche 1 bis 15, **dadurch gekennzeichnet, dass** das lösliche Thrombomodulin ein Peptid umfasst, das eine der folgenden Sequenzen aufweist, eine Wirkung zum Fördern der Aktivierung von Protein C mit Thrombin aufweist, und in der Abwesenheit von einer grenzflächenaktiven Verbindung gelöst werden kann:

i) eine Aminosäuresequenz an den Positionen 19 bis 132 von SEQ ID Nr. 3 in einer Sequenzliste;
ii) eine Aminosäuresequenz an den Positionen 19 bis 132 von SEQ ID Nr. 7 in der Sequenzliste; und
iii) eine Aminosäuresequenz, erhalten durch Addition von, Deletion von, oder Substitution mit mindestens einer Aminosäure in der Aminosäuresequenz von (i) oder (ii).

**17.** Verfahren zum Verhindern des Schäumens einer hochkonzentrierten lösliches Thrombomodulin umfassenden Lösung nach einem beliebigen der Ansprüche 1 bis 15, **dadurch gekennzeichnet, dass** das lösliche Thrombomodulin ein Peptid umfasst, das durch DNA kodiert wird, die zur Hybridisierung mit komplementärer DNA fähig ist, die eine Basensequenz aufweist, die den 55 bis 396 von SEQ ID Nr. 4 oder 8 in der Sequenzliste unter stringenten Bedingungen entspricht, das eine Wirkung zum Fördern der Aktivierung von Protein C mit Thrombin aufweist, und das in der Abwesenheit von einer grenzflächenaktiven Verbindung gelöst werden kann.

**18.** Verfahren zum Verhindern des Schäumens einer hochkonzentrierten lösliches Thrombomodulin umfassenden Lösung nach einem beliebigen der Ansprüche 1 bis 15, **dadurch gekennzeichnet, dass** das lösliche Thrombomodulin ein Peptid umfasst, das eine der folgenden Sequenzen umfasst, das eine Wirkung zum Fördern der Aktivierung von Protein C mit Thrombin aufweist, und das in der Abwesenheit von einer grenzflächenaktiven Verbindung gelöst werden kann:

i) eine Aminosäuresequenz an den Positionen 19 bis 516 von SEQ ID Nr. 1 in einer Sequenzliste;
ii) eine Aminosäuresequenz an den Positionen 19 bis 516 von SEQ ID Nr. 5 in der Sequenzliste; und
iii) eine Aminosäuresequenz, erhalten durch Addition von, Deletion von, oder Substitution mit mindestens einer Aminosäure in der Aminosäuresequenz von (i) oder (ii).

**19.** Lösliches Thrombomodulin umfassende gefriergetrocknete Zubereitung, umfassend lösliches Thrombomodulin als einen Wirkstoff oder eine Bausatzzubereitung davon, die eine Kombination der lösliches Thrombomodulin umfassenden gefriergetrockneten Zubereitung und einer lösenden wässerigen Lösung dafür ist, wobei die lösliches Thrombomodulin umfassende gefriergetrocknete Zubereitung zum Herstellen einer hochkonzentrierten lösliches Thrombomodulin umfassenden Lösung mit einer Konzentration von 10 mg/mL oder mehr bereitgestellt wird,
wobei die lösliches Thrombomodulin umfassende gefriergetrocknete Zubereitung oder die Bausatzzubereitung davon dadurch mindestens durch eines der folgenden Merkmale gekennzeichnet ist:

(a) dass mindestens eine Verbindung, die ausgewählt wird aus der Gruppe, die aus einer nichtionischen grenzflächenaktiven Verbindung, Benzylalkohol, und Chlorbutanol besteht, in der lösliches Thrombomodulin umfassenden gefriergetrockneten Zubereitung vorhanden ist und/oder mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorhanden ist,
(b) dass eine Innenwand eines Behälters, der beim Lösen der lösliches Thrombomodulin umfassenden gefriergetrockneten Zubereitung verwendet werden soll, mit Silicon beschichtet ist, und
(c) dass der Druck in dem Behälter, der beim Lösen der lösliches Thrombomodulin umfassenden gefriergetrockneten Zubereitung verwendet werden soll, bei einem verringerten Druck, der um -13332,2 Pa bis -39996,6 Pa (-100 bis -300 mmHg) verringert ist, liegt.

**20.** Lösliches Thrombomodulin umfassende gefriergetrocknete Zubereitung oder Bausatzzubereitung der lösliches Thrombomodulin umfassenden gefriergetrockneten Zubereitung und einer lösenden wässerigen Lösung dafür nach Anspruch 19, wobei die lösliches Thrombomodulin umfassende Lösung zum Herstellen einer hochkonzentrierten lösliches Thrombomodulin umfassenden Lösung mit einer Konzentration von 10 mg/mL oder mehr bereitgestellt wird, wobei die lösliches Thrombomodulin umfassende gefriergetrocknete Zubereitung oder die Bausatzzubereitung davon **dadurch gekennzeichnet ist, dass** mindestens eine Verbindung, die aus der Gruppe ausgewählt wird, die aus einer nichtionischen grenzflächenaktiven Verbindung, Benzylalkohol, und Chlorbutanol besteht, in der lösliches Thrombomodulin umfassenden gefriergetrockneten Zubereitung vorhanden ist, und/oder dass es zugelassen ist, dass mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorhanden ist.

**21.** Lösliches Thrombomodulin umfassende gefriergetrocknete Zubereitung oder Bausatzzubereitung davon nach Anspruch 19 oder 20, **dadurch gekennzeichnet, dass** die hochkonzentrierte lösliches Thrombomodulin umfassende Lösung eine Konzentration an löslichem Thrombomodulin von 25 mg/mL oder mehr aufweist.

**22.** Lösliches Thrombomodulin umfassende gefriergetrocknete Zubereitung nach einem beliebigen der Ansprüche 19 bis 21, **dadurch gekennzeichnet, dass**: es zugelassen ist, dass die lösliches Thrombomodulin umfassende gefriergetrocknete Zubereitung mindestens eine Kombination umfasst, die aus der Gruppe ausgewählt wird, die besteht aus:

(1) einer Kombination, die zwei von Glutaminsäure oder einem Salz davon und Mannitol umfasst,
(2) einer Kombination, die zwei von Glutaminsäure oder einem Salz davon und Lysin oder einem Salz davon umfasst,
(3) einer Kombination, die zwei von Glutaminsäure oder einem Salz davon und Asparaginsäure oder einem Salz davon umfasst; und
(4) einer Kombination, die zwei von Asparaginsäure oder einem Salz davon und Mannitol umfasst; und

dass die lösliches Thrombomodulin umfassende gefriergetrocknete Zubereitung in 0,1 mL bis 2 mL einer lösenden wässerigen Lösung gelöst wird, um eine hochkonzentrierte lösliches Thrombomodulin umfassende Lösung, die eine Konzentration von 10 mg/mL oder mehr mit einem Verhältnis des osmotischen Drucks, das berechnet wird, indem die Osmolalität der Thrombomodulin umfassenden Lösung durch die Osmolalität einer physiologischen Salzlösung, d.h. 286 mOsm, geteilt wird, in einem Bereich von 0,5 bis 2,0 beim Lösen davon aufweist.

23. Lösliches Thrombomodulin umfassende gefriergetrocknete Zubereitung nach einem beliebigen der Ansprüche 19 bis 21, **dadurch gekennzeichnet, dass**: es zugelassen ist, dass die lösliches Thrombomodulin umfassende gefriergetrocknete Zubereitung mindestens eine Kombination umfasst, die aus der Gruppe ausgewählt wird, die besteht aus:

(1) einer Kombination, die zwei von Glutaminsäure oder einem Salz davon und Mannitol umfasst,
(2) einer Kombination, die zwei von Glutaminsäure oder einem Salz davon und Lysin oder einem Salz davon umfasst,
(3) einer Kombination, die zwei von Glutaminsäure oder einem Salz davon und Asparaginsäure oder einem Salz davon umfasst, und
(4) einer Kombination, die zwei von Asparaginsäure oder einem Salz davon und Mannitol umfasst; und

dass die lösliches Thrombomodulin umfassende gefriergetrocknete Zubereitung in 0,1 mL bis 2 mL einer lösenden wässerigen Lösung gelöst werden kann, um eine hochkonzentrierte lösliches Thrombomodulin umfassende Lösung, die eine Konzentration von 10 mg/mL oder mehr mit einem Verhältnis des osmotischen Drucks, das berechnet wird, indem die Osmolalität der Thrombomodulin umfassenden Lösung durch die Osmolalität einer physiologischen Salzlösung, d.h. 286 mOsm, geteilt wird, in einem Bereich von 0,5 bis 2,0 beim Lösen davon aufweist; und dass es zugelassen ist, dass mindestens eine Verbindung, die aus der Gruppe ausgewählt wird, die aus einer nichtionischen grenzflächenaktiven Verbindung, Benzylalkohol, und Chlorbutanol besteht, in der lösliches Thrombomodulin umfassenden gefriergetrockneten Zubereitung vorhanden ist.

24. Bausatzzubereitung einer lösliches Thrombomodulin umfassenden gefriergetrockneten Zubereitung nach einem beliebigen der Ansprüche 19 bis 21, **dadurch gekennzeichnet, dass**: es zugelassen ist, dass die lösliches Thrombomodulin umfassende gefriergetrocknete Zubereitung mindestens eine Kombination umfasst, die aus der Gruppe ausgewählt wird, die besteht aus:

(1) einer Kombination, die zwei von Glutaminsäure oder einem Salz davon und Mannitol umfasst,
(2) einer Kombination, die zwei von Glutaminsäure oder einem Salz davon und Lysin oder einem Salz davon umfasst,
(3) einer Kombination, die zwei von Glutaminsäure oder einem Salz davon und Asparaginsäure oder einem Salz davon umfasst, und
(4) einer Kombination, die zwei von Asparaginsäure oder einem Salz davon und Mannitol umfasst; und

dass die lösliches Thrombomodulin umfassende gefriergetrocknete Zubereitung in 0,1 mL bis 2 mL einer lösenden wässerigen Lösung gelöst werden kann, um eine hochkonzentrierte lösliches Thrombomodulin umfassende Lösung, die eine Konzentration von 10 mg/mL oder mehr mit einem Verhältnis des osmotischen Drucks, das berechnet wird, indem die Osmolalität der Thrombomodulin umfassenden Lösung durch die Osmolalität einer physiologischen Salzlösung, d.h. 286 mOsm, geteilt wird, in einem Bereich von 0,5 bis 2,0 beim Lösen davon aufweist;
und die durch mindestens eines der folgenden Merkmale gekennzeichnet ist:

(a) dass mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorhanden ist,
(b) dass eine Innenwand eines Behälters, der beim Lösen der lösliches Thrombomodulin umfassenden gefriergetrockneten Zubereitung verwendet werden soll, mit Silicon beschichtet ist, und

(c) dass der Druck in dem Behälter, der beim Lösen der lösliches Thrombomodulin umfassenden gefriergetrockneten Zubereitung verwendet werden soll, bei einem verringerten Druck, der um -13332,2 Pa bis -39996,6 Pa (-100 bis -300 mmHg) verringert ist, gehalten wird.

**25.** Bausatzzubereitung einer lösliches Thrombomodulin umfassenden gefriergetrockneten Zubereitung nach einem beliebigen der Ansprüche 19 bis 21, **dadurch gekennzeichnet, dass** die lösliches Thrombomodulin umfassende gefriergetrocknete Zubereitung mindestens eine Kombination umfasst, die aus der Gruppe ausgewählt wird, die besteht aus:

(1) einer Kombination, die zwei von Glutaminsäure oder einem Salz davon und Mannitol umfasst,
(2) einer Kombination, die zwei von Glutaminsäure oder einem Salz davon und Lysin oder einem Salz davon umfasst,
(3) einer Kombination, die zwei von Glutaminsäure oder einem Salz davon und Asparaginsäure oder einem Salz davon umfasst, und
(4) einer Kombination, die zwei von Asparaginsäure oder einem Salz davon und Mannitol umfasst; und

dass die lösliches Thrombomodulin umfassende gefriergetrocknete Zubereitung in 0,1 mL bis 2 mL einer lösenden wässerigen Lösung gelöst werden kann, um eine hochkonzentrierte lösliches Thrombomodulin umfassende Lösung, die eine Konzentration von 10 mg/mL oder mehr mit einem Verhältnis des osmotischen Drucks, das berechnet wird, indem die Osmolalität der Thrombomodulin umfassenden Lösung durch die Osmolalität einer physiologischen Salzlösung, d.h. 286 mOsm, geteilt wird, in einem Bereich von 0,5 bis 2,0 beim Lösen davon aufweist; und die weiter durch mindestens eines der folgenden Merkmale gekennzeichnet ist:

(a) dass mindestens eine Verbindung, die aus der Gruppe ausgewählt wird, die aus einer nichtionischen grenzflächenaktiven Verbindung, Benzylalkohol, und Chlorbutanol besteht, in der lösliches Thrombomodulin umfassenden gefriergetrockneten Zubereitung vorhanden ist, und dass es zugelassen ist, dass mindestens eine Verbindung, die aus der Gruppe ausgewählt wird, die aus einer nichtionischen grenzflächenaktiven Verbindung, Benzylalkohol, und Chlorbutanol besteht, in der lösenden wässerigen Lösung zum Lösen der lösliches Thrombomodulin umfassenden gefriergetrockneten Zubereitung vorhanden ist, enthält,
(b) dass eine Innenwand eines Behälters, der beim Lösen der lösliches Thrombomodulin umfassenden gefriergetrockneten Zubereitung verwendet werden soll, mit Silicon beschichtet ist, und
(c) dass der Druck in dem Behälter, der beim Lösen der lösliches Thrombomodulin umfassenden gefriergetrockneten Zubereitung verwendet werden soll, bei einem verringerten Druck, der um -13332,2 Pa bis -39996,6 Pa (-100 bis -300 mmHg) verringert ist, gehalten wird.

**26.** Bausatzzubereitung einer lösliches Thrombomodulin umfassenden gefriergetrockneten Zubereitung nach einem beliebigen der Ansprüche 19 bis 21, 24, und 25, **dadurch gekennzeichnet, dass** ein fluides Volumen der lösenden wässerigen Lösung zum Lösen der lösliches Thrombomodulin umfassenden gefriergetrockneten Zubereitung in einem Bereich von 0,1 mL bis 2 mL liegt und dass ein Verhältnis des osmotischen Drucks davon, das berechnet wird, indem die Osmolalität der Thrombomodulin umfassenden Lösung durch die Osmolalität einer physiologischen Salzlösung, d.h. 286 mOsm, geteilt wird, beim Lösen davon in einem Bereich von 0,5 bis 2,0 liegt.

**27.** Lösliches Thrombomodulin umfassende gefriergetrocknete Zubereitung oder Bausatzzubereitung davon nach einem beliebigen der Ansprüche 19 bis 26, **dadurch gekennzeichnet, dass** die lösliches Thrombomodulin umfassende gefriergetrocknete Zubereitung oder die Bausatzzubereitung davon eine Zubereitung für subkutane oder intramuskuläre Injektion umfassen.

**28.** Lösliches Thrombomodulin umfassende gefriergetrocknete Zubereitung oder Bausatzzubereitung davon nach einem beliebigen der Ansprüche 19, 20 und 23 bis 25, **dadurch gekennzeichnet, dass** die nichtionische grenzflächenaktive Verbindung mindestens eine Verbindung ist, die aus der Gruppe ausgewählt wird, die aus Polyoxyethylensorbitanfettsäureester, Polyoxyethylen/Polyoxypropylen-Copolymer, Polyoxyethylenalkylether, Polyoxyethylenfettsäureester, und Polyoxyethylen-hydriertes Rizinusöl besteht.

**29.** Lösliches Thrombomodulin umfassende gefriergetrocknete Zubereitung oder Bausatzzubereitung davon nach einem beliebigen der Ansprüche 19, 20, 23 bis 25, und 28, **dadurch gekennzeichnet, dass** die nichtionische grenzflächenaktive Verbindung eine Existenzmenge von 0,01 mg oder mehr pro 10 mg des löslichen Thrombomodulins aufweist.

**30.** Lösliches Thrombomodulin umfassende gefriergetrocknete Zubereitung oder Bausatzzubereitung davon nach einem beliebigen der Ansprüche 19 bis 29, **dadurch gekennzeichnet, dass** das lösliche Thrombomodulin ein Peptid umfasst, das in Wasser in einer Konzentration von 30 mg/mL oder mehr gelöst werden kann.

**31.** Lösliches Thrombomodulin umfassende gefriergetrocknete Zubereitung oder Bausatzzubereitung davon nach einem beliebigen der Ansprüche 19 bis 30, **dadurch gekennzeichnet, dass** das lösliche Thrombomodulin ein Peptid umfasst, das eine der folgenden Sequenzen umfasst, eine Wirkung zum Fördern der Aktivierung von Protein C mit Thrombin aufweist, und in der Abwesenheit von einer grenzflächenaktiven Verbindung gelöst werden kann:

i) eine Aminosäuresequenz an den Positionen 19 bis 132 von SEQ ID Nr. 3 in einer Sequenzliste;
ii) eine Aminosäuresequenz an den Positionen 19 bis 132 von SEQ ID Nr. 7 in der Sequenzliste; und
iii) eine Aminosäuresequenz, erhalten durch Addition von, Deletion von, oder Substitution mit mindestens einer Aminosäure in der Aminosäuresequenz von (i) oder (ii).

**32.** Lösliches Thrombomodulin umfassende gefriergetrocknete Zubereitung oder Bausatzzubereitung davon nach einem beliebigen der Ansprüche 19 bis 30, **dadurch gekennzeichnet, dass** das lösliche Thrombomodulin ein Peptid umfasst, das durch DNA kodiert wird, die zur Hybridisierung mit komplementärer DNA fähig ist, die eine Basensequenz aufweist, die den 55 bis 396 von SEQ ID Nr. 4 oder 8 in der Sequenzliste unter stringenten Bedingungen entspricht, das eine Wirkung zum Fördern der Aktivierung von Protein C mit Thrombin aufweist, und das in der Abwesenheit von einer grenzflächenaktiven Verbindung gelöst werden kann.

**33.** Lösliches Thrombomodulin umfassende gefriergetrocknete Zubereitung oder Bausatzzubereitung davon nach einem beliebigen der Ansprüche 19 bis 30, **dadurch gekennzeichnet, dass** das lösliche Thrombomodulin ein Peptid umfasst, das eine der folgenden Sequenzen umfasst, das eine Wirkung zum Fördern der Aktivierung von Protein C mit Thrombin aufweist, und das in der Abwesenheit von einer grenzflächenaktiven Verbindung gelöst werden kann:

i) eine Aminosäuresequenz an den Positionen 19 bis 516 von SEQ ID Nr. 1 in einer Sequenzliste;
ii) eine Aminosäuresequenz an den Positionen 19 bis 516 von SEQ ID Nr. 5 in der Sequenzliste; und
iii) eine Aminosäuresequenz, erhalten durch Addition von, Deletion von, oder Substitution mit mindestens einer Aminosäure in der Aminosäuresequenz von (i) oder (ii).

**34.** Verfahren zum Stabilisieren von löslichem Thrombomodulin in einer lösliches Thrombomodulin umfassenden gefriergetrockneten Zubereitung, die das lösliche Thrombomodulin als einen Wirkstoff umfasst, und umfassend mindestens eine Kombination, die aus der Gruppe ausgewählt wird, die besteht aus:

(1) einer Kombination, die zwei von Glutaminsäure oder einem Salz davon und Mannitol umfasst,
(2) einer Kombination, die zwei von Glutaminsäure oder einem Salz davon und Lysin oder einem Salz davon umfasst,
(3) einer Kombination, die zwei von Glutaminsäure oder einem Salz davon und Asparaginsäure oder einem Salz davon umfasst, und
(4) einer Kombination, die zwei von Asparaginsäure oder einem Salz davon und Mannitol umfasst; und

Lösen der lösliches Thrombomodulin umfassenden gefriergetrockneten Zubereitung in 0,1 mL bis 2 mL einer lösenden wässerigen Lösung, um eine hochkonzentrierte lösliches Thrombomodulin umfassende Lösung herzustellen, die eine Konzentration von 10 mg/mL oder mehr mit einem Verhältnis des osmotischen Drucks in einem Bereich von 0,5 bis 2,0 beim Lösen davon aufweist.

**35.** Verfahren zum Stabilisieren von löslichem Thrombomodulin nach Anspruch 34, weiter **gekennzeichnet durch** Umfassen einer nichtionischen grenzflächenaktiven Verbindung in der lösliches Thrombomodulin umfassenden gefriergetrockneten Zubereitung.

**36.** Verfahren zum Stabilisieren von löslichem Thrombomodulin nach Anspruch 35, **dadurch gekennzeichnet, dass** die nichtionische grenzflächenaktive Verbindung mindestens eine Verbindung ist, die aus der Gruppe ausgewählt wird, die aus Polyoxyethylensorbitanfettsäureester, Polyoxyethylen/Polyoxypropylen-Copolymer, Polyoxyethylenalkylether, Polyoxyethylenfettsäureester, und Polyoxyethylen-hydriertes Rizinusöl besteht.

**Revendications**

1. Procédé d'inhibition de mousse d'une solution contenant de la thrombomoduline soluble très concentrée, qui est utilisé dans la préparation d'une solution contenant de la thrombomoduline soluble très concentrée ayant une concentration supérieure ou égale à 10 mg/ml par dissolution d'une préparation lyophilisée contenant de la thrombomoduline soluble qui contient de la thrombomoduline soluble en tant que principe actif, le procédé étant **caractérisé en ce qu'**il comprend au moins un de :

   (a) la présence d'au moins un composé choisi dans le groupe constitué par un tensioactif non ionique, l'alcool benzylique et le chlorobutanol ;
   (b) l'utilisation d'un contenant ayant la paroi interne revêtue de silicone, ledit contenant étant utilisé dans la dissolution de la préparation lyophilisée contenant de la thrombomoduline soluble ; et
   (c) une pression dans le contenant à utiliser pour dissoudre la préparation lyophilisée contenant de la thrombomoduline soluble est maintenue à une pression réduite, réduite par -13332,2 Pa à -39996,6 Pa (-100 à -300 mm Hg).

2. Procédé d'inhibition de mousse d'une solution contenant de la thrombomoduline soluble très concentrée selon la revendication 1, qui est utilisé dans la préparation d'une solution contenant de la thrombomoduline soluble très concentrée ayant une concentration supérieure ou égale à 10 mg/ml par dissolution de la préparation lyophilisée contenant de la thrombomoduline soluble,
   le procédé étant **caractérisé en ce qu'**il permet la présence d'au moins un composé choisi dans le groupe constitué par un tensioactif non ionique, l'alcool benzylique et le chlorobutanol.

3. Procédé d'inhibition de mousse d'une solution contenant de la thrombomoduline soluble très concentrée selon la revendication 1 ou 2, **caractérisé en ce qu'**il permet la présence d'au moins un composé choisi dans le groupe constitué par un tensioactif non ionique, l'alcool benzylique et le chlorobutanol dans la préparation lyophilisée contenant de la thrombomoduline soluble.

4. Procédé d'inhibition de mousse d'une solution contenant de la thrombomoduline soluble très concentrée selon l'une quelconque des revendications 1 à 3, **caractérisé en ce qu'**il permet la présence d'au moins un composé choisi dans le groupe constitué par un tensioactif non ionique, l'alcool benzylique et le chlorobutanol dans une solution aqueuse de dissolution pour dissoudre la préparation lyophilisée contenant de la thrombomoduline soluble.

5. Procédé d'inhibition de mousse d'une solution contenant de la thrombomoduline soluble très concentrée selon l'une quelconque des revendications 1 à 4, **caractérisé en ce que** la paroi interne du contenant à utiliser pour dissoudre la préparation lyophilisée contenant de la thrombomoduline soluble est revêtue de silicone.

6. Procédé d'inhibition de mousse d'une solution contenant de la thrombomoduline soluble très concentrée selon l'une quelconque des revendications 1 à 5, **caractérisé en ce que** la pression dans le contenant à utiliser pour dissoudre la préparation lyophilisée contenant de la thrombomoduline soluble est maintenue à une pression réduite, réduite par -13332,2 Pa à -39996,6 Pa (-100 à -300 mm Hg).

7. Procédé d'inhibition de mousse d'une solution contenant de la thrombomoduline soluble très concentrée selon l'une quelconque des revendications 1 à 6, **caractérisé en ce que** la solution contenant de la thrombomoduline soluble très concentrée a une concentration en thrombomoduline soluble supérieure ou égale à 17 mg/ml.

8. Procédé d'inhibition de mousse d'une solution contenant de la thrombomoduline soluble très concentrée selon l'une quelconque des revendications 1 à 6, **caractérisé en ce que** la solution contenant de la thrombomoduline soluble très concentrée a une concentration en thrombomoduline soluble supérieure ou égale à 25 mg/ml.

9. Procédé d'inhibition de mousse d'une solution contenant de la thrombomoduline soluble très concentrée selon l'une quelconque des revendications 1 à 8, **caractérisé en ce qu'**un volume fluide de la solution contenant de la thrombomoduline soluble très concentrée préparée par dissolution est compris dans une plage allant de 0,1 ml à 2 ml et **en ce qu'**un rapport de pression osmotique lors de la dissolution est compris dans une plage allant de 0,5 à 2,0.

10. Procédé d'inhibition de mousse d'une solution contenant de la thrombomoduline soluble très concentrée selon l'une quelconque des revendications 1 à 9, **caractérisé en ce que** la préparation lyophilisée contenant de la thrombomoduline soluble peut contenir au moins une combinaison choisie dans le groupe constitué par

(1) une combinaison contenant deux composés choisis parmi l'acide glutamique ou un sel de celui-ci et le mannitol,
(2) une combinaison contenant deux composés choisis parmi l'acide glutamique ou un sel de celui-ci et la lysine ou un sel de celle-ci,
(3) une combinaison contenant deux composés choisis parmi l'acide glutamique ou un sel de celui-ci et l'acide asparaginique ou un sel de celui-ci, et
(4) une combinaison contenant deux composés choisis parmi l'acide asparaginique ou un sel de celui-ci et le mannitol ; et

au moins un composé choisi dans le groupe constitué par un tensioactif non ionique, l'alcool benzylique et le chlorobutanol peut être présent dans la préparation lyophilisée contenant de la thrombomoduline soluble et/ou au moins un composé choisi dans le groupe constitué par un tensioactif non ionique, l'alcool benzylique et le chlorobutanol peut être présent dans la solution aqueuse de dissolution pour dissoudre la préparation lyophilisée contenant de la thrombomoduline soluble.

**11.** Procédé d'inhibition de mousse d'une solution contenant de la thrombomoduline soluble très concentrée selon l'une quelconque des revendications 1 à 9, **caractérisé en ce que** :

la préparation lyophilisée contenant de la thrombomoduline soluble peut contenir l'un quelconque de (1) une urée ou (2) une urée et un acide aminé ; et au moins un composé choisi dans le groupe constitué par un tensioactif non ionique, l'alcool benzylique et le chlorobutanol peut être présent dans la préparation lyophilisée contenant de la thrombomoduline soluble et/ou au moins un composé choisi dans le groupe constitué par un tensioactif non ionique, l'alcool benzylique et le chlorobutanol peut être présent dans la solution aqueuse de dissolution pour dissoudre la préparation lyophilisée contenant de la thrombomoduline soluble.

**12.** Procédé d'inhibition de mousse d'une solution contenant de la thrombomoduline soluble très concentrée selon l'une quelconque des revendications 1 à 8, **caractérisé en ce que** :

la préparation lyophilisée contenant de la thrombomoduline soluble peut contenir un ou deux composés choisis dans le groupe constitué par l'arginine, l'acide glutamique, la proline, la sérine, la glycine, l'histidine, l'asparagine, la lysine, la phénylalanine et la valine, ou des sels de ceux-ci, le tréhalose, le lactose et le saccharose ; en outre au moins un composé choisi dans le groupe constitué par un tensioactif non ionique, l'alcool benzylique et le chlorobutanol peut être présent dans la préparation lyophilisée contenant de la thrombomoduline soluble et/ou au moins un composé choisi dans le groupe constitué par un tensioactif non ionique, l'alcool benzylique et le chlorobutanol peut être présent dans la solution aqueuse de dissolution pour dissoudre la préparation lyophilisée contenant de la thrombomoduline soluble.

**13.** Procédé d'inhibition de mousse d'une solution contenant de la thrombomoduline soluble très concentrée selon l'une quelconque des revendications 1 à 12, **caractérisé en ce que** le tensioactif non ionique comprend au moins un composé choisi dans le groupe constitué par un ester d'acide gras de polyoxyéthylène sorbitan, un copolymère de polyoxyéthylène/polyoxypropylène, un éther alkylique de polyoxyéthylène, un ester d'acide gras de polyoxyéthylène et une huile de ricin hydrogénée de polyoxyéthylène.

**14.** Procédé d'inhibition de mousse d'une solution contenant de la thrombomoduline soluble très concentrée selon l'une quelconque des revendications 1 à 13, **caractérisé en ce que** le tensioactif non ionique a une quantité existentielle supérieure ou égale à 0,01 mg pour 10 mg de la thrombomoduline soluble.

**15.** Procédé d'inhibition de mousse d'une solution contenant de la thrombomoduline soluble très concentrée selon l'une quelconque des revendications 1 à 14, **caractérisé en ce que** la thrombomoduline soluble comprend un peptide qui peut être dissous dans de l'eau à une concentration supérieure ou égale à 30 mg/ml.

**16.** Procédé d'inhibition de mousse d'une solution contenant de la thrombomoduline soluble très concentrée selon l'une quelconque des revendications 1 à 15, **caractérisé en ce que** la thrombomoduline soluble comprend un peptide contenant une des séquences suivantes, a une action favorisant l'activation de la protéine C avec la thrombine, et peut être dissous en l'absence de tensioactif :

i) une séquence d'acides aminés en positions 19 à 132 de SEQ ID NO : 3 dans une liste de séquences ;
ii) une séquence d'acides aminés en positions 19 à 132 de SEQ ID NO : 7 dans une liste de séquences ; et

iii) une séquence d'acides aminés obtenue par addition de, suppression de ou substitution d'au moins un acide aminé dans la séquence d'acides aminés de (i) ou (ii).

**17.** Procédé d'inhibition de mousse d'une solution contenant de la thrombomoduline soluble très concentrée selon l'une quelconque des revendications 1 à 15, **caractérisé en ce que** la thrombomoduline soluble comprend un peptide codé par de l'ADN capable de s'hybrider avec de l'ADN complémentaire ayant une séquence de base correspondant à 55 à 396 de SEQ ID NO : 4 ou 8 dans la liste de séquences dans des conditions rigoureuses, a une action favorisant l'activation de la protéine C avec la thrombine, et peut être dissous en l'absence de tensioactif.

**18.** Procédé d'inhibition de mousse d'une solution contenant de la thrombomoduline soluble très concentrée selon l'une quelconque des revendications 1 à 15, **caractérisé en ce que** la thrombomoduline soluble comprend un peptide contenant l'une des séquences suivantes, a une action favorisant l'activation de la protéine C avec la thrombine, et peut être dissous en l'absence de tensioactif :

i) une séquence d'acides aminés en positions 19 à 516 de SEQ ID NO : 1 dans une liste de séquences ;
ii) une séquence d'acides aminés en positions 19 à 516 de SEQ ID NO : 5 dans une liste de séquences ; et
iii) une séquence d'acides aminés obtenue par addition de, suppression de ou substitution d'au moins un acide aminé dans la séquence d'acides aminés de (i) ou (ii).

**19.** Préparation lyophilisée contenant de la thrombomoduline soluble qui contient de la thrombomoduline soluble en tant que principe actif ou une préparation de kit de celle-ci qui est une combinaison de la préparation lyophilisée contenant de la thrombomoduline soluble et d'une solution aqueuse de dissolution, la préparation lyophilisée contenant de la thrombomoduline soluble étant utilisée pour la préparation d'une solution contenant de la thrombomoduline soluble très concentrée ayant une concentration supérieure ou égale à 10 mg/ml,
la préparation lyophilisée contenant de la thrombomoduline soluble ou la préparation de kit de celle-ci étant **caractérisée par** au moins un de ce qui suit

(a) au moins un composé choisi dans le groupe constitué par un tensioactif non ionique, l'alcool benzylique et le chlorobutanol est présent dans la préparation lyophilisée contenant de la thrombomoduline soluble et/ou au moins un composé choisi dans le groupe constitué par un tensioactif non ionique, l'alcool benzylique et le chlorobutanol est présent dans la solution aqueuse de dissolution pour la préparation lyophilisée contenant de la thrombomoduline soluble ;
(b) une paroi interne d'un contenant à utiliser pour dissoudre la préparation lyophilisée contenant de la thrombomoduline soluble est revêtue de silicone ; et
(c) la pression dans le contenant à utiliser pour dissoudre la préparation lyophilisée contenant de la thrombomoduline soluble est à une pression réduite, réduite par -13332,2 Pa à -39996,6 Pa (-100 à -300 mm Hg).

**20.** Préparation lyophilisée contenant de la thrombomoduline soluble ou une préparation de kit de la préparation lyophilisée contenant de la thrombomoduline soluble et d'une solution aqueuse de dissolution selon la revendication 19, la solution contenant de la thrombomoduline soluble étant utilisée pour la préparation d'une solution contenant de la thrombomoduline soluble très concentrée ayant une concentration supérieure ou égale à 10 mg/ml, la préparation lyophilisée contenant de la thrombomoduline soluble ou la préparation de kit de celle-ci étant **caractérisée en ce qu'**au moins un composé choisi dans le groupe constitué par un tensioactif non ionique, l'alcool benzylique et le chlorobutanol est présent dans la préparation lyophilisée contenant de la thrombomoduline soluble et/ou au moins un composé choisi dans le groupe constitué par un tensioactif non ionique, l'alcool benzylique et le chlorobutanol peut être présent dans la solution aqueuse de dissolution pour dissoudre la préparation lyophilisée contenant de la thrombomoduline soluble.

**21.** Préparation lyophilisée contenant de la thrombomoduline soluble ou une préparation de kit de celle-ci selon la revendication 19 ou 20, **caractérisée en ce que** la solution contenant de la thrombomoduline soluble très concentrée a une concentration en thrombomoduline soluble supérieure ou égale à 25 mg/ml.

**22.** Préparation lyophilisée contenant de la thrombomoduline soluble selon l'une quelconque des revendications 19 à 21, **caractérisée en ce que** la préparation lyophilisée contenant de la thrombomoduline soluble peut contenir au moins une combinaison choisie dans le groupe constitué par

(1) une combinaison contenant deux composés choisis parmi l'acide glutamique ou un sel de celui-ci et le mannitol,

(2) une combinaison contenant deux composés choisis parmi l'acide glutamique ou un sel de celui-ci et la lysine ou un sel de celle-ci,
(3) une combinaison contenant deux composés choisis parmi l'acide glutamique ou un sel de celui-ci et l'acide asparaginique ou un sel de celui-ci, et
(4) une combinaison contenant deux composés choisis parmi l'acide asparaginique ou un sel de celui-ci et le mannitol ; et

la préparation lyophilisée contenant de la thrombomoduline soluble peut être dissoute dans 0,1 ml à 2 ml d'une solution aqueuse de dissolution pour préparer une solution contenant de la thrombomoduline soluble très concentrée ayant une concentration supérieure ou égale à 10 mg/ml avec un rapport de pression osmotique calculé par division de l'osmolalité de la solution contenant de la thrombomoduline par l'osmolalité d'une solution saline physiologique, c'est-à-dire 286 mOsm, compris dans une plage allant de 0,5 à 2,0 lors de la dissolution.

**23.** Préparation lyophilisée contenant de la thrombomoduline soluble selon l'une quelconque des revendications 19 à 21, **caractérisée en ce que** la préparation lyophilisée contenant de la thrombomoduline soluble peut contenir au moins une combinaison choisie dans le groupe constitué par

(1) une combinaison contenant deux composés choisis parmi l'acide glutamique ou un sel de celui-ci et le mannitol,
(2) une combinaison contenant deux composés choisis parmi l'acide glutamique ou un sel de celui-ci et la lysine ou un sel de celle-ci,
(3) une combinaison contenant deux composés choisis parmi l'acide glutamique ou un sel de celui-ci et l'acide asparaginique ou un sel de celui-ci, et
(4) une combinaison contenant deux composés choisis parmi l'acide asparaginique ou un sel de celui-ci et le mannitol ; et

la préparation lyophilisée contenant de la thrombomoduline soluble peut être dissoute dans 0,1 ml à 2 ml d'une solution aqueuse de dissolution pour préparer une solution contenant de la thrombomoduline soluble très concentrée ayant une concentration supérieure ou égale à 10 mg/ml avec un rapport de pression osmotique calculé par division de l'osmolalité de la solution contenant de la thrombomoduline par l'osmolalité d'une solution saline physiologique, c'est-à-dire 286 mOsm, compris dans une plage allant de 0,5 à 2,0 lors de la dissolution ; et au moins un composé choisi dans le groupe constitué par un tensioactif non ionique, l'alcool benzylique et le chlorobutanol peut être présent dans la préparation lyophilisée contenant de la thrombomoduline soluble.

**24.** Préparation de kit d'une préparation lyophilisée contenant de la thrombomoduline soluble selon l'une quelconque des revendications 19 à 21, **caractérisée en ce que** la préparation lyophilisée contenant de la thrombomoduline soluble peut contenir au moins une combinaison choisie dans le groupe constitué par

(1) une combinaison contenant deux composés choisis parmi l'acide glutamique ou un sel de celui-ci et le mannitol,
(2) une combinaison contenant deux composés choisis parmi l'acide glutamique ou un sel de celui-ci et la lysine ou un sel de celle-ci,
(3) une combinaison contenant deux composés choisis parmi l'acide glutamique ou un sel de celui-ci et l'acide asparaginique ou un sel de celui-ci, et
(4) une combinaison contenant deux composés choisis parmi l'acide asparaginique ou un sel de celui-ci et le mannitol ; et

la préparation lyophilisée contenant de la thrombomoduline soluble peut être dissoute dans 0,1 ml à 2 ml d'une solution aqueuse de dissolution pour préparer une solution contenant de la thrombomoduline soluble très concentrée ayant une concentration supérieure ou égale à 10 mg/ml avec un rapport de pression osmotique calculé par division de l'osmolalité de la solution contenant de la thrombomoduline par l'osmolalité d'une solution saline physiologique, c'est-à-dire 286 mOsm, compris dans une plage allant de 0,5 à 2,0 lors de la dissolution ; et est **caractérisée par** au moins un de ce qui suit

(a) au moins un composé choisi dans le groupe constitué par un tensioactif non ionique, l'alcool benzylique et le chlorobutanol est présent dans la solution aqueuse de dissolution pour la préparation lyophilisée contenant de la thrombomoduline soluble,
(b) une paroi interne d'un contenant à utiliser pour dissoudre la préparation lyophilisée contenant de la thrombomoduline soluble est revêtue de silicone, et
(c) une pression dans le contenant à utiliser pour dissoudre la préparation lyophilisée contenant de la thrombomoduline soluble est maintenue à une pression réduite, réduite par -13332,2 Pa à -39996,6 Pa (-100 à -300 mm Hg).

**25.** Préparation de kit d'une préparation lyophilisée contenant de la thrombomoduline soluble selon l'une quelconque des revendications 19 à 21, **caractérisée en ce que** la préparation lyophilisée contenant de la thrombomoduline soluble contient au moins une combinaison choisie dans le groupe constitué par

(1) une combinaison contenant deux composés choisis parmi l'acide glutamique ou un sel de celui-ci et le mannitol,
(2) une combinaison contenant deux composés choisis parmi l'acide glutamique ou un sel de celui-ci et la lysine ou un sel de celle-ci,
(3) une combinaison contenant deux composés choisis parmi l'acide glutamique ou un sel de celui-ci et l'acide asparaginique ou un sel de celui-ci, et
(4) une combinaison contenant deux composés choisis parmi l'acide asparaginique ou un sel de celui-ci et le mannitol ; et

la préparation lyophilisée contenant de la thrombomoduline soluble peut être dissoute dans 0,1 ml à 2 ml d'une solution aqueuse de dissolution pour préparer une solution contenant de la thrombomoduline soluble très concentrée ayant une concentration supérieure ou égale à 10 mg/ml avec un rapport de pression osmotique calculé par division de l'osmolalité de la solution contenant de la thrombomoduline par l'osmolalité d'une solution saline physiologique, c'est-à-dire 286 mOsm, compris dans une plage allant de 0,5 à 2,0 lors de la dissolution, et **caractérisée en outre par** au moins un de ce qui suit

(a) au moins un composé choisi dans le groupe constitué par un tensioactif non ionique, l'alcool benzylique et le chlorobutanol est présent dans la préparation lyophilisée contenant de la thrombomoduline soluble et au moins un composé choisi dans le groupe constitué par un tensioactif non ionique, l'alcool benzylique et le chlorobutanol peut être présent dans la solution aqueuse de dissolution pour la préparation lyophilisée contenant de la thrombomoduline soluble,
(b) une paroi interne d'un contenant à utiliser pour dissoudre la préparation lyophilisée contenant de la thrombomoduline soluble est revêtue de silicone, et
(c) une pression dans le contenant à utiliser pour dissoudre la préparation lyophilisée contenant de la thrombomoduline soluble est maintenue à une pression réduite, réduite par -13332,2 Pa à -39996,6 Pa (-100 à -300 mm Hg).

**26.** Préparation de kit d'une préparation lyophilisée contenant de la thrombomoduline soluble selon l'une quelconque des revendications 19 à 21, 24 et 25, **caractérisée en ce qu'**un volume fluide de la solution aqueuse de dissolution pour la préparation lyophilisée contenant de la thrombomoduline soluble est compris dans une plage allant de 0,1 ml à 2 ml et **en ce qu'**un rapport de pression osmotique calculé par division de l'osmolalité de la solution contenant de la thrombomoduline par l'osmolalité d'une solution saline physiologique, c'est-à-dire 286 mOsm, lors de la dissolution est compris dans une plage allant de 0,5 à 2,0.

**27.** Préparation lyophilisée contenant de la thrombomoduline soluble ou une préparation de kit de celle-ci selon l'une quelconque des revendications 19 à 26, **caractérisée en ce que** la préparation lyophilisée contenant de la thrombomoduline soluble ou la préparation de kit de celle-ci comprend une préparation pour une injection sous-cutanée ou intramusculaire.

**28.** Préparation lyophilisée contenant de la thrombomoduline soluble ou une préparation de kit de celle-ci selon l'une quelconque des revendications 19, 20 et 23 à 25, **caractérisée en ce que** le tensioactif non ionique comprend au moins un composé choisi dans le groupe constitué par un ester d'acide gras de polyoxyéthylène sorbitan, un copolymère de polyoxyéthylène/polyoxypropylène, un éther alkylique de polyoxyéthylène, un ester d'acide gras de polyoxyéthylène et une huile de ricin hydrogénée de polyoxyéthylène.

**29.** Préparation lyophilisée contenant de la thrombomoduline soluble ou une préparation de kit de celle-ci selon l'une quelconque des revendications 19, 20, 23 à 25 et 28, **caractérisée en ce que** le tensioactif non ionique a une quantité existentielle supérieure ou égale à 0,01 mg pour 10 mg de la thrombomoduline soluble.

**30.** Préparation lyophilisée contenant de la thrombomoduline soluble ou une préparation de kit de celle-ci selon l'une quelconque des revendications 19 à 29, **caractérisée en ce que** la thrombomoduline soluble comprend un peptide qui peut être dissous dans de l'eau à une concentration supérieure ou égale à 30 mg/ml.

**31.** Préparation lyophilisée contenant de la thrombomoduline soluble ou une préparation de kit de celle-ci selon l'une quelconque des revendications 19 à 30, **caractérisée en ce que** la thrombomoduline soluble comprend un peptide contenant une des séquences suivantes, a une action favorisant l'activation de la protéine C avec la thrombine, et peut être dissous en l'absence de tensioactif :

i) une séquence d'acides aminés en positions 19 à 132 de SEQ ID NO : 3 dans une liste de séquences ;
ii) une séquence d'acides aminés en positions 19 à 132 de SEQ ID NO : 7 dans une liste de séquences ; et
iii) une séquence d'acides aminés obtenue par addition de, suppression de ou substitution d'au moins un acide aminé dans la séquence d'acides aminés de (i) ou (ii).

**32.** Préparation lyophilisée contenant de la thrombomoduline soluble ou une préparation de kit de celle-ci selon l'une quelconque des revendications 19 à 30, **caractérisée en ce que** la thrombomoduline soluble comprend un peptide codé par de l'ADN capable de s'hybrider avec de l'ADN complémentaire ayant une séquence de base correspondant à 55 à 396 de SEQ ID NO : 4 ou 8 dans la liste de séquences dans des conditions rigoureuses, a une action favorisant l'activation de la protéine C avec la thrombine, et peut être dissous en l'absence de tensioactif.

**33.** Préparation lyophilisée contenant de la thrombomoduline soluble ou une préparation de kit de celle-ci selon l'une quelconque des revendications 19 à 30, **caractérisée en ce que** la thrombomoduline soluble comprend un peptide contenant l'une des séquences suivantes, a une action favorisant l'activation de la protéine C avec la thrombine, et peut être dissous en l'absence de tensioactif :

i) une séquence d'acides aminés en positions 19 à 516 de SEQ ID NO : 1 dans une liste de séquences ;
ii) une séquence d'acides aminés en positions 19 à 516 de SEQ ID NO : 5 dans une liste de séquences ; et
iii) une séquence d'acides aminés obtenue par addition de, suppression de ou substitution d'au moins un acide aminé dans la séquence d'acides aminés de (i) ou (ii).

**34.** Procédé de stabilisation de thrombomoduline soluble dans une préparation lyophilisée contenant de la thrombomoduline soluble qui contient de la thrombomoduline soluble en tant que principe actif, et comprenant au moins une combinaison choisie dans le groupe constitué par

(1) une combinaison contenant deux composés choisis parmi l'acide glutamique ou un sel de celui-ci et le mannitol,
(2) une combinaison contenant deux composés choisis parmi l'acide glutamique ou un sel de celui-ci et la lysine ou un sel de celle-ci,
(3) une combinaison contenant deux composés choisis parmi l'acide glutamique ou un sel de celui-ci et l'acide asparaginique ou un sel de celui-ci, et
(4) une combinaison contenant deux composés choisis parmi l'acide asparaginique ou un sel de celui-ci et le mannitol ; et

consistant à dissoudre la préparation lyophilisée contenant de la thrombomoduline soluble dans 0,1 ml à 2 ml d'une solution aqueuse de dissolution pour préparer une solution contenant de la thrombomoduline soluble très concentrée ayant une concentration supérieure ou égale à 10 mg/ml avec un rapport de pression osmotique compris dans une plage allant de 0,5 à 2,0 lors de la dissolution.

**35.** Procédé de stabilisation de thrombomoduline soluble selon la revendication 34, caractérisé en outre en qu'il comprend un tensioactif non ionique dans la préparation lyophilisée contenant de la thrombomoduline soluble.

**36.** Procédé de stabilisation de thrombomoduline soluble selon la revendication 35, **caractérisé en ce que** le tensioactif non ionique est au moins un composé choisi dans le groupe constitué par un ester d'acide gras de polyoxyéthylène sorbitan, un copolymère de polyoxyéthylène/polyoxypropylène, un éther alkylique de polyoxyéthylène, un ester d'acide gras de polyoxyéthylène et une huile de ricin hydrogénée de polyoxyéthylène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 64006219 A
- JP 2255699 A
- JP 3133380 A
- JP 3259084 A
- JP 4210700 A
- JP 5213998 A
- WO 9200325 A
- WO 9203149 A
- WO 9315755 A
- JP 3086900 A
- JP 3218399 A
- GB 2015340 A
- JP 6321805 A
- WO 9516460 A
- WO 889811 A
- JP 5310787 A
- JP 7155176 A
- JP 5042920 B
- JP 64045398 A
- JP 6205692 A
- JP 9110900 A
- JP 2002009951 A


### Non-patent literature cited in the description


- **KOJI SUZUKI.** *Igaku-no Ayumi,* 1983, vol. 125, 901
- **M. ZUSHI et al.** *J. Biol. Chem.,* 1989, vol. 246, 10351-10353
- **WEN et al.** *Biochemistry,* 1987, vol. 26, 4350-4357
- **SAMBROOK et al.** Molecular Cloning. Cold Spring Harbor Laboratory Press, 1989
- **R. C. MULLIGAN et al.** *Proc. Natl. Acad. Sci., U.S.A.,* 1981, vol. 78, 2072
- **P. M. HOWLEY et al.** Method by Enzymology. Academic Press, 1983, vol. 101, 387
- *J. Biol. Chem.,* 1989, vol. 264, 10351-10353
- Method in Enzymology. Academic Press, 1983, vol. 100, 468
- **GOMI K. et al.** *Blood,* 1990, vol. 75, 1396-1399
- **QUARTAROLI M. et al.** *J. Pharmacol. Exp. Ther.,* 1999, vol. 290 (1), 158-69